(19)

**Europäisches Patentamt**
**European Patent Office**
**Office européen des brevets**

(11) **EP 3 767 587 B1**

(12) **EUROPÄISCHE PATENTSCHRIFT**

(45) Veröffentlichungstag und Bekanntmachung des Hinweises auf die Patenterteilung:
**23.10.2024 Patentblatt 2024/43**

(21) Anmeldenummer: **19187348.8**

(22) Anmeldetag: **19.07.2019**

(51) Internationale Patentklassifikation (IPC):
**G06T 7/11** (2017.01) **G06T 7/00** (2017.01)

(52) Gemeinsame Patentklassifikation (CPC):
**G06T 7/11; G06T 7/0012;** G06T 2207/10064;
G06T 2207/20084; G06T 2207/30024

(54) **DETEKTION VON PRÄSENZEN UNTERSCHIEDLICHER ANTINUKLEÄRER ANTIKÖRPER-FLUORESZENZMUSTERTYPEN UND VORRICHTUNG HIERFÜR**

DETECTION OF PRESENCE OF DIFFERENT ANTINUCLEAR ANTIBODY FLUORESCENCE PATTERN TYPES AND DEVICE FOR SAME

DÉTECTION DE LA PRÉSENCE DES DIFFÉRENTS TYPES DE MOTIFS FLUORESCENTS D'ANTICORPS ANTINUCLÉAIRES ET DISPOSITIF ASSOCIÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.01.2021 Patentblatt 2021/03**

(73) Patentinhaber: **EUROIMMUN Medizinische Labordiagnostika AG 23560 Lübeck (DE)**

(72) Erfinder:
• **KRAUTH, Jens**
**23552 Lübeck (DE)**
• **KRAUSE, Christopher**
**21514 Büchen (DE)**
• **VOIGT, Jörn**
**23558 Lübeck (DE)**
• **HAHN, Melanie**
**23617 Stockelsdorf (DE)**
• **MARZAHL, Christian**
**91052 Erlangen (DE)**
• **GERLACH, Stefan**
**23627 Groß Grönau (DE)**

(56) Entgegenhaltungen:
DE-A1- 102006 027 516

• INFANTINO M ET AL: "The burden of the variability introduced by the HEp-2 assay kit and the CAD system in ANA indirect immunofluorescence test", IMMUNOLOGIC RESEARCH, HUMANA PRESS, INC, US, vol. 65, no. 1, 25 July 2016 (2016-07-25), pages 345 - 354, XP036222202, ISSN: 0257-277X, [retrieved on 20160725], DOI: 10.1007/S12026-016-8845-3
• RICO HIEMANN1 ET AL: "Die HEp-2-Zelle als Target fur multiparametrische Autoantikorperanalytik - Automatisierung und Standardisierung1) The HEp-2 cell as target for multiparametric autoantibody analyses:automation and standardisation", J LAB MED, vol. 35, no. 6, 1 January 2011 (2011-01-01), pages 351 - 361, XP055864482
• EGERER KARL ET AL: "Automated evaluation of autoantibodies on human epithelial-2 cells as an approach to standardize cell-based immunofluorescence tests", ARTHRITIS RESEARCH AND THERAPY, BIOMED CENTRAL, LONDON, GB, vol. 12, no. 2, 9 March 2010 (2010-03-09), pages R40, XP021085168, ISSN: 1478-6354, DOI: 10.1186/AR2949
• PETTER STRANDMARK ET AL: "HEp-2 staining pattern classification", PATTERN RECOGNITION (ICPR), 2012 21ST INTERNATIONAL CONFERENCE ON, IEEE, 11 November 2012 (2012-11-11), pages 33 - 36, XP032329262, ISBN: 978-1-4673-2216-4

EP 3 767 587 B1

- XI JIA ET AL: "Deep convolutional neural network based HEp-2 cell classification", 2016 23RD INTERNATIONAL CONFERENCE ON PATTERN RECOGNITION (ICPR), IEEE, 4 December 2016 (2016-12-04), pages 77 - 80, XP033085562, DOI: 10.1109/ICPR.2016.7899611
- GUPTA KRATI ET AL: "CNN based Mitotic HEp-2 Cell Image Detection :", PROCEEDINGS OF THE 11TH INTERNATIONAL JOINT CONFERENCE ON BIOMEDICAL ENGINEERING SYSTEMS AND TECHNOLOGIES, 1 January 2018 (2018-01-01), pages 167 - 174, XP055864490, ISBN: 978-989-7583-07-0, Retrieved from the Internet <URL:https://pdfs.semanticscholar.org/7921/a9 e39138d63d69e479c24bd5a718a564db82.pdf?_g a=2.78673044.1673975225.1637658002-1613779 853.1637242792> DOI: 10.5220/0006721501670174
- CASCIO DONATO ET AL: "Deep Convolutional Neural Network for HEp-2 Fluorescence Intensity Classification", APPLIED SCIENCES, vol. 9, no. 3, 26 January 2019 (2019-01-26), pages 408, XP055864499, DOI: 10.3390/app9030408
- WAN SHENG ET AL: "Confnet: Predict with Confidence", 2018 IEEE INTERNATIONAL CONFERENCE ON ACOUSTICS, SPEECH AND SIGNAL PROCESSING (ICASSP), IEEE, 15 April 2018 (2018-04-15), pages 2921 - 2925, XP033401070, DOI: 10.1109/ICASSP.2018.8461745
- GAO ZHIMIN ET AL: "HEp-2 Cell Image Classification with Deep Convolutional Neural Networks", FACULTY OF ENGINEERING AND INFORMATION SCIENCES -PAPERS: PART B, 1 January 2017 (2017-01-01), pages 1 - 32, XP055864511, Retrieved from the Internet <URL:https://ro.uow.edu.au/cgi/viewcontent.cgi ?referer=https://www.google.com/&httpsredir=1 &article=1154&context=eispapers1> [retrieved on 20211123]
- DIVYA BS ET AL: "HEp-2 cell classification using artificial neural network approach", 2016 23RD INTERNATIONAL CONFERENCE ON PATTERN RECOGNITION (ICPR), IEEE, 4 December 2016 (2016-12-04), pages 84 - 89, XP033085564, DOI: 10.1109/ICPR.2016.7899613
- ORAIBI ZAKARIYA A ET AL: "Learning Local and Deep Features for Efficient Cell Image Classification Using Random Forests", 2018 25TH IEEE INTERNATIONAL CONFERENCE ON IMAGE PROCESSING (ICIP), IEEE, 7 October 2018 (2018-10-07), pages 2446 - 2450, XP033454860, DOI: 10.1109/ICIP.2018.8451287
- "Precision Medicine - Role of Autoantibodies in the Prediction and Care of Autoimmune Diseases Report on the 14th Dresden Symposium on Autoantibodies, September 10-13, 2019", 1 January 2019, PABST SCIENCE PUBLISHERS, article RICO HIEMANN ET AL: "Artificial intelligence for automated pattern recognition", pages: 415 - 416, XP055864591
- CONRAD KARSTEN: "14 th DRESDEN SYMPOSIUM ON AUTOANTIBODIES CALL FOR ABSTRACTS -DEADLINE JUNE 10, 2019", 10 June 2019 (2019-06-10), pages 1 - 2, XP055886226, Retrieved from the Internet <URL:chrome-extension://efaidnbmnnnibpcajpc glclefindmkaj/viewer.html?pdfurl=https%3A%2F %2Fwww.gfid-ev.de%2Fformulare%2F14DSA_a bstract.pdf&clen=207066&chunk=true>
- C.-H. LIN ET AL: "SAT0646 Development and validation of a deep learning algorithm for classifying anti-nuclear antibody patterns in indirect immunofluorescence images", SATURDAY, 16 JUNE 2018, 1 June 2018 (2018-06-01), pages 1173.2 - 1173, XP055655924, DOI: 10.1136/annrheumdis-2018-eular.6635
- RODRIGUES LARISSA FERREIRA ET AL: "Exploiting Convolutional Neural Networks and Preprocessing Techniques for HEp-2 Cell Classification in Immunofluorescence Images", 2017 30TH SIBGRAPI CONFERENCE ON GRAPHICS, PATTERNS AND IMAGES (SIBGRAPI), IEEE, 17 October 2017 (2017-10-17), pages 170 - 177, XP033248569, DOI: 10.1109/SIBGRAPI.2017.29

## Beschreibung

Die Erfindung betrifft ein Verfahren sowie eine Vorrichtung zur Detektion jeweiliger potentieller Präsenzen jeweiliger unterschiedlicher zellulärer Fluoreszenzmustertypen auf einem biologischen Zellsubstrat aufweisend humane Epitheliomzellen (HEp-Zellen), wobei die Fluoreszenzmustertypen unterschiedliche antinukleäre Antikörper-Fluoreszenzmustertypen umfassen. Die Erfindung betrifft ferner ein Verfahren zur Detektion potentieller Präsenzen unterschiedlicher zellulärer Fluoreszenzmustertypen auf einem biologischen Zellsubstrat aufweisend humane Epitheliomzellen mittels digitaler Bildverarbeitung sowie eine Recheneinheit, eine Datennetzwerkvorrichtung, ein Computerprogrammprodukt als auch ein Datenträgersignal hierfür.

Für die Zwecke medizinischer Diagnostik und insbesondere zum Zwecke einer Detektion von Autoantikörpern in einer flüssigen Patientenprobe wie beispielsweise Blut oder Blutserum ist es eine bekannte Methode, ein biologisches Substrat, welches humane Epitheliomzellen aufweist, mit der flüssigen Patientenprobe oder aber der verdünnten flüssigen Patientenprobe zu inkubieren. Abhängig von einem Vorhandensein bestimmter primärer Autoantikörper in der Patientenprobe ergibt sich eine Bindung dieser primären Antikörper in unterschiedlichen Bereichen der genannten Zellen. Es wird dann ferner das biologische Zellsubstrat mit sekundären Antikörpern inkubiert, welche mit einem sogenannten Fluoreszenzfarbstoff markiert sind. Solche sekundären Antikörper können an die primären Antikörper, welche auf dem Zellsubstrat gebunden sind, wiederum anbinden. Nach einem Bestrahlen des Substrates mit einer Anregungsstrahlung ergibt sich dann eine Fluoreszenz des erwähnten Fluoreszenzfarbstoffes, sodass in einem entsprechenden Fluoreszenzbild dann die primären Antikörper der Patientenprobe, welche an die Zellen des Zellsubstrats gebunden sind und an welche wiederum sekundäre Antikörper mit dem Fluoreszenzfarbstoff gebunden sind, in einem Fluoreszenzbild als ein Fluoreszenzmuster lokalisiert werden können. Abhängig von einer Erkrankung des Patienten bzw. einem Vorhandensein spezifischer primärer Antikörper in der Patientenprobe ergeben sich unterschiedliche spezifische Fluoreszenzmuster. Bei einer solchen Detektion von primären Antikörpern als sogenannte Autoantikörper, welche sich gegen die Zellkerne bzw. die Antigene des Zellkerns richten, ergeben sich also hierbei sogenannte antinukleäre Antikörper-Fluoreszenzmuster. Diese nennt man auch sogenannte ANA-Fluoreszenzmuster oder ein ANA-Muster.

Bei der Bezeichnung solcher Autoantigene hat man sich entweder nach biochemischen Merkmalen gerichtet (DNS, Histone, Ribonuclioproteine: RNP), oder nach mit den Autoantikörpern assoziierten Krankheiten.

Im Zuge der indirekten Immunfluoreszenz (IIFT) mit humanen Epithelzellen bzw. humanen Epitheliomzellen stellt sich also die Aufgabe, die sich ergebenden unterschiedlichen Fluoreszenzmustertypen zu erkennen und zu klassifizieren. Hierbei muss nicht unbedingt ein einzelnes Muster alleine für sich in einem Fluoreszenzbild präsent sein, sondern es können auch mehrere Muster gleichzeitig präsent sein.

Antikörper gegen nukleäre Antigene sind gegen verschiedene Zellkernbestandteile (biochemische Substanzen des Zellkerns) gerichtet. Diese umfassen die Nukleinsäuren, Zellkernproteine und Ribonuclioproteine.

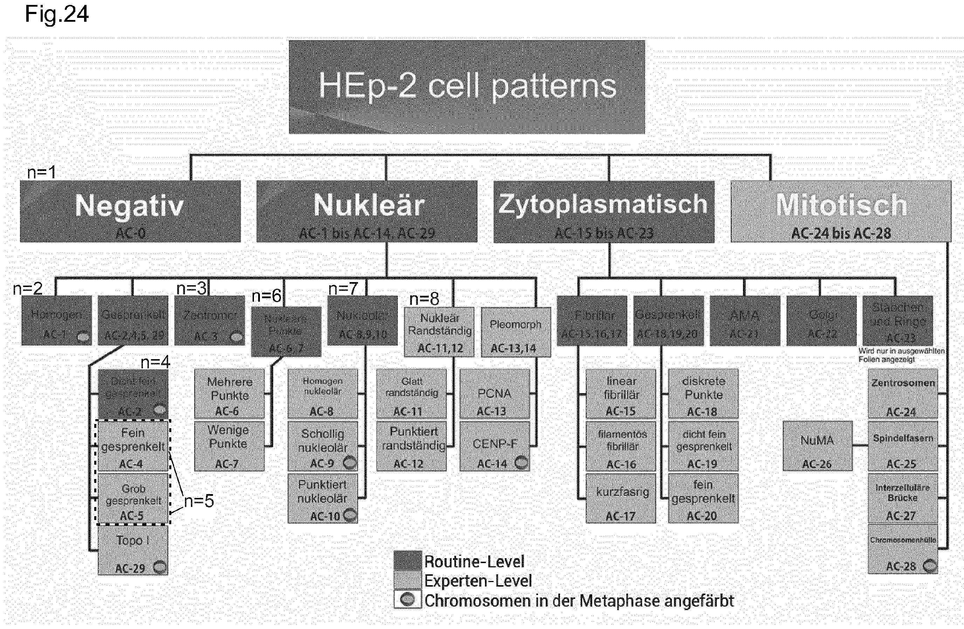

Die unterschiedlichen sich ergebenden antinukleären Antikörper-Fluoreszenzmustertypen werden in unterschiedliche Mustertypen unterschieden. Ein Ansatz hierzu findet sich auf der Internetseite www.anapatterns.org, auf welcher unter anderem unterschiedliche nukleäre Muster aufgeführt und unterschieden werden. Eine beispielhafte Klassifizierung gemäß dieses Schemas ist in der Figur 24 illustriert.

Das Dokument DE102006027516A1 offenbart ein Verfahren unter Verwendung eines Indikatorfarbstoffs als sogenannte Gegenfärbung und eines Fluoreszenzfarbstoffs zur Darstellung Antikörper-spezifischer Bindung an ein Substrat. Hierbei werden in der Gegenfärbung relevante Strukturen, wie beispielsweise Zellen im Mitosestadium identifiziert. Für solche relevanten Strukturen werden dann Einzelbilder der Fluoreszenzbilder extrahiert und einem Klassifikator zugeführt.

Das Dokument C.-H. LIN ET AL: "SAT0646 Development and validation of a deep learning algorithm for classifying anti-nuclear antibody patterns in indirect immunofluorescence images", SATURDAY, 16 JUNE 2018, 1. Juni 2018 (2018-06-01), offenbart eine Klassifikation von Fluoreszenzbildern mittels eines Convolutional Neural Networks.

Das Dokument Infantino M, Meacci F, Grossi V, Manfredi M, Benucci M, Merone M, Soda P. "The burden of the variability introduced by the HEp-2 assay kit and the CAD system in ANA indirect immunofluorescence test" Immunol Res. 2017 Feb;65(1):345-354, beschreibt ein Immunfluoreszenzverfahren, bei welchem von einer biologischen Probe Fluoreszenzbilder in zwei unterschiedlichen Farbkanälen, DAPI und FITC, aufgenommen werden, wobei das Bild des DAPI-Farbkanals zur Segmentierung von Zellbereichen analysiert wird, wobei ferner dann Teilbilder des FITC-Farbkanals so ausgewählt werden, dass eine Mindestanzahl bestimmter Zelltypen in den Teilbildern des FITC-Farbkanals vorhanden sind, wobei ferner die ausgewählten Teilbilder des FITC-Farbkanals unter Berücksichtigung ihrer Signalintensität in eine Gruppe positiver und eine Gruppe negativer Teilbilder aufgeteilt wird und wobei dann die positiven Teilbilder aus dem FITC-Farbkanal einer Musterklassifikation unterzogen werden, um zwischen verschiedenen ANA Fluorezenzmustern zu unterscheiden.

Das Dokument Hiemann, Rico, Roggenbuck, Dirk, Sack, Ulrich, Anderer, Ursula and Conrad, Karsten. "Die HEp-2-Zelle als Target für multiparametrische Autoantikörperanalytik - Automatisierung und Standardisierung/The HEp-2 cell as target for multiparametric autoantibody analyses: automation and standardisation" LaboratoriumsMedizin, vol.

35, no. 6, 2011, pp. 351-361, offenbart ebenfalls ein Verfahren, bei welchem eine Detektion von Fluoreszenzmustern mittels Mustererkennung in Fluoreszenzbildern eines einzelnen Farbkanals erfolgt.

Das Dokument Egerer, K., Roggenbuck, D., Hiemann, R. et al. "Automated evaluation of autoantibodies on human epithelial-2 cells as an approach to standardize cell-based immunofluorescence tests", Arthritis Res Ther 12, R40 (2010), offenbart ebenfalls ein Verfahren, bei welchem eine Detektion von Fluoreszenzmustern mittels Mustererkennung in Fluoreszenzbildern eines einzelnen Farbkanals erfolgt, wobei insbesondere ein Convolutional Neural Network zum Einsatz kommt.

Aufgabe der vorliegenden Erfindung ist es, mittels digitaler Bildverarbeitung ein Verfahren bereitzustellen, welches automatisiert jeweilige potentielle Präsenzen jeweiliger unterschiedlicher antinukleärer Antikörper-Fluoreszenzmustertypen in einem Fluoreszenzbild detektiert.

Die erfindungsgemäße Aufgabe wird gelöst durch das vorgeschlagene Verfahren nach Anspruch 1, die vorgeschlagene Recheneinheit nach Anspruch 6, die Datennetzwerkvorrichtung nach Anspruch 7, das vorgeschlagene Computerprogrammprodukt nach Anspruch 8 sowie das vorgeschlagene Datenträgersignal nach Anspruch 9.

Vorgeschlagen wird ein Verfahren zur Detektion jeweiliger potentieller Präsenzen jeweiliger unterschiedlicher zellulärer Fluoreszenzmustertypen auf einem biologischen Zellsubstrat aufweisend humane Epitheliomzellen, wobei die zellulären Fluoreszenzmustertypen mehrere unterschiedliche antinukleäre Antikörper-Fluoreszenzmustertypen umfassen. Das Verfahren weist unterschiedliche Schritte auf. Es erfolgt ein Inkubieren des Zellsubstrates mit einer flüssigen Patientenprobe, welche potentiell primäre Antikörper aufweist. Vorzugsweise ist die flüssige Patientenprobe verdünntes Patientenblut, besonders bevorzugt verdünntes Blutserum des Patienten. Das Zellsubstrat wird ferner mit einem ersten Fluoreszenzfarbstoff inkubiert. Außerdem wird das Zellsubstrat ferner mit sekundären Antikörpern, welche mit einem zweiten Fluoreszenzfarbstoff markiert sind, inkubiert. Es erfolgt ferner vorzugsweise ein Bestrahlen des Zellsubstrates mit einer Anregungsstrahlung. Ferner erfolgt ein Erfassen eines ersten Bildes, welches eine Färbung des Zellsubstrates durch den ersten Fluoreszenzfarbstoff repräsentiert. Ferner erfolgt ein Erfassen eines zweiten Bildes, welches eine Färbung des Zellsubstrates durch den zweiten Fluoreszenzfarbstoff repräsentiert.

Aufgrund der Färbung des Zellsubstrates mit dem ersten Fluoreszenzfarbstoff ergibt sich eine Färbung des Zellsubstrates in dem ersten Bild, welche es ermöglicht, unterschiedliche zelluläre Bereiche bezogen auf ihre Lage in dem ersten Bild optisch zu erkennen. Das erste Bild ist vorzugsweise räumlich gleich ausgerichtet wie das zweite Bild. Ein solcher erster Fluoreszenzfarbstoff bindet unspezifisch von einer Präsenz von primären Antikörpern in einer Patientenprobe an zelluläre Bereiche an und erlaubt somit ein prinzipielles Erkennen unterschiedlicher Zellbereiche in dem ersten Bild.

Dadurch, dass die sekundären Antikörper an solche primären Antikörper binden können, welche aus der flüssigen Patientenprobe stammen und an spezifische Bereiche des Zellsubstrates bzw. des Zellkerns gebunden sind, können abhängig von der Präsenz jeweiliger unterschiedlicher Typen von primären Antikörpern jeweilige unterschiedliche Antikörper-Fluoreszenzmuster in dem zweiten Bild vorliegen und dann erkannt werden.

Es erfolgt in dem erfindungsgemäßen Verfahren ferner ein Detektieren jeweiliger Bildsegmente, welche jeweils wenigstens eine mitotische Zelle von einer hinreichenden Qualität repräsentieren, auf Basis des ersten Bildes, wobei eine Mitosezelle dann eine hinreichende Qualität aufweist, wenn sie sich in der Metaphase befindet. Es erfolgt dann ferner ein Selektieren von Teilbildern des ersten Bildes und dazu korrespondierenden Teilbildern des zweiten Bildes auf Basis der detektierten Bildsegmente, welche jeweils wenigstens eine mitotische Zelle (MZX, MZY) einer hinreichenden Qualität repräsentieren. Es erfolgt ferner ein Detektieren jeweiliger tatsächlicher Präsenzen jeweiliger der zellulären Fluoreszenzmustertypen mittels eines Convolutional Neural Network auf Basis der selektierten Teilbilder des ersten Bildes und der selektierten Teilbilder des zweiten Bildes. Es erfolgt ferner ein Bestimmen jeweiliger Konfidenzmaße für die jeweiligen tatsächlichen Präsenzen der jeweiligen Fluoreszenzmustertypen mittels des Convolutional Neural Network auf Basis der selektierten Teilbilder des ersten Bildes und der selektierten Teilbilder des zweiten Bildes, wobei für ein jeweiliges Teilbild-Tupel, welches ein Teilbild des ersten Bildes und ein korrespondierendes Teilbild des zweiten Bildes aufweist, jeweilige Teilbild-Konfidenzmaße für jeweilige tatsächliche Teilbild-Präsenzen jeweiliger zellulärer Fluoreszenzmustertypen mittels des Convolutional Neural Network bestimmt werden, so dass das Convolutional Neural Network zueinander korrespondierende Teilbilder unterschiedlicher Farbkanäle gleichzeitig auswertet. Schließlich erfolgt das Bestimmen der jeweiligen Konfidenzmaße für die jeweiligen tatsächlichen Präsenzen der jeweiligen Fluoreszenzmustertypen auf Basis der Teilbild-Konfidenzmaße.

Für eine Detektion von unterschiedlichen ANA-Mustern wäre es prinzipiell möglich, ein gesamtes Fluoreszenzbild bzw. das gesamte zweite Bild mit einer großen Mehrzahl von humanen Epitheliomzellen (HEp-Zellen) zu analysieren und für eine Erkennung der unterschiedlichen ANA-Muster ein solches gesamtes Fluoreszenzbild einem einzelnen Convolutional Neural Network für das Detektieren der jeweiligen Präsenzen der jeweiligen Fluoreszenzmustertypen zuzuführen. Das Convolutional Neural Network (CNN) müsste dann die Gesamtheit aller HEp-Zellen des Bildes auswerten, um die unterschiedlichen antinukleären Antikörper-Fluoreszenzmustertypen zu erkennen. Für eine genaue Detektion ist es zwar einerseits aus statistischen Gründen vorteilhaft, möglichst viele HEp-Zellen zu betrachten. Jedoch gibt es hierbei auch verschiedene Nachteile. Einerseits wäre ein Rechenaufwand zur Verarbeitung eines gesamten Fluoreszenzbildes mit einer großen Mehrzahl von HEp-Zellen sehr hoch. Ferner würde eine Verarbeitung eines solchen großen Gesamtbildes mit sehr vielen HEp-Zellen einen gro-ßen Freiheitsgrad an abstrakter Bildinformation darstellen. Würde das Convolutional Neural Network während einer Trainingsphase mit solchen großen gesamten Fluoreszenzbildern trainiert werden, so würde die Menge und die Varianz abstrakter Bildinformation möglicherweise zu groß sein, als dass das Convolutional Neural Network in der Trainingsphase hinreichend zu einem Zustand konvergieren könnte um eine sichere Detektion unterschiedlicher Fluoreszenzmustertypen bzw. Fluoreszenzmusterklassen zu ermöglichen.

Daher wird erfindungsgemäß vorgeschlagen, dass auf Basis des ersten Bildes, welches mit dem ersten Fluoreszenzfarbstoff unspezifisch von einer Antikörperbindung gefärbt ist, zunächst bestimmte Bildsegmente detektiert werden, welche jeweils wenigstens eine mitotische Zelle repräsentieren, um dann solche Teilbilder des ersten Bildes und solche Teilbilder des zweiten Bildes zu selektieren, welche ein entsprechendes Bildsegment mit wenigstens einer mitotischen Zelle aufweisen. Eine mitotische Zelle wird auch als eine Mitosezelle bezeichnet. Insbesondere sind die Bildsegmente in ihrer räumlichen Ausdehnung kleiner als die Teilbilder.

Hierdurch ergibt sich zum einen der Vorteil, dass das Convolutional Neural Network jeweils nur ein Teilbild des zweiten Bildes mit antikörperspezifischer Fluoreszenzfarbstoff-Färbung und das dazu korrespondierende Teilbild des ersten Bildes prozessieren muss und nicht ein gesamtes erstes Bild und ein gesamtes zweites Bild auf einmal. Daher kann das Convolutional Neural Network auf eine Größe derartiger Teilbilder hin trainiert werden und muss nur solche Teilbilder mit einer begrenzten Anzahl von HEp-Zellen und somit nur eine begrenzte Menge an abstrakter Bildinformation auswerten. Ferner ist das vorgeschlagene Verfahren insbesondere daher vorteilhaft, da eben nur solche Teilbilder selektiert werden, welche auch eine Mitosezelle enthalten. Dadurch, dass die Teilbilder eine Mitosezelle enthalten, eignen sie sich besonders gut für eine Differenzierung unterschiedlicher nukleärer Muster, da für eine Detektion bestimmter nukleärer Muster nicht nur sogenannte Interphase-Zellen hinsichtlich ihrer Färbung durch den Fluoreszenzfarbstoff betrachtet werden müssen, sondern eben auch Mitosezellen. Dieses erlaubt ein Differenzieren zwischen manchen Fluoreszenzmustertypen. Durch das Sicherstellen, dass ein Teilbild ein Bildsegment mit einer Mitosezelle aufweist, wird also eine Qualität der Detektion der jeweiligen antinukleären Antikörper-Fluoreszenzmustertypen erhöht.

Insbesondere ist das vorgeschlagene Verfahren insbesondere daher vorteilhaft, da das Convolutional Neural Network durch das Sicherstellen, dass die Teilbilder entsprechende Segmente mit wenigstens einer Mitosezelle aufweisen, für eine Detektion der jeweiligen Präsenzen der jeweiligen Fluoreszenzmustertypen auch eine möglicherweise vorhandene Färbung einer Metaphasenplatte einer mitotische Zelle erkennen kann, um zwischen unterschiedlichen antinukleären Anitkörper-Fluoreszenzmustertypen zu unterscheiden.

Da in dem erfindungsgemäßen Verfahren das Convolutional Neural Network nicht nur das selektierte Teilbild des zweiten Bildes, welches eine patientenspezifische Färbung mit dem zweiten Fluoreszenzfarbstoff aufweist, sondern eben auch ein dazu korrespondierendes Teilbild des ersten Bildes, welches eine patientenunspezifische Färbung des Zellsubstrates mit dem ersten Fluoreszenzfarbstoff aufweist, gemeinsam verarbeitet, kann das Convolutional Neural Network hier auf eine Lokalisationsinformation von Zellbereichen bzw. Zellkernbereichen, welche in dem ersten Teilbild vorhanden sind, abstellen und gleichzeitig auch die tatsächliche patientenspezifischen Fluoreszenzmuster in den Teilbildern des zweiten Bildes detektieren. Das Convolutional Neural Network wertet hier also gleichzeitig zu einander korrespondierende Fluoreszenzteilbilder unterschiedlicher Farbkanäle aus.

Vorteilhafte Ausführungsformen der Erfindung sind Gegenstand der abhängigen Ansprüche und werden in der folgenden Beschreibung unter teilweiser Bezugnahme auf die Figur näher erläutert.

Im Sinne dieser Anmeldung weist eine Mitosezelle dann eine hinreichende Qualität auf bzw. ist dann valide, wenn sie sich in einem korrekten bzw. validen Mitosestadium befindet. Ein korrektes bzw. valides Mitosestadium ist die sogenannte Metaphase. Eine Mitosezelle, die sich nicht in einer Methapase befindet, ist also eine falsche bzw. invalide Mitosezelle.

Vorzugsweise erfolgt eine Ausgabe der jeweiligen Konfidenzmaße.

Vorzugsweise weist das Convolutional Neural Network ein Ausgabe-Layer auf, welches für einen jeweiligen zellulären Fluoreszenzmustertypen eine jeweilige Feature-Map generiert, insbesondere für ein jeweiliges Teilbild-Tupel, wobei ferner das Convolutional Neural Network ein jeweiliges Konfidenzmaß, insbesondere für ein jeweiliges Teilbild-Tupel, auf Basis einer jeweiligen Feature-Map bestimmt. Ein Teilbild-Tupel weist ein Teilbild des ersten Bildes und ein dazu korrespondierendes Teilbild des zweiten Bildes auf.

Vorzugsweise weist das Verfahren ferner auf: Segmentieren des ersten Bildes in Bildsegmente unterschiedlicher Segmentklassen, wobei vorzugsweise zumindest eine erste Segmentklasse ein Zellstadium einer Zelle repräsentiert und wobei vorzugsweise zumindest eine zweite Segmentklasse einen Zellbereich innerhalb einer Zelle repräsentiert, ferner Bestimmen eines Helligkeitswertes für wenigstens einen Fluoreszenzmustertypen basierend auf einem oder mehreren Bildsegmenten wenigstens einer bestimmten Segmentklasse, sowie ferner Verifizieren des Konfidenzmaßes des Fluoreszenzmustertypen auf Basis des Helligkeitswertes des Fluoreszenzmustertypen.

Vorzugsweise erfolgt das Verifizieren des Konfidenzmaßes auf Basis des Helligkeitswertes und in Abhängigkeit eines durch einen Nutzer vorgebbaren Schwellenwertes.

Vorzugsweise weist das Verfahren ferner auf: Aufteilen des zweiten Bildes in eine Menge von Teilbildern nach einem vorgegebenen Aufteilungsschema, Selektieren von Teilbildern des zweiten Bildes auf Basis der detektierten Bildsegmente sowie ferner Selektieren von dazu korrespondierenden Teilbildern des ersten Bildes und Detektieren jeweiliger tatsächlicher Präsenzen jeweiliger der zellulären Fluoreszenzmustertypen mittels eines Convolutional Neural Network auf Basis der selektierten Teilbilder des zweiten Bildes und auf Basis der selektierten Teilbilder des ersten Bildes.

Vorgeschlagen wird ferner eine erfindungsgemäße Vorrichtung zur Detektion jeweiliger potentieller Präsenzen jeweiliger unterschiedlicher zellulärer Fluoreszenzmustertypen auf einem biologischen Zellsubstrat aufweisend humane Epitheliomzellen mittels digitaler Bildverarbeitung. Die Vorrichtung weist auf: eine Haltevorrichtung für das biologische Substrat, welches mit einer flüssigen Patientenprobe, welche potentiell primäre Antikörper aufweist, ferner mit einem ersten Fluoreszenzfarbstoff sowie ferner mit sekundären Antikörpern, welche mit einem zweiten Fluoreszenzfarbstoff markiert sind, inkubiert wurde. Die Vorrichtung weist ferner auf: wenigstens eine Bilderfassungseinheit zum Erfassen eines ersten Bildes, welches eine Färbung des Zellsubstrates durch den ersten Fluoreszenzfarbstoff repräsentiert, sowie zum Erfassen eines zweiten Bildes, welches eine Färbung des Zellsubstrates durch den zweiten Farbstoff repräsentiert. Die Vorrichtung ist gekennzeichnet durch wenigstens eine Recheneinheit, welche ausgebildet ist, auf Basis des ersten Bildes jeweilige Bildsegmente zu detektieren, welche jeweils wenigstens eine mitotische Zelle von einer hinreichenden Qualität repräsentieren, wobei eine Mitosezelle dann eine hinreichende Qualität aufweist, wenn sie sich in der Metaphase befindet, ferner Teilbilder des ersten Bildes und dazu korrespondierende Teilbildern des zweiten Bildes auf Basis der detektierten Bildsegmente zu selektieren, welche jeweils wenigstens eine mitotische Zelle (MZX, MZY) einer hinreichenden Qualität repräsentieren, und ferner auf Basis der selektierten Teilbilder des ersten Bildes und der selektierten Teilbilder des zweiten Bildes jeweilige tatsächliche Präsenzen jeweiliger der zellulären Fluoreszenzmustertypen mittels eines Convolutional Neural Network zu detektieren. Die Recheneinheit ist ferner ausgebildet, jeweilige Konfidenzmaße für die jeweiligen tatsächlichen Präsenzen der jeweiligen Fluoreszenzmustertypen mittels des Convolutional Neural Network auf Basis der selektierten Teilbilder des ersten Bildes und der selektierten Teilbilder des zweiten Bildes zu bestimmen, wobei für ein jeweiliges Teilbild-Tupel, welches ein Teilbild des ersten Bildes und ein korrespondierendes Teilbild des zweiten Bildes aufweist, jeweilige Teilbild-Konfidenzmaße für jeweilige tatsächliche Teilbild-Präsenzen jeweiliger zellulärer Fluoreszenzmustertypen mittels des Convolutional Neural Network bestimmt werden, so dass das Convolutional Neural Network zueinander korrespondierende Teilbilder unterschiedlicher Farbkanäle gleichzeitig auswertet. Ferner ist die Recheneinheit ausgebildet, die jeweiligen Konfidenzmaße für die jeweiligen tatsächlichen Präsenzen der jeweiligen Fluoreszenzmustertypen auf Basis der Teilbild-Konfidenzmaße zu bestimmen.

Vorgeschlagen wird ferner eine Recheneinheit, welche ausgebildet ist im Zuge einer digitalen Bildverarbeitung ein erstes Bild entgegenzunehmen, welches eine Färbung eines biologischen Zellsubstrates durch einen ersten Fluoreszenzfarbstoff repräsentiert, wobei das biologische Zellsubstrat humane Epitheliomzellen aufweist, und ein zweites Bild entgegenzunehmen, welches eine Färbung des biologischen Zellsubstrates durch einen zweiten Fluoreszenzfarbstoff repräsentiert. Die Recheneinheit ist dadurch gekennzeichnet, dass sie ferner ausgebildet ist, auf Basis des ersten Bildes jeweilige Bildsegmente zu detektieren, welche jeweils wenigstens eine mitotische Zelle von einer hinreichenden Qualität repräsentieren, wobei eine Mitosezelle dann eine hinreichende Qualität aufweist, wenn sie sich in der Metaphase befindet, auf Basis der detektierten Bildsegmente Teilbilder des ersten Bildes und dazu korrespondierende Teilbilder des zweiten Bildes zu selektieren, welche jeweils wenigstens eine mitotische Zelle (MZX, MZY) einer hinreichenden Qualität repräsentieren, und auf Basis der selektierten Teilbilder des ersten Bildes und der selektierten Teilbilder des zweiten Bildes jeweilige tatsächliche Präsenzen jeweiliger der zellulären Fluoreszenzmustertypen mittels eines Convolutional Neural Network zu detektieren. Die Recheneinheit ist ferner ausgebildet, jeweilige Konfidenzmaße für die jeweiligen tatsächlichen Präsenzen der jeweiligen Fluoreszenzmustertypen mittels des Convolutional Neural Network auf Basis der selektierten Teilbilder des ersten Bildes und der selektierten Teilbilder des zweiten Bildes zu bestimmen, wobei für ein jeweiliges Teilbild-Tupel, welches ein Teilbild des ersten Bildes und ein korrespondierendes Teilbild des zweiten Bildes aufweist, jeweilige Teilbild-Konfidenzmaße für jeweilige tatsächliche Teilbild-Präsenzen jeweiliger zellulärer Fluoreszenzmustertypen mittels des Convolutional Neural Network bestimmt werden, so dass das Convolutional Neural Network zueinander korrespondierende Teilbilder unterschiedlicher Farbkanäle gleichzeitig auswertet. Ferner ist die Recheneinheit ausgebildet, die jeweiligen Konfidenzmaße für die jeweiligen tatsächlichen Präsenzen der jeweiligen Fluoreszenzmustertypen auf Basis der Teilbild-Konfidenzmaße zu bestimmen.

Vorgeschlagen wird ferner eine Datennetzwerkvorrichtung, welche wenigstens eine Datenschnittstelle zum Entgegennehmen eines ersten Bildes aufweist, welches eine Färbung eines biologischen Zellsubstrates durch einen ersten Fluoreszenzfarbstoff repräsentiert, wobei das biologische Zellsubstrat humane Epitheliomzellen aufweist, sowie zum Entgegennehmen eines zweiten Bildes, welches eine Färbung des biologischen Zellsubstrates durch einen zweiten Fluoreszenzfarbstoff repräsentiert. Die Datennetzwerkvorrichtung weist ferner eine erfindungsgemäße Recheneinheit auf.

Vorgeschlagen wird ferner ein Computerprogrammprodukt, welches Befehle umfasst, die bei der Ausführung des Programms durch einen Computer diesen veranlassen, ein Verfahren zur digitalen Bildverarbeitung durchzuführen, aufweisend: Entgegennehmen eines ersten Bildes, welches eine Färbung eines biologischen Zellsubstrates durch einen ersten Fluoreszenzfarbstoff repräsentiert, wobei das biologische Zellsubstrat humane Epitheliomzellen aufweist, Entgegennehmen eines zweiten Bildes, welches eine Färbung des biologischen Zellsubstrates durch einen zweiten Fluoreszenzfarbstoff repräsentiert, Detektieren jeweiliger Bildsegmente, welche jeweils wenigstens eine mitotische Zelle von einer hinreichenden Qualität repräsentieren, auf Basis des ersten Bildes, wobei eine Mitosezelle dann eine hinreichende Qualität aufweist, wenn sie sich in der Metaphase befindet, Selektieren von Teilbildern des ersten Bildes und dazu korrespondierenden Teilbildern des zweiten Bildes auf Basis der detektierten Bildsegmente, welche jeweils wenigstens eine mitotische Zelle einer hinreichenden Qualität repräsentieren, und Detektieren jeweiliger tatsächlicher Präsenzen jeweiliger der zellulären Fluoreszenzmustertypen mittels eines Convolutional Neural Network auf Basis der selektierten Teilbilder des ersten Bildes und der selektierten Teilbilder des zweiten Bildes. Das Verfahren ist gekennzeichnet durch die Schritte: Bestimmen jeweiliger Konfidenzmaße für die jeweiligen tatsächlichen Präsenzen der jeweiligen Fluoreszenzmustertypen mittels des Convolutional Neural Network auf Basis der selektierten Teilbilder des ersten Bildes und der selektierten Teilbilder des zweiten Bildes, wobei für ein jeweiliges Teilbild-Tupel, welches ein Teilbild des ersten Bildes und ein korrespondierendes Teilbild des zweiten Bildes aufweist, jeweilige Teilbild-Konfidenzmaße für jeweilige tatsächliche Teilbild-Präsenzen jeweiliger zellulärer Fluoreszenzmustertypen mittels des Convolutional Neural Network bestimmt werden, so dass das Convolutional Neural Network zueinander korrespondierende Teilbilder unterschiedlicher Farbkanäle gleichzeitig auswertet, sowie schließlich Bestimmen der jeweiligen Konfidenzmaße für die jeweiligen tatsächlichen Präsenzen der jeweiligen Fluoreszenzmustertypen auf Basis der Teilbild-Konfidenzmaße.

Vorgeschlagen wird ferner ein Datenträgersignal, welches das Computerprogrammprodukt überträgt.

Im Folgenden wird die Erfindung anhand spezieller Ausführungsformen ohne Beschränkung des allgemeinen Erfindungsgedankens anhand der Figuren näher erläutert.

Figur 1 Schritte des erfindungsgemäßen Verfahrens gemäß einer bevorzugten Ausführungsform,

Figur 2 eine Prozessierung von Teilbildern durch ein Convolutional Neural Network gemäß einer bevorzugten Ausführungsform,

Figur 3 eine Prozessierung mehrerer Teilbilder durch ein Convolutional Neural Network gemäß einer bevorzugten Ausführungsform,

Figur 4 Schritte für ein Segmentieren des ersten Bildes sowie ein Verifizieren von Konfidenzmaßen auf Basis von ermittelten Helligkeitswerten gemäß einer bevorzugten Ausführungsform,

Figur 5 detaillierte Schritte eines Verfahrens für ein Segmentieren des ersten Bildes gemäß einer bevorzugten Ausführungsform,

Figur 6 eine vorgeschlagene Vorrichtung gemäß einer bevorzugten Ausführungsform,

Figur 7A eine vorgeschlagene Recheneinheit gemäß einer bevorzugten Ausführungsform,

Figur 7B eine vorgeschlagene Datennetzwerkvorrichtung gemäß einer bevorzugten Ausführungsform,

Figur 7C ein vorgeschlagenes Computerprogrammprodukt sowie ein Datenträgersignal gemäß einer bevorzugten Ausführungsform,

Figur 8 ein aus zwei Farbkanälen überlagertes Fluoreszenzbild,

Figur 9 ein erstes Fluoreszenzbild eines ersten Farbkanals,

Figur 10 ein zweites Fluoreszenzbild eines zweiten Farbkanals,

Figur 11 unterschiedliche beispielhafte nukleäre Antikörper-Fluoreszenzmustertypen,

Figur 12a ein segmentiertes Fluoreszenzbild,

Figur 12b eine Legende zu Figur 12a,

Figur 13 eine Aufteilung des Fluoreszenzbildes in Teilbilder sowie eine Hervorhebung unterschiedlicher Mitosezellen,

Figur 14 ein erstes Teilbild, ein zweites Teilbild und ein segmentiertes Teilbild,

Figur 15 unterschiedliche Featuremaps für unterschiedliche Fluoreszenzmustertypen bzw. Fluoreszenzmusterklassen,

Figur 16a das zweite Teilbild aus Figur 14,

Figur 16b eine Featuremap eines bestimmten Fluoreszenzmustertypen wie auch ein zugehöriges Überlagerungsbild,

Figur 16c eine Featuremap für einen anderen bestimmten Fluoreszenzmustertypen als auch ein zugehöriges Überlagerungsbild,

Figur 17a ein erstes weiteres Fluoreszenzbild,

Figur 17b eine hervorgehobene Interphase-Zelle als auch eine hervorgehobene Mitosezelle zu dem Fluoreszenzbild aus Figur 17a,

Figur 18 ein zweites weiteres Fluoreszenzmusterbild,

Figur 19a eine hervorgehobene Interphase-Zelle zu dem Fluoreszenzmusterbild aus Figur 18,

Figur 19b eine hervorgehobene Mitosezelle zu dem Fluoreszenzmusterbild aus Figur 18,

Figur 20 ein drittes weiteres Fluoreszenzmusterbild mit hervorgehobenen Teilbildern und hervorgehobenen Mitosezellen,

Figur 21 ein erstes und ein zweites Teilbild sowie ein segmentiertes Teilbild zu dem Fluoreszenzmusterbild aus Figur 20,

Figur 22 unterschiedliche Featuremaps für die Teilbilder aus der Figur 21,

Figur 23a das zweite Teilbild aus der Figur 21,

Figur 23b eine Featuremap für ein einen bestimmten Fluoreszenzmustertypen sowie ein sich ergebendes Überlagerungsbild zu dem Teilbild aus Figur 23a,

Figur 23c eine vergrößerte Darstellung einer Mitosezelle aus dem zweiten Teilbild aus Figur 23a bzw. aus Figur 20,

Figur 24 eine Klassifikationsschema für unterschiedliche Fluoreszenzmustertypen,

Figur 25 ein Layer eines Convolutional Neural Network zur Erkennung von Fluoreszenzmustertypen gemäß einer bevorzugten Ausführungsform,

Figur 26 ein Convolutional Neural Network für eine Bildsegmentierung gemäß einer bevorzugten Ausführungsform,

Figur 27 eine Tabelle mit experimentellen Ergebnissen.

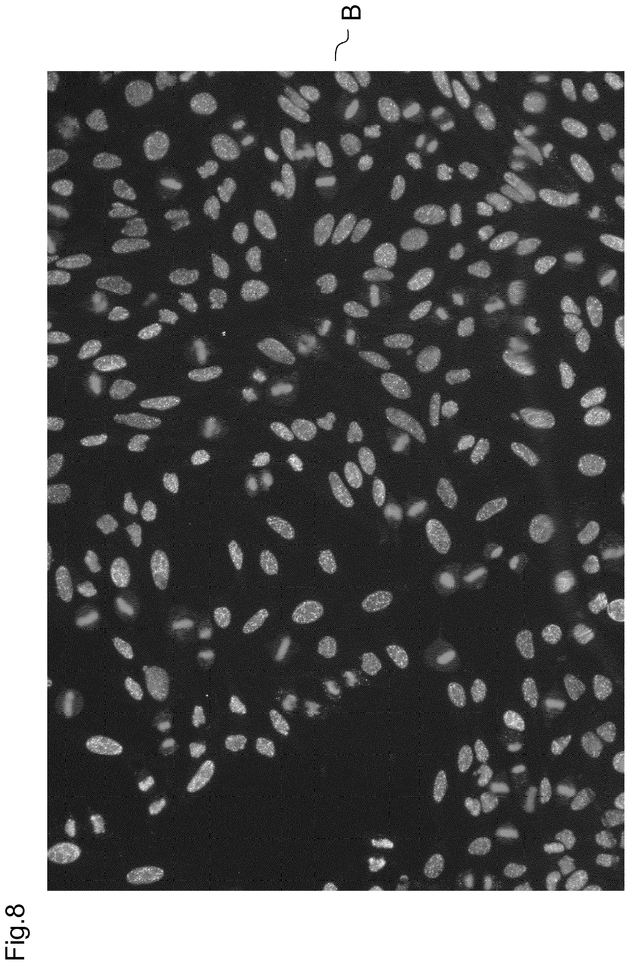

Die Figur 8 zeigt ein aus zwei Farbkanälen überlagertes Fluoreszenzmusterbild B, welches eine Graufärbung aufweist, die eine Färbung eines Zellsubstrates in einem sogenannten Rotkanal als einem ersten Farbkanal durch einen erste Fluoreszenzfarbstoff und auch eine überlagerte Färbung des Zellsubstrates in einem sogenannten Grünkanal als einem zweiten Farbkanal durch einen zweiten Fluoreszenzfarbstoff repräsentiert.

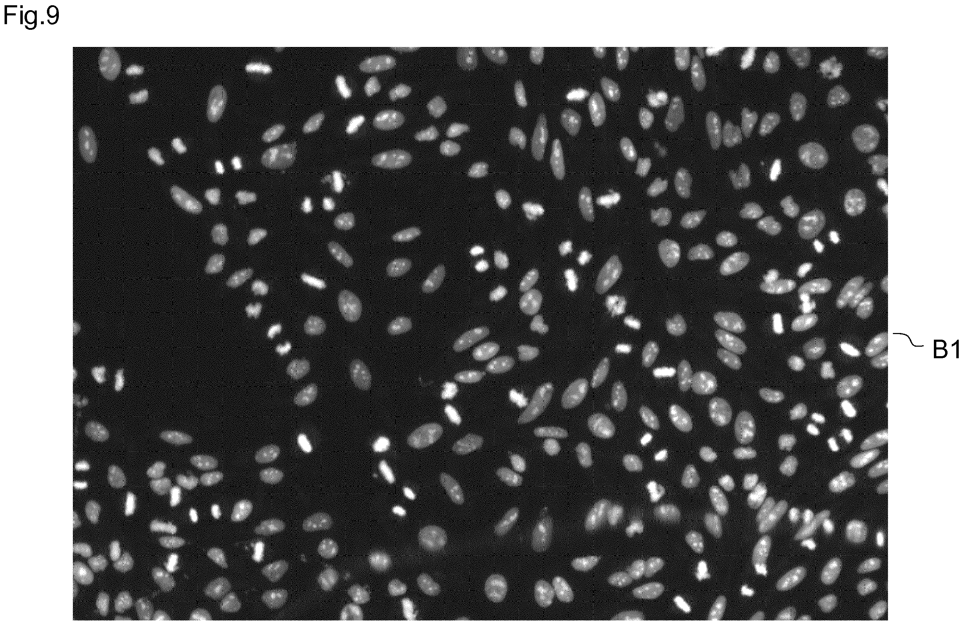

Aufgrund einer Inkubation des hier dargestellten Zellsubstrates, aufweisend HEp-Zellen, mit einem ersten Fluoreszenzfarbstoff, vorzugsweise Propidiumiodid, sind bestimmte Zellbereiche eingefärbt, wie in dem Bild B1 in Figur 9 dargestellt. Die HEp-Zellen sind vorzugsweise sogenannte HEp2-Zellen.

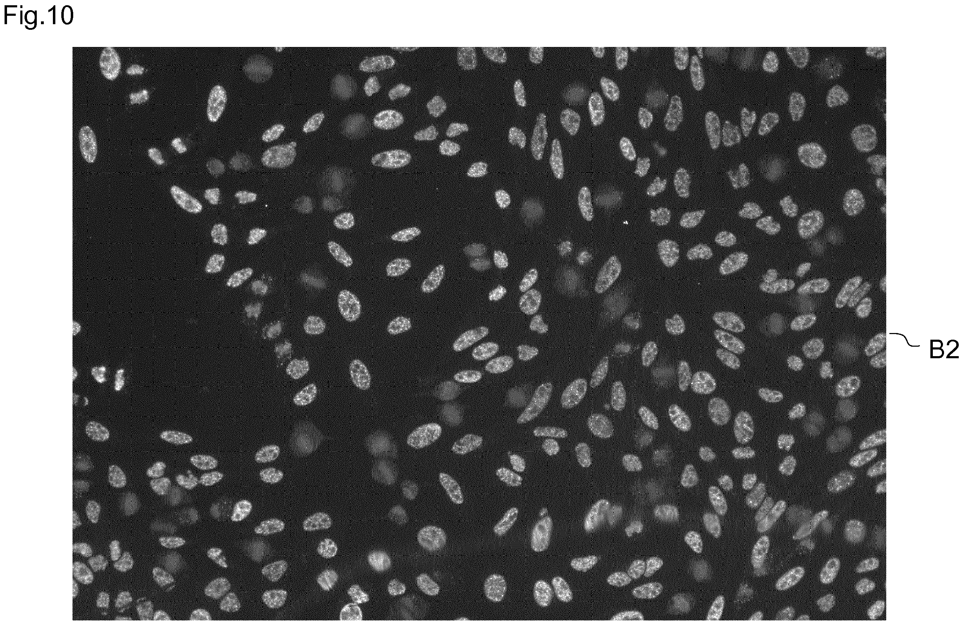

Aufgrund einer Inkubation des hier dargestellten Zellsubstrates mit einem verdünnten patientenspezifischen Blutserum und dadurch resultierender Bindung primärer Autoantikörper ergibt sich aufgrund einer weiteren Inkubation des Zellsubstrates mit sekundären Antikörpern, welche mit einem zweiten Fluoreszenzfarbstoff markiert sind, eine Färbung in einem Grünkanal, welche in der Figur 10 als ein zweites Bild B2 dargestellt ist. Der zweite Fluoreszenzfarbstoff ist vorzugsweise Fluoresceinisothiocyanat (FITC).

Es ist ersichtlich, dass die Färbung durch den ersten Fluoreszenzfarbstoff in dem ersten Bild B1 eine prinzipielle Erkennung von Zellstrukturen bzw. Zellbereichen erlaubt. Ferner erlaubt eine differenzierte musterartige Färbung von Zellbereichen in dem zweiten Bild B2 des Grünkanals eine differenzierte Erkennung einer Präsenz von Fluoreszenzmustertypen.

In dem zweiten Bild B2 aus der Figur 10 muss nicht unbedingt nur ein einzelnes gefärbtes Muster bzw. ein einzelnes antinukleäres Antikörper-Fluoreszenzmuster eines bestimmten Mustertyps vorhanden sei, es können auch mehrere solcher antinukleären Antikörper-Fluoreszenzmustertypen auftreten.

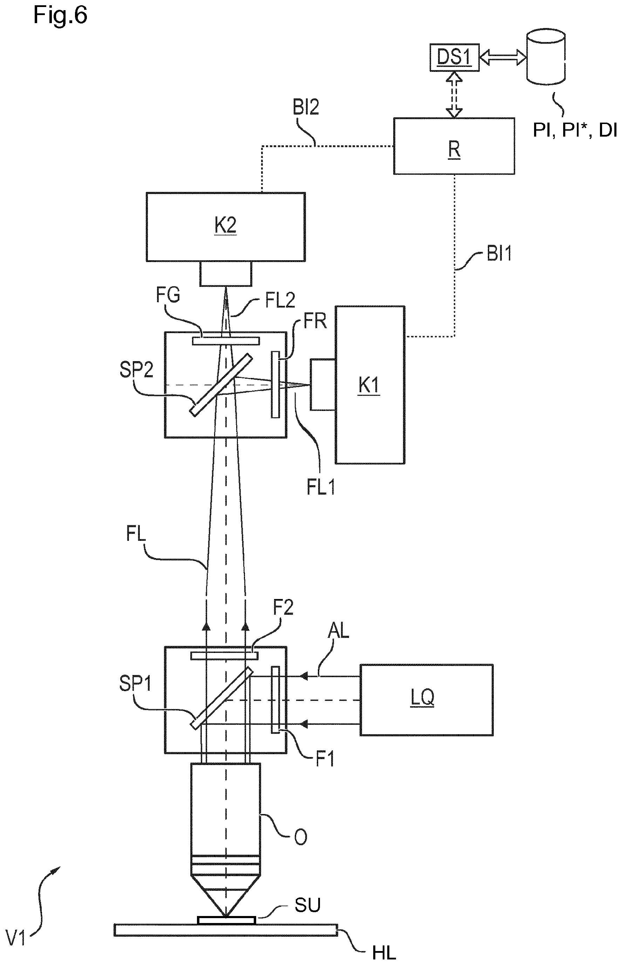

Das erste Bild B1 und das zweite Bild B2 können mittels wenigstens einer Bilderfassungseinheit im Form von einer oder mehrerer Kameras K1 sowie K2 einer Vorrichtung V1 aus Figur 6 erfasst werden. In dem Fall, dass das erste Bild B1 in dem ersten Farbkanal als eine Bildinformation BI1 durch eine erste Bilderfassungseinheit K1 erfasst wird und dass ferner das zweite Bild B2 in dem zweiten Farbkanal eine Bildinformation B2 durch eine separate zweite Bilderfassungseinheit K2 erfasst wird, können die Bilder B1 und B2 vor einer weiteren Verarbeitung räumlich zueinander ausgerichtet werden, damit bestimmte Bildbereiche des ersten Bildes B1 mit ihren entsprechenden Pixelindizes zu bestimmten Bildbereichen des zweiten Bildes mit gleichen Pixelindizes korrespondieren. Es ist jedoch auch möglich, durch eine entsprechende Ausgestaltung der Vorrichtung V1 hinsichtlich optischer Komponenten und optischer Filter die beiden Bilder B1 und B2 mittels einer einzelnen Bilderfassungseinheit bzw. einer einzelnen Kamera zu erfassen, welche einen Sensor aufweist, welcher Bilder in den beiden Farbkanäle getrennt voneinander erfassen kann, beispielsweise also ein Farbsensor.

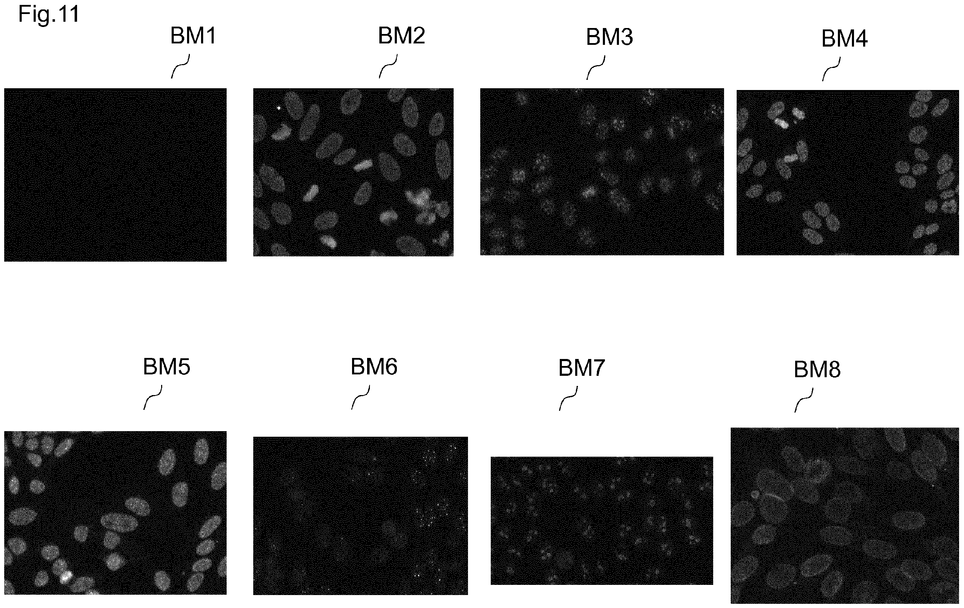

Die Figur 11 zeigt unterschiedliche antinukleäre Antikörper-Fluoreszenzmustertypen BM1,..., BM8. Der Mustertyp BM1 stellt eine sogenannte Negativfärbung dar, in dem Fall, dass keiner der weiteren zu detektierenden antinukleären Antikörper-Fluoreszenzmustertypen vorhanden ist. Dieses entspricht dem Muster AC-0 aus der Figur 24 mit dem hier beispielhaft verwendeten Musterindex n=1. Die beispielhafte Musterklassifikation aus der Figur 24 entstammt der Quelle www.anapatterns.org/trees-full.php.

Das Muster BM2 ist eine sogenannte homogene Färbung bzw. der Mustertyp homogen, welcher in der Figur 24 als AC-1 bezeichnet ist mit dem hier beispielhaft verwendeten Musterindex n=2.

Der Mustertyp des Musters BM3 ist eine sogenannte Zentromer-Färbung, welche in der Figur 24 als AC-3 bezeichnet ist mit dem hier beispielhaft verwendeten Musterindex n=3.

Der Mustertyp BM4 ist eine sogenannte dichte feine Sprenkelung, welcher als Mustertyp AC-2 in der Figur 24 bezeichnet ist mit dem hier beispielhaft verwendeten Musterindex n=4.

Der Mustertyp BM5 ist ein sogenanntes fein- oder grob gesprenkeltes Muster, welches in der Figur 24 als AC4 oder AC5 bezeichnet wird mit dem hier beispielhaft verwendeten Musterindex n=5.

Der Mustertyp BM6 stellt als Mustertyp sogenannte nukleäre Punkte dar, welches in der Figur 24 als Mustertyp AC-6,7 bezeichnet ist mit dem hier beispielhaft verwendeten Musterindex n=6.

Der Mustertyp BM7 ist ein sogenannter nukleolärer Mustertyp, welcher in der Figur 24 als AC-8, 9,10 bezeichnet ist mit dem hier beispielhaft verwendeten Musterindex n=7.

Der Mustertyp BM8 ist ein sogenannter nukleär randständiger bzw. Kernmembranmustertyp, welcher in der Figur 8 als Mustertyp AC-11,12 bezeichnet ist mit dem hier beispielhaft verwendeten Musterindex n=8.

Die unterschiedlichen antinukleären Antikörper-Fluoreszenzmustertypen umfassen somit homogene Muster (AC-1), gesprenkelte Muster (AC-2, 4, 5), ein Zentromermuster (AC-3), nukleäre Punktmuster (AC-6,7), nukleoläre Muster (AC-8, 9,10), als auch den Fluoreszenzmustertypen nukleär randständig (AC-11,12). Eine weitere Fluoreszenzmustertypklasse ist dann die sogenannte Negativklasse (AC-0) im Falle einer Patientenprobe ohne spezifische primäre antinukleären Antikörper.

Das Bild B aus der Figur 8 ist ein beispielhaft gewähltes Fluoreszenzbild, für welches das zweite Bild B2 aus dem Grünkanal in der Figur 10 sowohl eine Präsenz des homogenen Musters (AC-1) als auch fein- oder grob-gesprenkelter Muster (AC-4, AC-5) Muster aufweist. Es wird nun im Folgenden später noch darauf eingegangen, in welcher Weise das hier vorgeschlagene Convolutional Neural Network diese beiden Mustertypen detektieren kann.

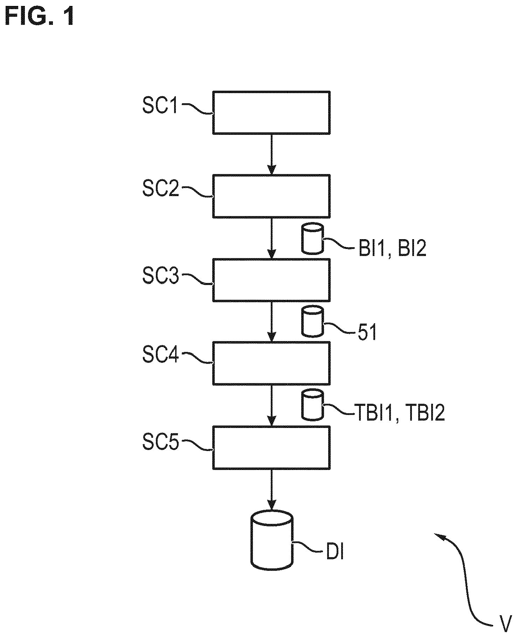

Figur 1 zeigt die wesentlichen Schritte des erfindungsgemäßen Verfahrens V. In einem ersten Schritt SC1 erfolgt das Inkubieren des Zellsubstrates wie zuvor erläutert.

In einem Schritt SC2 erfolgt das Erfassen des ersten Bildes sowie des zweiten Bildes, sodass eine erste Bildinformation BI1 und eine zweite Bildinformation BI2 bereitgestellt wird.

In einem nächsten Schritt SC3 erfolgt auf Basis des ersten Bildes bzw. der ersten Bildinformation BI1 ein Detektieren jeweiliger Bildsegmente, welche jeweils wenigstens eine mitotische Zelle repräsentieren. Hieraus ergibt sich eine sogenannte Segmentierungsinformation SI.

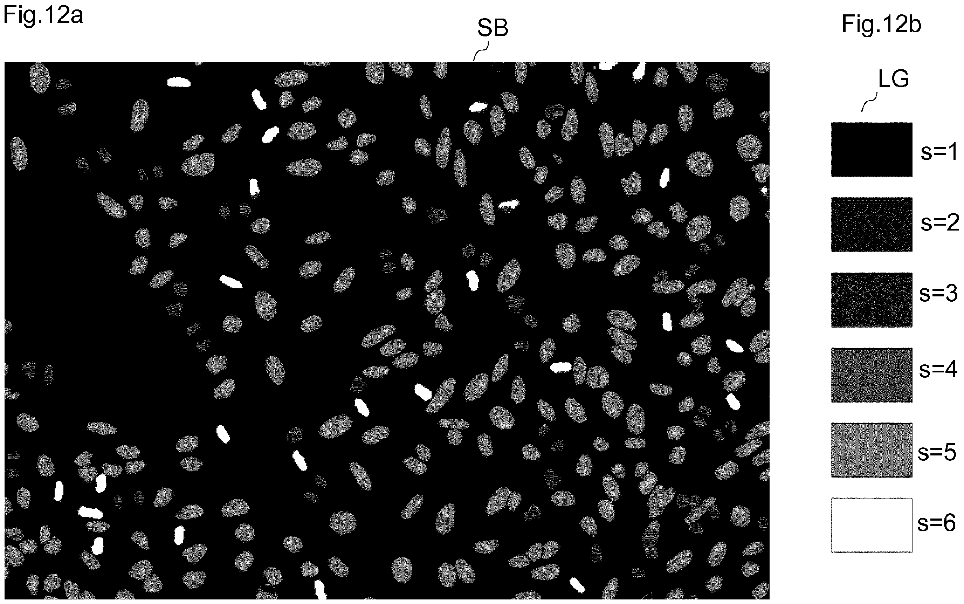

Eine solche Segmentierungsinformation SI ist als ein Bild SB in der Figur 12a dargestellt. Die Figur 12b zeigt hierzu zu den unterschiedlichen Segmentklassen s=1 ...S für S=6 unterschiedliche Grauwertstufen, welche hier die unterschiedlichen Segmentklassen in dem Bild SB aus der Figur 12a indizieren. Die erste Segmentklasse s=1 ist eine sogenannte Hintergrundklasse. Eine zweite Klasse s=2 indiziert einen Zellrand. Eine dritte Klasse s=3 indiziert eine schlechte Mitosezelle bzw. eine Mitosezelle von nicht hinreichender Qualität, alternativ auch eine invalide Mitosezelle genannt. Im Sinne dieser Anmeldung weist eine Mitosezelle dann eine nicht hinreichende Qualität auf bzw. ist dann invalide, wenn sie sich in einem falschen bzw. invaliden Mitosestadium befindet. Eine weitere Klasse s=4 indiziert eine Interphase-Zelle. Eine weitere Klasse s=5 indiziert einen Bereich mit Nucleoli. Eine weitere Klasse s=6 indiziert eine sogenannte gute Mitosezelle bzw. eine Mitosezelle von hinreichender Qualität, auch valide Mitosezellen genannt. Im Sinne dieser Anmeldung weist eine Mitosezelle dann eine hinreichende Qualität auf bzw. ist dann valide, wenn sie sich in einem korrekten bzw. validen Mitosestadium befindet. Ein korrektes bzw. valides Mitosestadium ist die sogenannte Metaphase. Eine Mitosezelle, die sich nicht in einer Methapase befindet, ist also eine falsche bzw. invalide Mitosezelle.

Gemäß der Figur 1 erfolgt dann in einem Schritt SC4 ein Selektieren von Teilbildern des ersten Bildes und dazu korrespondierenden Teilbildern des zweiten Bildes auf Basis der detektierten Bildsegmente, welche jeweils wenigstens eine mitotische Zelle repräsentieren.

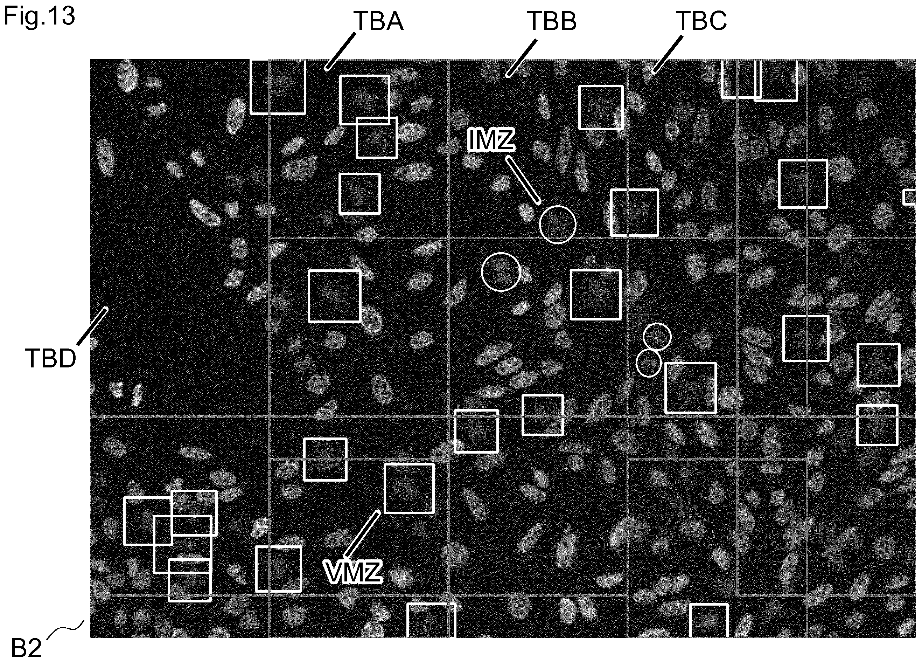

Hierzu zeigt die Figur 13 noch einmal das zweite Bild B2, welches in Teilbilder TBA, TBB, TBC, TBD und auch weitere Teilbilder unterteilt ist. Mittels weißer Quadrate werden Mitosezellen VMZ indiziert, welche vorhanden sind und welche eine hinreichende Qualität aufweisen. Das Teilbild TBD wird hier nicht selektiert, da es keine Mitosezelle MZ aufweist. Weiße Kreise indizieren Mitosezellen IMZ von nicht hinreichender Qualität.

Das Aufteilen des zweiten Bildes B2 in entsprechende Teilbilder kann vorzugsweise derart erfolgen, dass aus einer Lageinformation von Mitosezellen aus dem Bild SB der Figur 12a darauf geschlossen wird, ob innerhalb eines Teilbildes des ersten Bildes und dem dazu korrespondierenden Teil des zweiten Bildes eine solche Mitosezelle von hinreichender Qualität vorhanden ist. Das Detektieren von Bildsegmenten, welche jeweils wenigstens eine mitotische Zelle von hinreichender Qualität repräsentieren, erfolgt auf Basis des ersten Bildes B1 aus der Figur 9.

Die Figur 13 zeigt hierzu eine entsprechende für das zweite Bild B2 übernommene Aufteilung in Teilbilder und detektierte Bildsegmenten, welche die Mitosezellen VMZ von hinreichender Qualität aufweisen.

Vorzugsweise wird hierfür das erste Bild in einer Menge von Teilbildern nach einem vorgegebenen Aufteilungsschema aufgeteilt und dann ein solches Teilbild des ersten Teilbildes ausgewählt, welches solche Bildsegmente aufweist, welche Mitosezellen von hinreichender Qualität repräsentieren, sodass dann zu den selektierten Teilbildern des ersten Bildes auch dazu korrespondierende Teilbilder des zweiten Bildes selektiert werden können.

Gemäß der Figur 1 erfolgt das Selektieren der Teilbilder in dem Schritt SC4, sodass sich eine erste Teilbildinformation TBI1 von selektierten Teilbildern des ersten Bildes und eine zweite Teilbildinformation TBI2 von selektierten Teilbildern des zweiten Bildes, welche zu den Teilbildern des ersten Bildes korrespondieren, ergibt.

In einem Schritt SC5 erfolgt dann das Detektieren der jeweiligen tatsächlichen Präsenzen der jeweiligen antinukleären Antikörper-Fluoreszenzmustertypen mittels eines Convolutional Neural Network CNN auf Basis der selektierten Teilbilder des ersten Bildes und der selektierten Teilbilder des zweiten Bildes. Das Detektionsergebnis wird dann als eine Detektionsinformation DI ausgegeben.

Dadurch, dass auf Bildsegmente mit einer mitotischen Zelle von hinreichender Qualität abgestellt wird und dass ferner eine Selektion der Teilbilder in Abhängigkeit jener detektierten Bildsegmente, welche jeweils wenigstens eine mitotische Zelle einer hinreichenden Qualität repräsentieren, erfolgt, wird sichergestellt, dass die zu betrachtende Mitosezelle bzw. die in dem Teilbild vorhandene Mitosezelle in einem korrekten Stadium ist um als eine valide Mitosezelle hinsichtlich ihrer Färbung ihrer Metaphasenplatte eine valide und sichere Information für die Detektion der unterschiedlichen antinukleären Antikörper-Fluoreszenzmustertypen darzustellen.

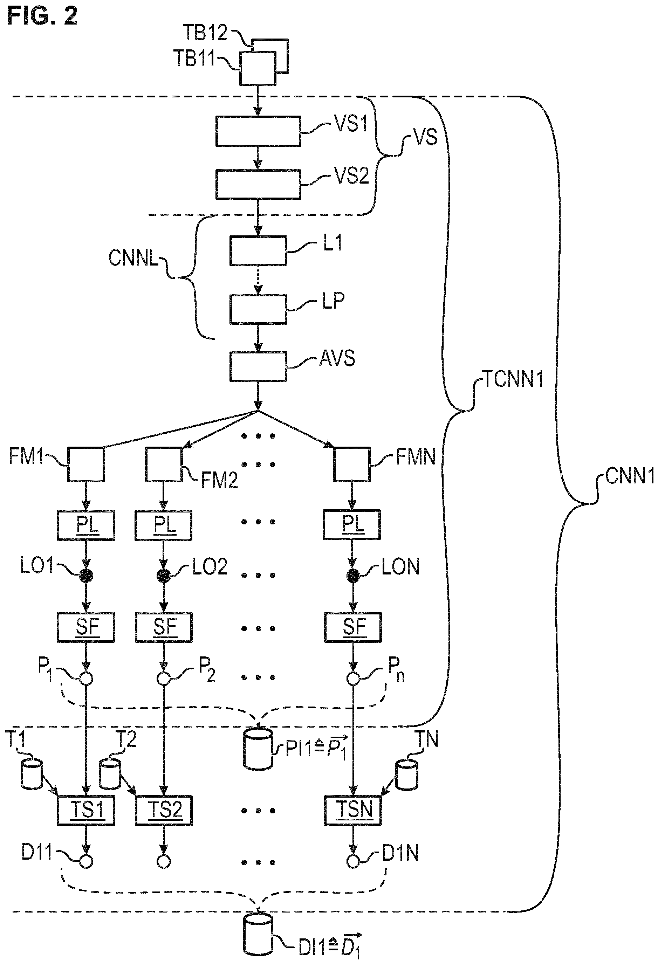

Figur 2 zeigt wesentliche Schritte des Convolutional Neural Network in einer Ausführungsform CNN1 zur Bestimmung einer Detektionsinformation DI1 hinsichtlich einer Präsenz von antinukleären Antikörper-Fluoreszenzmustertypen für ein erstes Teilbild TB11 und ein zweites Teilbild TB12. Die Teilbilder TB11 und TB12 stellen somit ein Teilbild-Tupel dar. Die Teilbilder TB11, TB12 sind vorzugsweise auf einen Wertebereich bzw. Grauwertebereich von 0 bis 1 normiert.

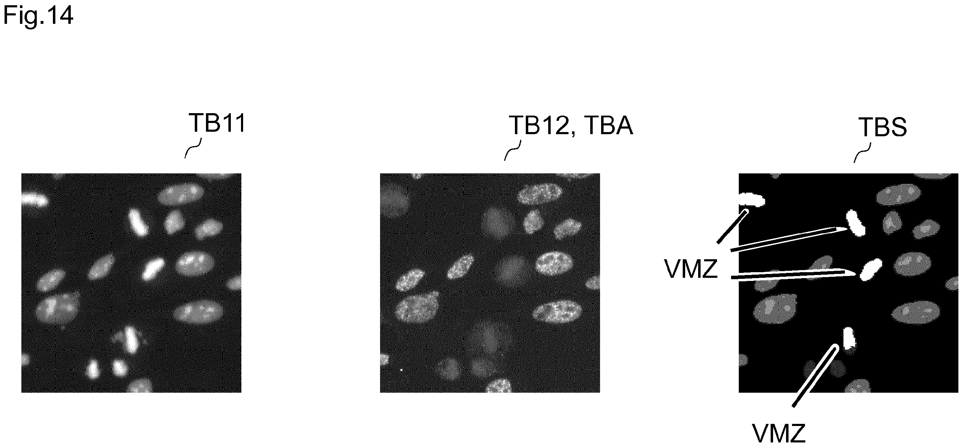

Die Figur 14 zeigt ein beispielhaftes erstes Teilbild TB11 aus dem ersten Bild B1 sowie ein dazu korrespondierendes zweites Teilbild TB12 aus dem zweiten Bild B2. Aus der Figur 13 und der dazugehörigen Darstellung des zweiten Bildes B2 wird ersichtlich, dass es sich bei dem zweiten Teilbild TB12 um das Teilbild TBA handelt.

In der Figur 14 ist ferner ein segmentiertes Teilbild TBS für die hier dargestellten Teilbilder TB11, TB12 darstellt. Klar erkennbar wird, dass die Bereiche VMZ valide Mitosezellen bzw. Mitosezellen von hinreichender Qualität repräsentieren.

Derartige Teilbilder TB11, TB12 werden dann gemäß der Figur 2 durch das Convolutional Neural Network CNN1 prozessiert, um eine Detektionsinformation DI1 zu bestimmen, welche für N unterschiedliche Klassen, hier beispielhaft mit Index n=1... N und N=8, jeweils einen Wert auszugeben, welcher in dem Wertebereich $D_n \in \{0,1\}$ als boolescher Wert die Präsenz des Musters detektiert bzw. indiziert. Die Detektionsinformation DI1 für alle N Mustertypen bzw. Musterklassen mit den Werten D11, ..., D1N entspricht dann einem Vektor

$$\overrightarrow{D_1} = \begin{Bmatrix} D_{11} \\ \vdots \\ D_{1N} \end{Bmatrix} \quad .$$

Gemäß der Figur 2 erfolgt dann ein Prozessieren über mehrere Layer L1,... , LP eines Convolutional Neural Network CNN1, wobei jedes Layer L1,..., LP wenigstens ein Convolutional Layer aufweist.

Das Convolutional Neural Network CNN1 bestimmt dann für jede der N=8 Klassen bzw. N=8 Fluoreszenzmustertypen eine jeweilige Featuremap FM1,..., FMN. Es wird dann auf Basis einer jeweiligen Featuremap FM1,..., FMN ein jeweiliges Konfidenzmaß P1,..., PN bzw. ein jeweiliger Prädiktionswert P1,..., PN bezogen auf eine jeweilige tatsächliche Präsenz des jeweiligen Fluoreszenzmustertyps mit Index n=1... N bestimmt.

Dies erfolgt vorzugsweise dadurch, dass mittels eines Pooling Layers PL bzw. einer Pooling Layer Funktion PL, welches bzw. welche vorzugsweise ein sogenanntes Global Average Pooling durchführt, eine Featuremap FM1 auf einen einzelnen Skalarwert bzw. einen gemittelten Wert, welcher vorzugsweise auch ein Logit genannt wird, als ein Wert LO1 reduziert wird.

Die Logit-Werte LO1,.., LON der N Klassen bzw. Fluoreszenzmustertypen werden dann jeweils einzeln einer sogenannten Sigmoidfunktion SF unterworfen, um für eine jeweilige Klasse n auf Basis der jeweiligen Featuremap FMn mit Index n einen jeweiligen Prädiktionswert bzw. ein jeweiliges Konfidenzmaß Pn mit Index n zu bestimmen. Durch diese Struktur des Convolutional Neural Network ergibt sich also der Vorteil, dass für jeden einzelnen Fluoreszenzmustertypen ein eigener Detektionskanal vorhanden ist und dass nicht nur ein einzelnes Muster bzw. ein einzelner Mustertyp als präsent detektiert werden kann sondern eben auch mehrere Mustertypen gleichzeitig.

Das Bestimmen jeweiliger Konfidenzmaße Pn als eine Prädiktionsinformation bzw. Präsenzinformation PI1 ermöglicht dann eine Ausgabe von Prädiktionswerten bzw. Konfidenzmaßen in Form eines Vektors

$$\overrightarrow{P_1} = \left\{ \begin{matrix} P_1 \\ \vdots \\ P_N \end{matrix} \right\}$$

mit einem Wertebereich $0 \le P_n \le 1$.

Werden solche Konfidenzmaße Pn bestimmt und vorzugsweise auch ausgegeben, so ist dies vorteilhaft, da ein Nutzer bei einer späteren finalen Befundung ein jeweiliges Konfidenzmaß als Indikator für die Präsenz eines jeweiligen Musters verwenden kann.

Der Teil TCNN1 des Convolutional Neural Network CNN1, welcher zur Bestimmung der Konfidenzmaße Pn auf Basis der Teilbilder TB11, TB12 verwendet wird, kann auf jeweilige Teilbild-Tupel TB11, TB12, bestehend aus einem ersten Teilbild des ersten Farbkanals TB11 und einem dazu korrespondierenden zweiten Teilbild des zweiten Farbkanals TB12, jeweils separat angewendet werden, wie später im Detail noch erläutert wird.

Die Figur 2 zeigt ferner weitere Schritte, mittels welcher auf Basis der Konfidenzmaße bzw. Prädiktionswerte, welche als Prädiktionsinformation PI1 zusammengefasst werden können, und mittels welcher Detektionsergebnisse bzw. Detektionswerte D11,..., D1N bestimmt werden können, welche wiederum als Detektionsinformation DI1 zusammengefasst werden können.

Ein Konfidenzmaß Pn kann dann über eine Schwellenwertfunktion TS1 unter Anwendung eines vorzugsweise durch einen Nutzer vorgebbaren Schwellenwertes oder einen auf andere Art vorgegebenen Schwellenwert T1 ausgewertet werden, um die entsprechende Detektionsinformation DI1 zu bestimmen. Vorzugsweise kann der Detektionswert D11 den Wert 1 annehmen, also ein Vorhandensein des Musters mit dem Index n=1 indizieren, wenn das Konfidenzmaß Pn einen Schwellenwert T1 von 0,5 überschreitet.

Eine Anwendung weiterer Schwellenwertoperationen TS1,..., TSN unter Verwendung jeweiliger vorzugsweise für die jeweiligen Muster jeweiliger individueller Schwellenwerte T1,..., TN ermöglicht dann die Ermittlung der Detektionswerte D11,..., D1N.

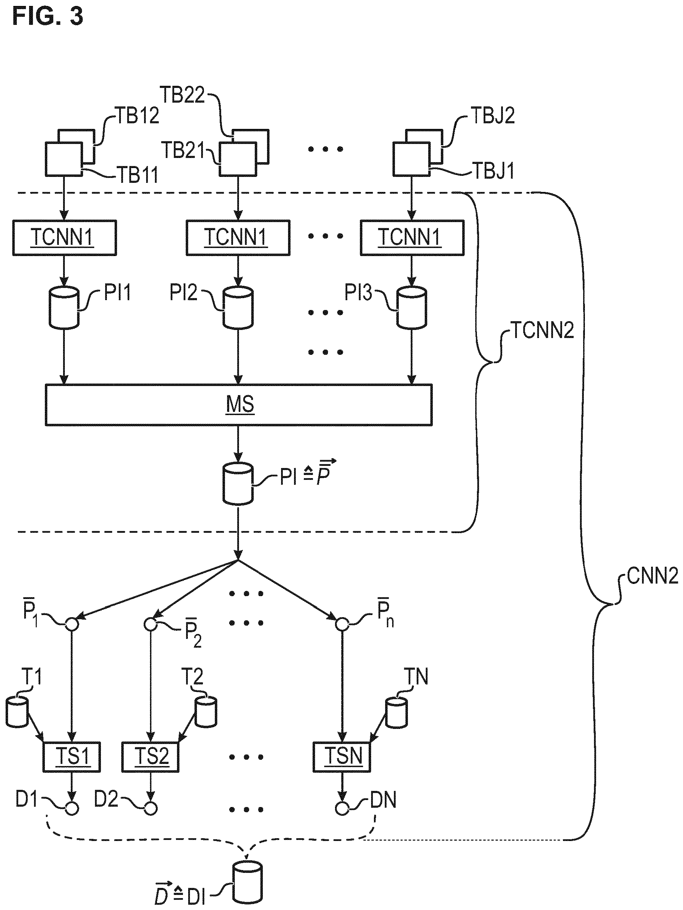

Das Convolutional Neural Network CNN1 aus Figur 2 weist ein partielles Convolutional Neural Network TCNN1 auf, welches später noch in der Figur 3 Anwendung findet.

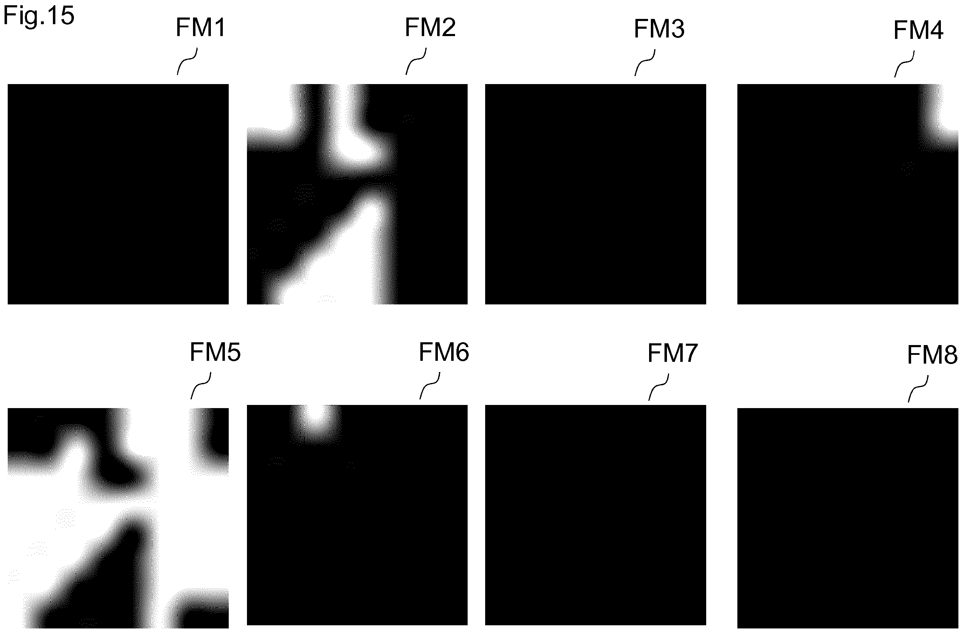

Die Figur 15 zeigt beispielhafte Featuremaps FM1,..., FM8 für die unterschiedlichen Fluoreszenzmustertypen bzw. Beispielmuster, welche in Figur 11 dargestellt sind und Beispiele für die entsprechenden Fluoreszenzmustertypen aus der Figur 24 mit den entsprechenden Bezeichnungen darstellen. Die Featuremap FM2 stellt eine Aktivierung bezüglich eines homogenen Musters für die Teilbilder TB11 und TB12 aus der Figur 14 dar. Das Maß an Helligkeit in der Featuremap FM2 indiziert also ein wahrscheinliches Vorliegen eines homogenen Fluoreszenzmustertypen in dem Teilbild TB12. Ferner stellt die Featuremap FM5 eine wahrscheinliche Präsenz fein gesprenkelter oder grob gesprenkelter Muster in dem entsprechenden Teilbild TB12 dar. Für die weiteren Featuremaps FM1, FM3, FM4, FM6, FM7, FM8 ergibt sich keine oder eine relativ geringe Aktivierung. Die Featuremaps FM1,..., FM8 aus der Figur 15 wurden ursprünglich mit einer Aufllösung von 8x8 Pixeln bestimmt und dann mittels Interpolation auf die Auflösung von 512x512 Pixeln des zweiten Teilbildes TB12 interpoliert.

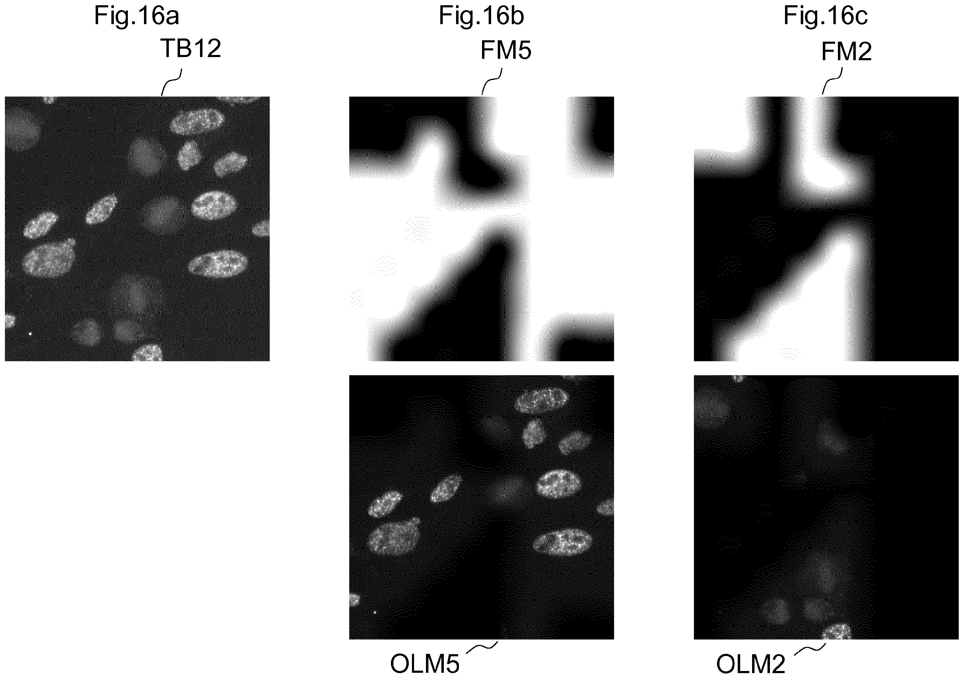

Die Figur 16a zeigt noch einmal das zweite Teilbild TB12 aus der Figur 14 zusammen mit der Featuremap FM5 in der Figur 16b sowie eine Überlagerung dieser Featuremap FM5 über das Teilbild TB12 als eine überlagerte Featuremap OLM5. Es wird hier klar ersichtlich, dass es mittels des vorgeschlagenen Convolutional Neural Network gelungen ist, eine Featuremap FM5 zu erzeugen, welche Zellkerne mit einer feinen bzw. groben Sprenkelung hervorhebt bzw. erkennt.

Die Figur 16c zeigt die Featuremap FM2 aus der Figur 15 für homogene Muster sowie eine über das Teilbild TB12 der Figur 16a überlagerte Featuremap OLM2. Auch hier ist es ersichtlich, dass es gelungen ist mittels des vorgeschlagenen Convolutional Neural Network aus dem Teilbild TB12 jene Region hervorzuheben bzw. zu detektieren, welche Zellkerne mit einer homogenen Färbung bzw. einem homogenen Mustertypen darstellen.

Betrachtet man die Figur 14 und das segmentierte Teilbild TBS, so wird ersichtlich, dass gerade die validen Mitosezellen VMZ jene Zellen sind, welche für die Detektion des homogenen Musters gemäß der Figur 16c in der überlagerten Featuremap OLM2 detektiert werden. Auch wenn prinzipiell auf den Interphase-Zellen, welche in der überlagerten Featuremap OLM5 aus der Figur 16b selektiert wurden, eine homogene Färbung vorhanden sein kann, so wird jedoch diese durch die gesprenkelte Färbung dieser Interphase-Zellen überdeckt, sodass eine Detektion eines homogenen Musters alleine auf diesen Interphase-Zellen aus der Figur 16b in der überlagerten Featuremap OLM5 nicht sicher erfolgen kann. Dadurch jedoch, dass sichergestellt wurde, dass die selektierten Teilbilder TB11 und TB12 aus der Figur 14 auf jeden Fall valide Mitosezellen VMZ aufweisen bzw. das segmentierte Bild TBS solche validen Mitosezellen VMZ aufweist, kann also vorteilhafterweise die homogene Musterfärbung bzw. der homogene Fluoreszenzmustertyp gerade auf diesen Mitosezellen erkannt werden, wie durch eine gemeinsame Betrachtung der überlagerten Featuremap OLM2 aus der Figur 16c und des segmentierten Teilbildes TBS aus der Figur 14 ersichtlich wird.

Die Figur 3 illustriert weitere vorteilhafte Ausgestaltungen des erfindungsgemäßen Verfahrens. Das hier dargestellte Convolutional Neural Network CNN2 verwendet das Teil-Convolution Neural Network TCNN1 aus der Figur 2, um für jeweilige Teilbild-Tupel TB11, TB12,..., TBJ1, TBJ2 mit Tupel-Indexj=1...J jeweilige Teilbild-Konfidenzmaße PI1, ..., PIJ zu bestimmen, wie zuvor in der Figur 2 dargestellt und detailliert erläutert, um dann die jeweiligen tatsächlichen Präsenzen der jeweiligen Fluoreszenzmustertypen und die dazugehörigen jeweiligen Konfidenzmaße auf Basis der Teilbild-Konfidenzmaße als eine Präsenzinformation bzw. Prädiktionsinformation PI zu bestimmen. Hierbei entspricht ein Teilbild-Konfidenzmaß PI einem Vektor $\vec{P}_j$ mit Index j. Die Information PI entspricht hierbei einem Vektor

$$\vec{P} = \begin{Bmatrix} \bar{P}_1 \\ \vdots \\ \bar{P}_N \end{Bmatrix}$$

mit einem jeweiligen Konfidenzmaß $\vec{P}_n$ für einen jeweiligen Fluoreszenzmustertypen mit Index n.

Die sich ergebenden Teilbild-Konfidenzmaße $\vec{P}_j$ mit Indexj=1... J werden dann in einem Mittelungsschritt MS über alle selektierten j=1...J Teilbilder bzw. Teilbild-Tupel gemittelt gemäß

$$\vec{P} = \frac{1}{J}\sum_{i=1}^{J} \vec{P}_i \ .$$

Diese Konfidenzmaße

$$\vec{P} = \begin{Bmatrix} \bar{P}_1 \\ \vdots \\ \bar{P}_N \end{Bmatrix}$$

können dann als die Prädiktionsinformation bzw. eine Konfidenzinformation PI ausgegeben werden.

Diese Ausgestaltung des erfindungsgemäßen Verfahrens ist vorteilhaft, da somit ein Convolutional Neural Network CNN2 nicht gleichzeitig alle Informationen eines gesamten ersten und eines gesamten zweiten Bildes B1, B2 aus den Figuren 9 und 10 auf einmal prozessieren muss, sondern in separaten, voneinander getrennten Prozessierungspfaden die Teilbild-Tupel jeweils für sich ausgewertet werden können.

Es können dann die ermittelten Konfidenzmaße PI bzw.

$$\vec{P} = \begin{Bmatrix} \bar{P}_1 \\ \vdots \\ \bar{P}_N \end{Bmatrix}$$

in jeweiligen Schwellenwert-Beurteilungsschritten TS1,..., TSN unter Verwendung vorzugsweise vorgebbarer oder aber auf andere Art vorgegebener Schwellwerte T1,..., TN bewertet werden, um eine entsprechende Detektionsinformation DI mit entsprechenden Detektionswerten D1,..., DN mit Index n=1... N als

$$\overrightarrow{D_1} = \begin{Bmatrix} D_1 \\ \vdots \\ D_N \end{Bmatrix}$$

auszugeben.

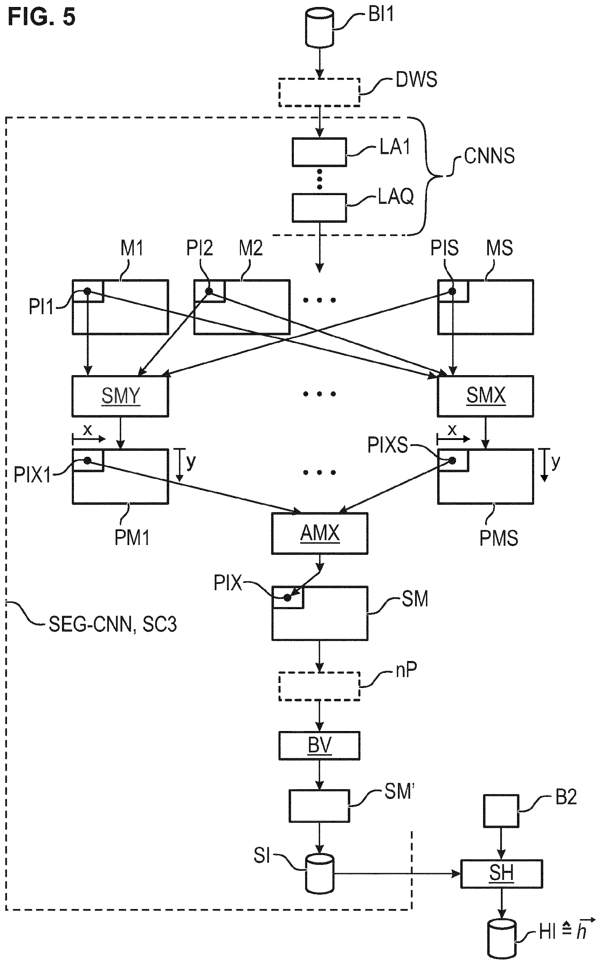

Die Figur 5 zeigt Details des Schrittes SC3 aus der Figur 1 zum Detektieren von Bildsegmenten, welche jeweils wenigstens eine mitotische Zelle von hinreichender Qualität repräsentieren, auf Basis des ersten Bildes BI1. Dieses erfolgt mittels eines Segmentierungs-Convolutional Neural Network SEG-CNN. Das Segmentierungs-Convolutional Neural Network SEG-CNN weist Schritte bzw. Layer LA1, ..., LAQ auf, welche wiederum als ein Convolutional Neural Network aufgefasst werden können. Die Segmentierung erfolgt also mittels eines weiteren Convolutional Neural Network SEG-CNN, welches sich von dem Convolutional Neural Network CNN2 zum Detektieren jeweiliger tatsächlicher Präsenzen jeweiliger der zellulären Fluoreszenzmustertypen mittels eines Convolutional Neural Network auf Basis der selektierten Teilbilder des ersten Bildes und der selektierten Teilbilder des zweiten Bildes unterscheidet. Die Aufteilung der Segmentierungsaufgabe und der Detektionsaufgabe auf zwei unterschiedliche Convolutional Neural Networks ist vorteilhaft, da das Convolutional Neural Network zur Detektion nicht auch zum Zwecke der Segmentierung trainiert werden muss und somit das Convolutional Neural Network zum Zwecke der Segmentierung besonders gezielt trainiert werden kann.

Gemäß der Figur 5 wird das erste Bild, z.B. das Bild B1 aus der Figur 9, bzw. die entsprechende Bildinformation BI1 einem Convolutional Neural Network CNNS zum Zwecke einer Segmentierung zugeführt.

In der Figur 5 ist das Segmentierungs-Convolutional Neural Network SEG-CNN dargestellt, welches mittels mehrerer Layer LA1, LAQ eine Segmentierung eines Eingangsbildes herbeiführt. Vorzugsweise erfolgt hierbei eine Normierung der Bilddaten BI1 auf einem Wertebereich von 0 bis 1. Vorzugsweise erfolgt ferner in einem optionalen Schritt DWS ein sogenanntes Downscaling. Vorzugsweise weist ein erstes Bild der Bilddaten BI1 eine Dimensionalität von 2400×1700 Pixeln auf. Dieses erste Bild kann dann durch ein sogenanntes Downsampling auf eine Größe von 800×800 Pixeln reduziert werden. Die weiteren Prozessierungsschritte sind hier im Detail für den beispielhaften Fall dargelegt, dass ein Downscaling DWS des ersten Bildes auf eine Größe von 800×800 Pixeln erfolgte. Dieses ist aber nicht zwingend notwendig, das Segmentierungs-Convolutional Neural Network SEG-CNNkann auch in seiner Dimensionierung so ausgestaltet werden, dass es das erste Bild mit einer Größe von 2400x1700 Pixeln ohne ein Downscaling verarbeiten kann.

Das Convolutional Neural Network CNNS weist mehrere Layer LA1,..., LAQ auf, wobei jedes dieser Layer wenigstens ein Convolutional Layer aufweist.

Das Convolutional Neural Network CNNS erzeugt dann für jede der Segmentklassen s=1... S mit hier beispielsweise S=6 Klassen, wie in Figur 12b durch die Legende indiziert, eine jeweilige Aktivierungsmap M1,..., MS. Die Aktivierungsmaps M1, ..., MS weisen jeweils vorzugsweise eine gleiche Größe bzw. Auflösung von vorzugsweise 800x800 Pixeln auf, wie das erste Bild bzw. die ersten Bilddaten BI1 nach dem Downscaling DWS.

Die Aktivierungsmap M1 für die erste Segmentklasse s=1 indiziert in ihrem ersten Pixel PI1 mit dem dort vorhandenen Wert, zu welchem Grade das dazu korrespondierende Pixel des ersten Bildes B1 bzw. der Bildinformation BI1 zu dieser ersten Segmentklasse gehört. Entsprechende einzelne Pixel PI1,...PIS der Aktivierungsmaps M1,...MS indizieren jeweils, zu welchem Grade das an gleicher Stelle lokalisierte Pixel des ersten Bildes B1 bzw. der ersten Bildinformation BI1 zu der entsprechenden Klasse gehört. Durch Verknüpfung der Werte der Aktivierungsmaps M1, M2,..., MS an der gleichen Pixelstelle bzw. der entsprechenden Werte dieser korrespondierenden Pixelstelle PI1, PI2,..., PIS mittels einer sogenannten Softmaxfunktion SMX kann dann eine Wahrscheinlichkeitsmap PM1 erstellt werden, um für eine entsprechende Pixelposition PIX1 einen Wert zu generieren, welcher indiziert, mit welcher Wahrscheinlichkeit in einem Wertebereich von 0 bis 1 das korrespondierende Pixel des ersten Bildes B1 zu der ersten Klasse mit s=1 gehört. Entsprechend kann dies für die weiteren S Klassen mit s=1...S mittels einer jeweils separat angewandten Softmaxfunktion SMX für entsprechende Pixel bestimmt und entsprechende Wahrscheinlichkeitsmaps PMs für die s=1...S Segmentklasen eingetragen werden. Die hier dargestellten Achsenverläufe x sowie y indizieren jeweilige Indizes für die entsprechenden Pixelpositionen innerhalb der Wahrscheinlichkeitsmaps PM1, ..., PMS. Die Wahrscheinlichkeitsmaps PM1, ..., PMS weisen vorzugsweise eine gleiche Größe bzw. Auflösung von vorzugsweise 800x800 Pixeln auf wie das erste Bild bzw. die ersten Bilddaten BI1 nach dem Downscaling DWS und wie die Aktivierungsmaps M1, ... , MS.

Es wird dann eine Segmentierungsmap SM bestimmt, welche an einer entsprechenden Pixelposition PIX1 einen Wert aus dem Wertbereich $s \in \{1, ...,S\}$ aufweist und indiziert, zu welcher Segmentklasse s=1...S das entsprechende Pixel dieser Position PIX1 gehört. Hierfür erfolgt eine Verknüpfung der entsprechenden Pixelwerte der korrespondierenden Pixelposition PIX1 über alle Wahrscheinlichkeitsmaps PM1,..., PMS mittels einer Argmax-Funktion. Ein Pixel der Segmentierungsmap SM mit dem Index x, y enthält dann also jenen Indexwert jener Wahrscheinlichkeitsmap PM1,..., PMS, für welche der entsprechende Wahrscheinlichkeitswert an der Pixelposition am höchsten war. Dieses kann bestimmt werden gemäß

$$SM(x,y) = \underset{s \in \{1 \dots S\}}{argmax} \; PM_s(x,y) \, .$$

Die Segmentierungs-Map SM enthält dann also für jedes einzelne Pixel einen Wert aus dem Wertebereich s=1... S, welcher das entsprechende Pixel einer jeweiligen Segmentklasse zuordnet.

Die Segmentierungs-Map SM weist vorzugsweise eine gleiche Größe bzw. Auflösung von vorzugsweise 800×800 Pixeln auf wie das erste Bild bzw. die ersten Bilddaten BI1 nach dem Downscaling DWS, ebenso wie die Aktivierungsmaps M1, ..., MS und ebenso wie die Wahrscheinlichkeitsmaps PM1, ..., PMS.

In dem Fall, dass vor der Segmentierung ein sogenanntes Downscaling des ersten Bildes B1 bzw. der ersten Bildinformation BI in einem optionalen Schritt DWS erfolgte, kann dann vorzugsweise in einem vorzugsweise durchzuführenden Schritt UP ein sogenanntes Upscaling der Segmentierungs-Map SM von vorzugsweise 800x800 Pixeln zurück zur ursprünglichen Bildauflösung des Bildes B1 erfolgen, beispielsweise 2400 x 1700 Pixel.

Die Segmentierungs-Map SM kann dann zum Zusammenführen von Teilflächen bzw. Segmentflächen mittels digitaler Bildverarbeitung in einem Bildverarbeitungsschritt BV durch übliche Bildverarbeitungsmaßnahmen wie Eroding, Delation und/oder Contour Matching weiterverarbeitet werden, um dann zu einer modifizierten Segmentierungs-Map SM' zu gelangen, welche als eine Segmentierungsinformation SI ausgegeben werden kann. Eine solche Information mit einer Segmentierung SI ist in der Figur 12a dargestellt.

Die Figur 5 illustriert, in welcher Weise das Segmentieren des ersten Bildes in Bildsegmente unterschiedlicher Segmentklassen erfolgen kann, wobei die Segmentklassen wie in der Figur 12b illustriert gewählt werden können. Es repräsentiert vorzugsweise zumindest eine erste Segmentklasse ein Zellstadium einer Zelle und es repräsentiert ferner zumindestens eine zweite Segmentklassen einen Zellbereich innerhalb einer Zelle.

Die Segmentierungsinformation SI, wie beispielhaft als das Segmentierungsbild SB in der Figur 12a dargestellt, kann dann genutzt werden, um basierend auf einem oder mehreren Bildsegmenten wenigstens einer bestimmten Segmentklasse wenigstens einen Helligkeitswert für wenigstens einen Fluoreszenzmustertyp zu bestimmen und um dann wenigstens ein Konfidenzmaß eines Fluoreszenzmustertypes auf Basis wenigstens eines solchen Helligkeitswertes zu verifizieren.

Hierzu zeigt die Figur 5 die Möglichkeit, basierend auf dem zweiten Bild B2 und basierend auf der Segmentinformation SI, wenigstens einen Helligkeitswert für wenigstens einen Fluoreszenzmustertyp aus dem zweiten Bild B2 zu extrahieren. Dies erfolgt in einem Schritt SH zur Helligkeitsbestimmung.

Es erfolgt also gemäß der Figur 5 eine Bestimmung eines Helligkeitswertes für wenigstens einen Fluoreszenzmustertypen basierend auf einem oder mehreren Bildsegmenten wenigstens einer bestimmten Segmentklasse sowie ein Verifizieren des Konfidenzmaßes des Fluoreszenzmustertypen auf Basis des Helligkeitswertes des Fluoreszenzmustertypen.

Für die unterschiedlichen n=1... N Klassen können dann entsprechende Helligkeitswerte $h_n$ mit Index n=1..N als Vektor

$$\vec{h} = \begin{Bmatrix} h_1 \\ \vdots \\ h_N \end{Bmatrix}$$

bestimmt und als eine Helligkeitsinformation HI ausgegeben werden.

Wird beispielsweise auf einen Helligkeitswert für ein Muster "dicht feingesprenkelt" (AC-2) des Mustertyps n=4 abgestellt, so kann eine Helligkeitswertermittlung in der Weise erfolgen, dass die Interphase-Zellen der Segmentklasse s=4, welche auch in der Figur 12a eingezeichnet sind, mit ihren Helligkeitswerten an den entsprechenden korrespondierenden Stellen des Bildes B2 aus der Figur 10 herangezogen werden. Es kann dann beispielsweise für eine jede Interphase-Zelle bzw. jede Segmentfläche mit Index s=4 das 0,65 Quantil der Helligkeitswerte für sich bestimmt werden und dann die sich ergebenden mehreren Quantil-Helligkeitswerte der mehreren Interphase-Zellen noch mal mittels Mittelwertbildung gemittelt werden, um einen Helligkeitswert $h_4$ für das Fluoreszenzmuster mit Index n=4 zu bestimmen.

Für sogenannte fein oder grob gesprenkelte Muster des Typs AC-4, AC-5 mit Index n=5 kann dann beispielsweise auf ein 0,65-Quantil der Helligkeitswerte einer Interphase-Zelle bzw. einer entsprechenden Segmentfläche der Klasse s=4 abgestellt werden und dann über diese Helligkeitswerte zur Bestimmung eines Helligkeitswertes $h_5$ für das

Fluoreszenzmuster mit Index n=5 gemittelt werden.

Für sogenannte homogene Muster (AC-1) des Typs mit Index n=2, wie auch in Figur 11 anhand des Beispielmusters BM2 illustriert, kann dann beispielsweise zunächst für jede valide Mitose-Zelle bzw. jede entsprechende Segmentfläche der Klasse s=6 ein 0,5-Quantil ermittelt werden und dann über die Quantilwerte aller validen Mitosezellen gemittelt werden, um einen Helligkeitswert $h_2$ für das Fluoreszenzmuster mit Index n=2 zu bestimmen.

Derart ermittelte Helligkeitswerte

$$\vec{h} = \begin{Bmatrix} h_1 \\ \vdots \\ h_N \end{Bmatrix}$$

können dann also Helligkeitsinformation HI bereitgestellt werden.

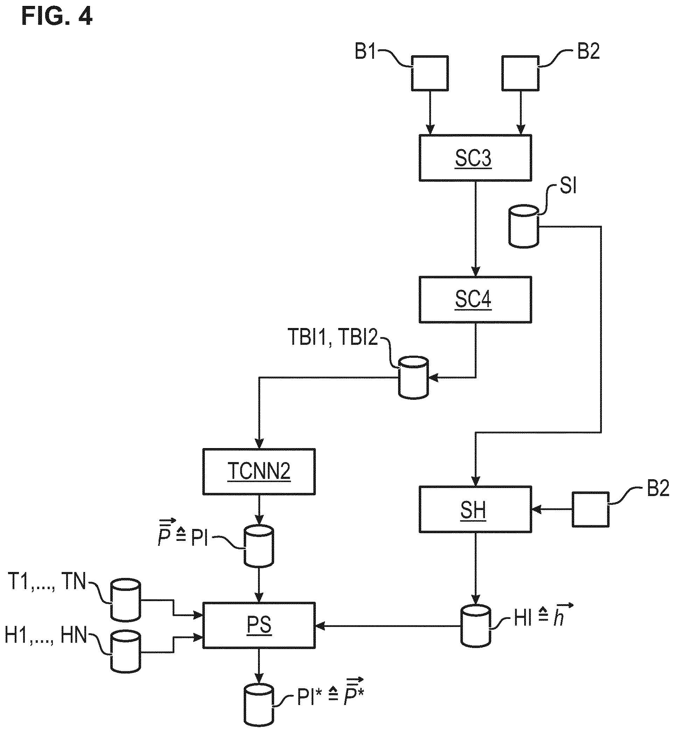

Die Figur 4 illustriert hierzu ein beispielhaftes Vorgehen, bei welchem auf Basis des ersten Bildes B1 und des zweiten Bildes B2 mittels eines Teil-Convolutional Neural Network TCNN2, welches in der Figur 3 indiziert ist, ein Vektor

$$\vec{\bar{P}} = \begin{Bmatrix} \bar{P}_1 \\ \vdots \\ \bar{P}_N \end{Bmatrix}$$

mit entsprechenden Konfidenzmaßen für die entsprechenden n=1... n Klassen ermittelt wird. Dieses wird dann als Konfidenzmaßinformation bzw. Prädiktionsinformation PI bereitgestellt.

Hierfür verwendet das Teil-Convolutional Neural Network TCNN2 jene Teilbilder bzw. jene Teilbildinformation TBI1, TBI2, welche auf Basis des ersten Bildes B1 mittels des Segmentationsschrittes SC3 und der daraus gewonnenen Segmentierungsinformation SI als auch des zweiten Bildes B2 ermittelt wurde. In das Teil-Convolutional Neural Network TCNN2 gehen also jene Teilbilder ein, welche durch den Segmentationsschritt SC3 und den Selektionsschritt SC4 aus dem ersten Bild B1 und dem zweiten Bild B2 selektiert wurden.

Die Segmentierungsinformation SI wird dann in dem Schritt SH zur Bestimmung der Helligkeitswerte, welcher bereits in der Figur 5 dargelegt wurde, verwendet, um auf Basis der Helligkeitswerte des zweiten Bildes B2 die Helligkeitsinformation HI zu ermitteln und bereitzustellen, welche eben auch als Vektor

$$\vec{h} = \begin{Bmatrix} h_1 \\ \vdots \\ h_N \end{Bmatrix}$$

beschrieben werden kann.

In einem Prüfungsschritt PS erfolgt dann das Verifizieren der Konfidenzmaßinformation PI auf Basis der Helligkeitsinformation HI.

Hierbei werden dann die Werte des Konfidenzmaßes

$$\vec{\bar{P}} = \begin{Bmatrix} \bar{P}_1 \\ \vdots \\ \bar{P}_N \end{Bmatrix}$$

anhand eines bzw. mehrerer Schwellwerte bezogen auf die Konfidenzmaßwerte in Form von Schwellenwerten T1,..., TN verifiziert als auch vorzugsweise anhand von Helligkeitsschwellenwerten in H1,..., HN, welche auf die Helligkeitswerte

$$\vec{h} = \begin{Bmatrix} h_1 \\ \vdots \\ h_N \end{Bmatrix}$$

angewendet werden.

Hierdurch wird dann also eine Ausgabe verifizierter Konfidenzmaße PI* ermöglicht. Die Schwellenwerte T1,..., TN sind vorgegeben. Die Schwellenwerte H1,..., HN sind vorgegeben. Vorzugsweise können die Schwellenwerte T1,...,

TN durch einen Nutzer vorgegeben werden, um Einfluss auf die Auswertung zu nehmen. Vorzugsweise können die Schwellenwerte H1,..., HN durch einen Nutzer vorgegeben werden, um Einfluss auf die Auswertung zu nehmen. Die Schwellenwerte H1, ..., HN sind individuelle Schwellenwerte je Fluoreszenzmustertyp. Die Ermittlung der verifizierten Konfidenzmaße PI* als Vektor

$$\overrightarrow{\overline{P^*}} = \left\{ \begin{array}{c} \overline{P^*}_1 \\ \vdots \\ \overline{P^*}_N \end{array} \right\}$$

erfolgt dann vorzugsweise gemäß

$$\overline{P^*}_n = \begin{cases} 1 & fuer\ \bar{P}_n > T_n\ \cap\ h_n > HS_n \\ 0 & sonst \end{cases}.$$

Das hier vorgeschlagene Vorgehen ist vorteilhaft, da die Konfidenzmaße zwar zunächst auf Basis des ersten Bildes und des zweiten Bildes bzw. der entsprechenden Teilbilder bestimmt werden, in denen ein Convolutional Neural Network die Konfidenzmaße PI ermittelt, aber nun auch explizit noch Helligkeitswerte HI aus dem zweiten Bild bzw. dem gesamten zweiten Bild, insbesondere ohne eine Reduktion auf Teilbilder, ermittelt werden, um das Konfidenzmaß bzw. die Konfidenzmaße PI zu verifizieren. Hierdurch wird eine noch genauere Detektion von Fluoreszenzmustertypen ermöglicht. Ein Vorteil ist hierbei insbesondere, dass das gesamte zweite Bild B2 hinsichtlich von Helligkeitswerten in entsprechenden Segmentklassen betrachtet wird, so dass hierdurch nicht nur eine Helligkeit von Zellen bzw. Segmentflächen eines einzelnen Teilbildes betrachtet werden, sondern dass eben auch Segmentflächen außerhalb selektierter Teilbilder in Betracht gezogen werden. Dieses bewirkt, dass ein möglicher Helligkeitsartefakt auf einer einzelnen Zelle bzw. einer einzelnen Segmentfläche eines Teilbildes sich weniger stark auf die Bestimmung der Helligkeitswerte und somit der Bestimmung der verifizierten Konfidenzmaße auswirkt, da auf Konfidenzmaße eines Teilbildes Helligkeitswerte aus Segmentflächen auch außerhalb des Teilbildes angewendet werden und ein Helligkeitsartefakt einer einzelnen Segmentfläche weniger stark gewichtet wird, um die verifizierten Konfidenzmaße zu bestimmen.

Die Figur 2 zeigt Vorverarbeitungsschritte VS, VS1, VS2, welche zur Vorverarbeitung des ersten Teilbildes TB11 und des zweiten Teilbildes TB12 verwendet werden können. In dem ersten Vorverarbeitungsschritt VS1 erfolgt eine Faltung des ersten Teilbildes TB11 und des zweiten Teilbildes TB12 als Eingangs-Featuremaps mit vorzugsweise acht verschiedenen Faltungskernel, wobei kein Striding durchgeführt wird und wobei die Dimensionalität der Teilbilder TB11, TB12 mit einer beispielhaften Dimensionalität von 512x512 Bildpixeln beigehalten wird. Die Wahl der Anzahl von acht Faltungskernel muss nicht notwendigerweise mit der Anzahl der N=8 Fluoreszenzmustertyp bzw. Fluoreszenzmusterklassen übereinstimmen.

In dem weiteren Vorverarbeitungsschritt VS2 erfolgt dann eine sogenannte Batch Normalization, wobei die Anzahl der Featuremaps, hier beispielsweise acht Featuremaps, beibehalten wird.

Die sich aus dem Schritt VS2 ergebenden acht Featuremaps werden dann in einer Abfolge von P unterschiedlichen Layern L1,..., LP mit Index p=1... P und hier beispielhaft P=6 prozessiert. Am Ende des Layers L1 ergibt sich eine Menge von beispielsweise R Featuremaps FMA1,.., FMAR mit Index r=1...R. In diesem Beispiel ist vorzugsweise der Parameter R=11. Die Anzahl R der Featuremaps am Ende des ersten Layers L1 kann von der Anzahl der Fluoreszenzmustertypen N abweichen.

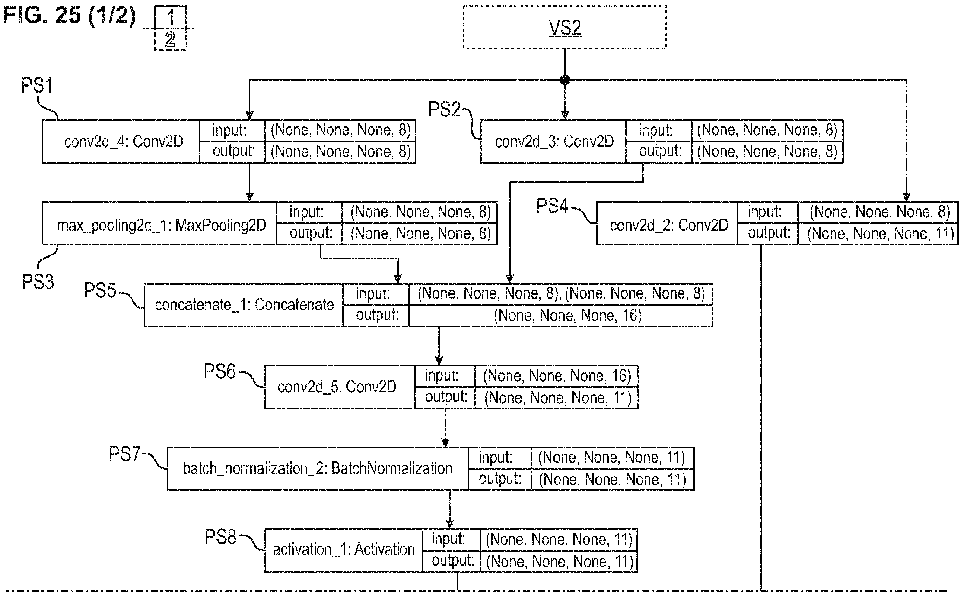

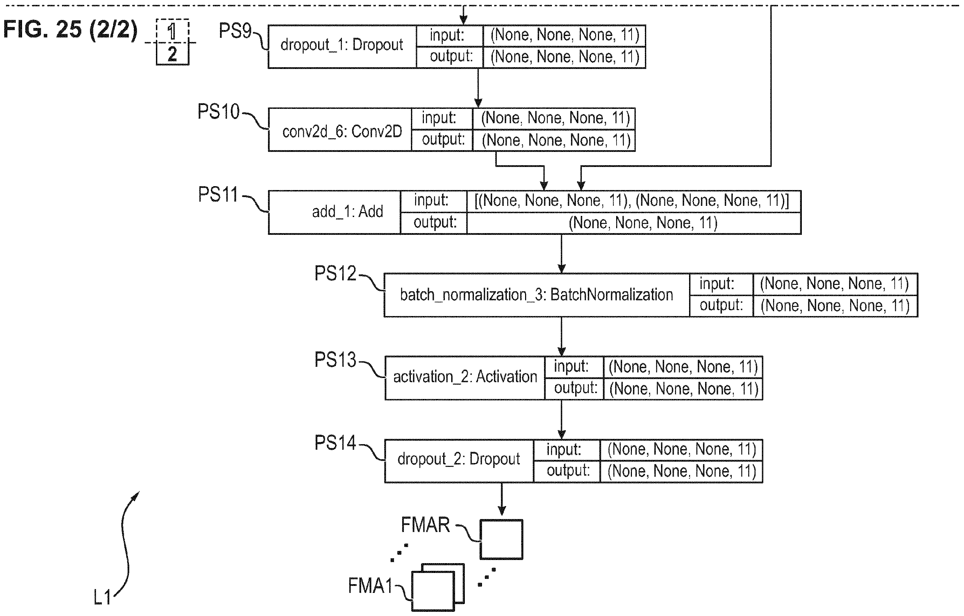

Im Detail folgen die P Layer L1,..., LP aufeinander, wobei die Figur 25 eine beispielhafte Struktur des ersten Layers L1 darlegt.

Nach dem letzten Layer LP ergeben sich beispielsweise 56 Featuremaps der Dimensionalität 8x8 Pixel. In einem abschließenden Verarbeitungsschritt AVS erfolgt dann eine Faltung mit N Faltungskerneln entsprechend der Anzahl der N Klassen bzw. der N Fluoreszenzmustertypen, um die N unterschiedlichen Featuremaps FM1,..., FMN zu generieren.

Die Figur 25 zeigt hierzu eine beispielhafte Ausführung des ersten Layers L1 aus der Figur 2. Der Vorverarbeitungsschritt VS2 ist hierbei gestrichelt dargestellt, da er nicht Gegenstand dieses Layers L1 ist sondern ihm nur vorgeschaltet ist.

Die Featuremaps aus dem Vorverarbeitungsschritt VS2 werden jeweils an die Verarbeitungsschritte PS1, PS2, PS4 weitergeleitet. Jeder dieser Verarbeitungsschritte PS1, PS2, PS4 verarbeitet all die von dem Schritt VS2 entgegengenommenen Featuremaps für sich.

In dem Schritt PS1 erfolgt eine Faltung mit 8 Kerneln ohne Striding. In dem Schritt PS2 erfolgt eine Faltung mit 8 Kerneln mit einem Striding-Faktor 2. In dem Schritt PS4 erfolgt eine Faltung mit 11 Kerneln mit dem Striding-Faktor 2.

In einem Schritt PS3 erfolgt ein sogenanntes Max Pooling mit einem Striding-Faktor vom Wert 2 für jede der Featuremaps.

In einem Schritt PS5 werden die 8 Featuremaps aus dem Schritt PS3 mit den 8 Featuremaps aus dem Schritt PS2 konkateniert. In einem Schritt PS6 erfolgt wiederum eine Faltung der 16 eingehen Featuremaps mit 11 Kernen ohne Striding.

In einem Schritt PS7 erfolgt eine sogenannte Batch Normalisierung auf jeder der 11 Featuremaps.

In einem Schritt PS8 erfolgt eine sogenannte Activation vorzugsweise in Form einer RELU Activation.

In einem Schritt PS9 erfolgt vorzugsweise während der Trainingsphase ein sogenanntes Dropout mit einem Dropout-Faktor von 20 %. Dieses Dropout erfolgt nicht während der Klassifikationsphase.

In einem Schritt PS10 erfolgt eine Faltung mit 11 Kernel ohne Striding.

Die Ergebnisse der Featuremaps bzw. der 11 Featuremaps aus dem Schritt PS10 werden mit den 11 Featuremaps aus dem Schritt PS4 in dem Schritt PS11 elementweise addiert, sodass wiederum der Schritt PS11 11 Featuremaps generiert. Hierbei erfolgt kein Striding.

In einem Schritt PS12 erfolgt wiederum eine sogenannte Batch Normalization.

In einem Schritt PS13 erfolgt eine sogenannte Activation vorzugsweise in Form einer RELU Activation.

In einem Schritt PS14 erfolgt dann vorzugsweise ein Dropout während der Trainingsphase mit einem Dropout-Faktor von beispielsweise 20 %.

Zurückkommend zur Figur 2 kann angemerkt werden, dass vorzugsweise P=6 derartige Layer aufeinanderfolgen, welche so dimensioniert werden, dass das letzte Layer LP mit P=6 dann Featuremaps erzeugt, welche eine Größe von 8x8 Pixeln aufweisen. Vorzugsweise erzeugt das letzte Layer LP 56 (sechsundfünzig) verschiedene solche Featuremaps, wobei dann in einem in Figur 2 eingezeichneten Nachverarbeitungsschritt AVS eine Faltung der 56 Featuremaps mit N=8 Kerneln erfolgt, um die N=8 Featuremaps FM1,..., FMN zu erzeugen.

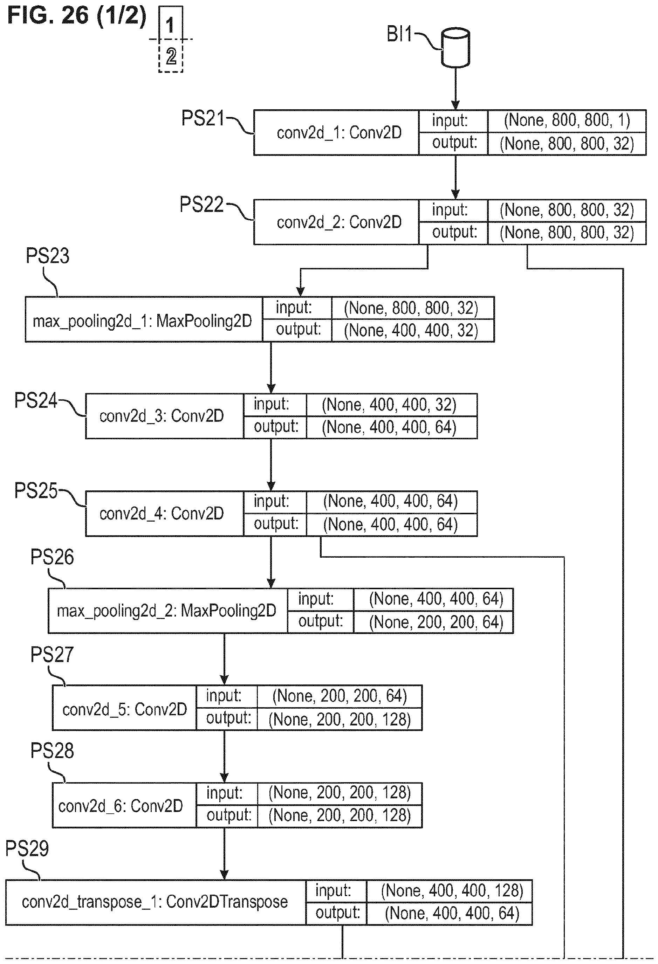

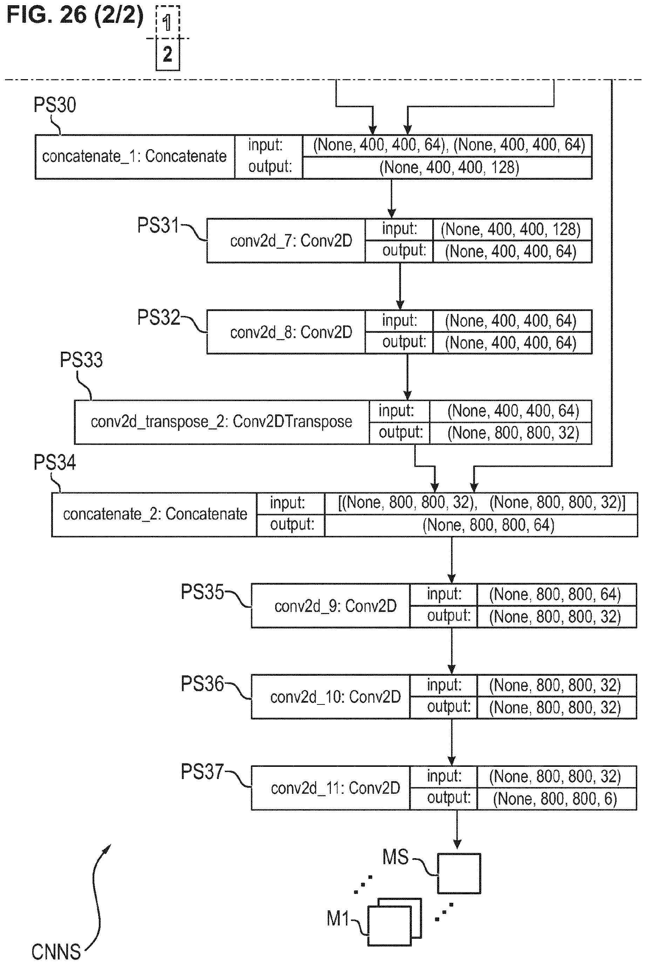

In der Figur 5 ist das Segmentierungs-Convolutional Neural Network SEG-CNN dargestellt, welches mittels der Layer LA1, LAQ eine Segmentierung des ersten Bildes B1 bzw. der Bildinformation BI1 herbeiführt. Vorzugsweise erfolgt in einem optionalen Schritt DWS das sogenannte Downscaling. Vorzugsweise weist das erste Bild in Form der Bilddaten BI1 eine Dimensionalität von 2400×1700 Pixeln auf. Dieses erste Bild kann dann durch ein sogenanntes Downsampling auf eine Größe von 800×800 Pixeln reduziert werden. Die weiteren Prozessierungsschritte aus der Figur 26 sind im Detail für den beispielhaften Fall dargelegt, dass ein Downscaling DWS des ersten Bildes auf eine Größe von 800×800 Pixeln erfolgte. Dieses ist aber nicht zwingend notwendig, das Convolutional Neural Network CNNS aus der Figur 26 kann auch in seiner Dimensionierung so ausgestaltet werden, dass es das erste Bild mit einer Größe von 2400x1700 Pixeln ohne ein Downscaling verarbeiten kann.

Die Figur 26 illustriert Details des Convolutional Neural Network CNNS aus der Figur 5. In Figur 26 erfolgt in einem ersten Schritt PS21 erfolgt eine Faltung mit 32 Kerneln, welches aufgrund des Parameters 32 an dem Ausgangslayer bzw. Output-Layer abgelesen werden kann.

Daran, dass die Eingangsgröße des Schrittes PS21 eine Dimensionalität von 800×800 Pixeln aufweist und auch die Ausgangsgröße des Schrittes PS21 eine Dimensionalität von 800×800 Pixeln, kann abgeleitet werden, dass kein Striding erfolgt. Für einen Verarbeitungsschritt PS21, ..., P37 ist jeweils die Dimensionalität der Eingangsgröße als "input" in Klammern anhand der zwei Zahlen hinter der Angabe "None" angegeben. Für einen Verarbeitungsschritt PS21, ...,P37 ist ferner jeweils die Dimensionalität der Ausgangsgröße als "output" in Klammern anhand der zwei Zahlen hinter der Angabe "None" angegeben.

Durch die verschiedenen Schritte PS21 bis PS37 erfolgt eine Prozessierung des ersten Bildes bzw. der Bilddaten BI1 hin zu den Featuremaps M1,.., MS, wie bereits in der Figur 5 illustriert.

Anzumerken ist hierbei, dass der Schritt PS29 hierbei eine sogenannte Dekonvolution bzw. Transposed Convolution durchführt.

Die weiteren Schritte führen entweder eine Faltung durch, wie bereits im Zuge der Beschreibung der Figur 25 dargelegt, oder aber ein Max Pooling oder eine Konkatenierung, wie auch bereits im Zuge der Beschreibung zu Figur 25 dargelegt.

Erfolgte vor Prozessieren der Bilddaten BI1, welche auch in der Figur 5 illustriert sind, ein sogenanntes Downsampling des ersten Bildes bzw. der Bilddaten BI1, so kann nach Bestimmung der Segmentierungs-Map SM ein entsprechendes Upsampling erfolgen, bevor die Segmentierungs-Map SM dem weiteren Bildverarbeitungsschritt BV zugeführt wird.

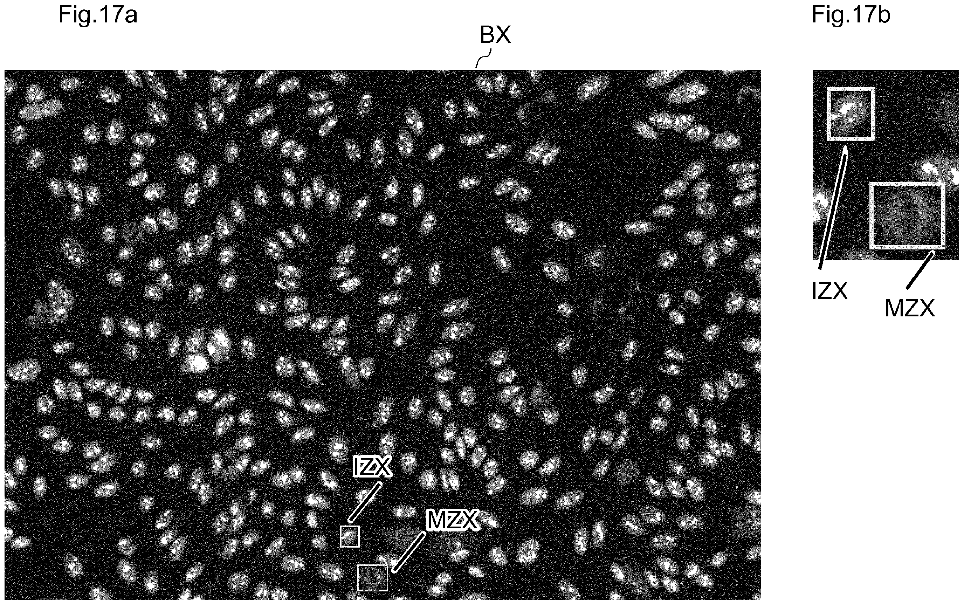

Die Figur 17a zeigt ein Bild BX als ein Fluoreszenzbild eines zweiten Farbkanals bzw. bezogen auf einen zweiten Fluoreszenzfarbstoff für ein Mischmuster, bei welchem ein nukleoläres Muster (AC- 8, 9,10) der Klasse n=7 vorhanden ist als auch ein Muster einer feinen oder groben Sprenkelung als der Fluoreszenzmustertyp der Klasse n=5 (AC-4, AC-5).

Beispielhaft zeigt hierzu die Figur 17b vergrößert eine Interphase-Zelle IZX als auch eine Mitosezelle MZX. Es ist klar zu erkennen, dass die Metaphasenplatte der Mitosezelle MZX nicht signifikant gefärbt ist bzw. die Mitosezelle mit ihren Chromosomen der Metaphasenplatte nicht gefärbt ist. Diese Information erlaubt es zu unterscheiden, ob eben hier ein sogenanntes fein oder grob gesprenkelte Muster (AC-4, AC-5) vorliegt oder aber ob vielleicht doch ein homogenes Muster (AC-1). Da nämlich die Mitosezelle MZX mit ihrer Metaphasenplatte dort im Chromosomenbereich nicht gefärbt ist kann die homogene Färbung der Klasse n=2 ausgeschlossen werden, denn bei einer homogenen Färbung müsste auch die Mitosezelle im Bereich der Metaphasenplatte homogen gefärbt sein, welches hier aber nicht der Fall ist. Daher kann durch das Einbeziehen der Mitosezelle in die Fluoreszenzmustertypdetektion sicher detektiert werden, dass es sich nicht um einen homogenen Fluoreszenzmustertyp sondern um einen fein gesprenkelten Fluoreszenzmustertyp mit n=5 (AC-4) oder aber um einen grob gesprenkelten Fluoreszenzmustertyp mit n=5 (AC-5) handelt.

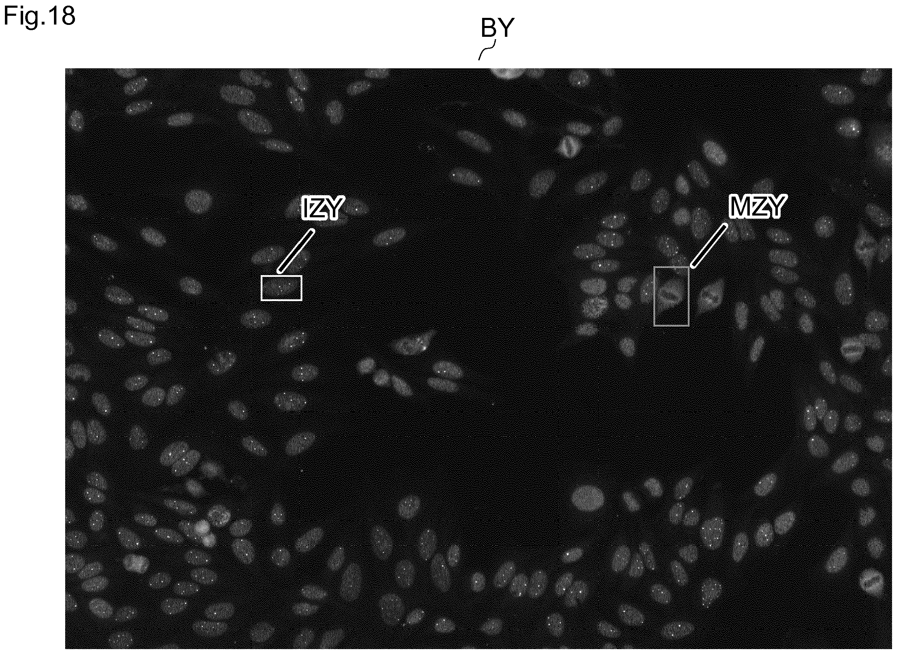

Die Figur 18 zeigt ein weiteres Beispiel BY eines Bildes im zweiten Fluoreszenzkanal bzw. bezogen auf den zweiten Fluoreszenzfarbstoff unter einer Hervorhebung einer Mitosezelle MZY als auch einer Interphase-Zelle IZY.

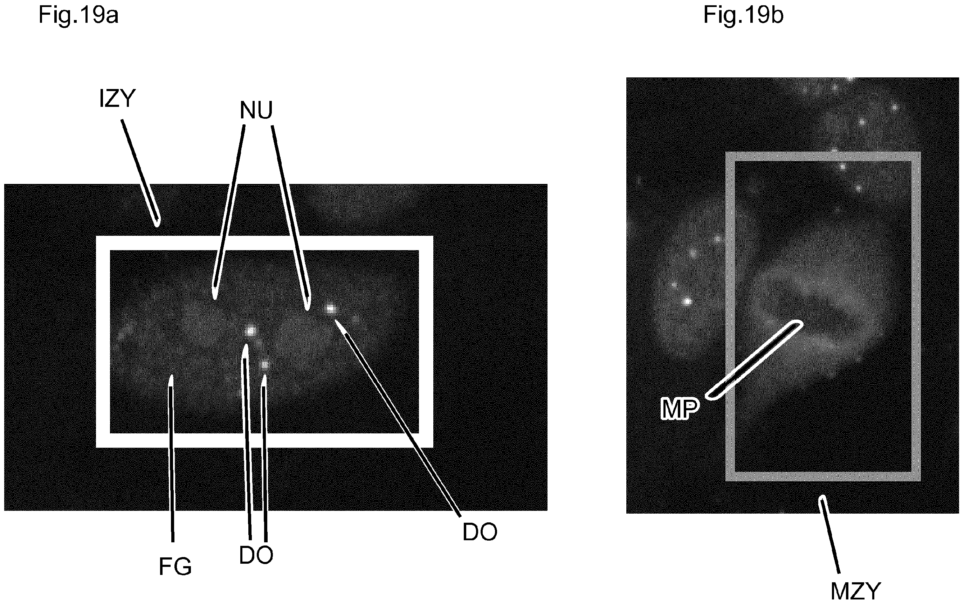

Die Figur 19a zeigt hierzu in vergrößerter Darstellung diese Interphase-Zelle IZY und in der Figur 19b die entsprechende Mitosezelle MZY mit ihrer Metaphasenplatte MP.

Es handelt sich bei dem Bild BY aus der Figur 18 um ein Bild mit drei präsenten Fluoreszenzmustertypen. In diesem Fluoreszenzmusterbild ist der Fluoreszenzmustertyp n=7, auch nukleolär genannt (AC-8, 9,10), präsent, ferner der Fluoreszenzmustertyp n=6, auch nukleäre Punkte genannt (AC-6,7), als auch der Fluoreszenzmustertyp n=5, auch feingesprenkelt genannt (AC-4), als eine der Variante des Fluoreszenzmustertyps n=5 (AC-4, AC-5). Die Figur 19a indiziert hierzu die Färbung des Musters nukleolär durch die Bereiche NU, die Färbung des Musters nukleolärer Punkte durch die Bereiche DO und auch die Färbung durch das fein gesprenkelte Muster durch den weiteren Bereich FG des Zellkerns.

Hierbei ist es gerade vorteilhaft, dass gemäß des erfindungsgemäßen Verfahrens auch die Mitosezelle MZY in einem Teilbild vorhanden sein muss, da die Metaphasenplatte MP eben nicht gefärbt ist bzw. deren Chromosomen nicht gefärbt sind, sodass hier der Bereich der feinen Sprenkelung FG sicher als fein gesprenkelt (AC-4) mit n=5 detektiert werden kann und nicht fälschlicherweise als homogen gefärbt angesehen wird, wie es bei dem homogenen Muster n=2 (AC-1) der Fall sein könnte, da bei diesem homogenen Muster die Metaphasenplatte MP signifikant gefärbt sein müsste. Ebenso kann hier eine Musterfärbung des Typs "dicht fein gesprenkelt" mit n=4 (AC-2) ausgeschlossen werden, da bei diesem Fluoreszenzmustertyp die Metaphasenplatte MP der Mitosezelle MZY signifikant gefärbt sein müsste.

Hierdurch zeigt sich hier noch einmal, dass das erfindungsgemäße Verfahren, bei welchem Teilbilder abhängig davon selektiert werden, ob sie Bildsegmente mit wenigstens einer mitotischen Zelle von hinreichender Qualität aufweisen, selektiert werden, eine besonders hohe Güte eine Detektion unterschiedlicher Fluoreszenzmustertyp ermöglicht.

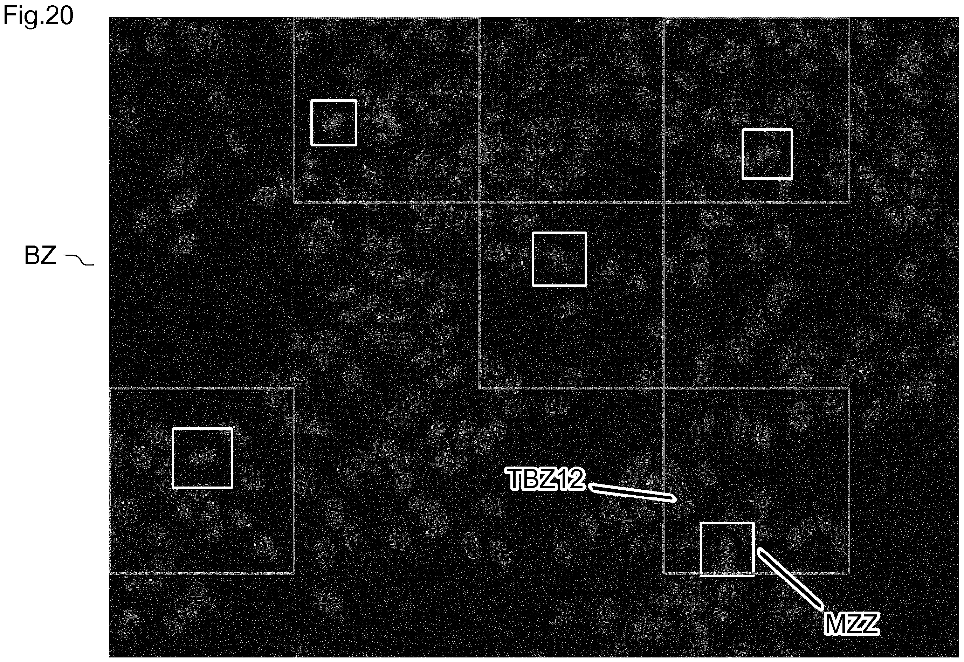

Die Figur 20 zeigt ein weiteres beispielhaftes Fluoreszenzbild BZ, bei welchem ein Teilbildbereich TBZ12 eine Mitosezelle MZZ aufweist. Dieses Fluoreszenzbild des zweiten Farbkanals beziehungsweise bezogen auf den zweiten Fluoreszenzfarbstoff zeigt eindeutig ein sogenanntes dicht fein gesprenkeltes Musters des Typs n=4 (AC-2).

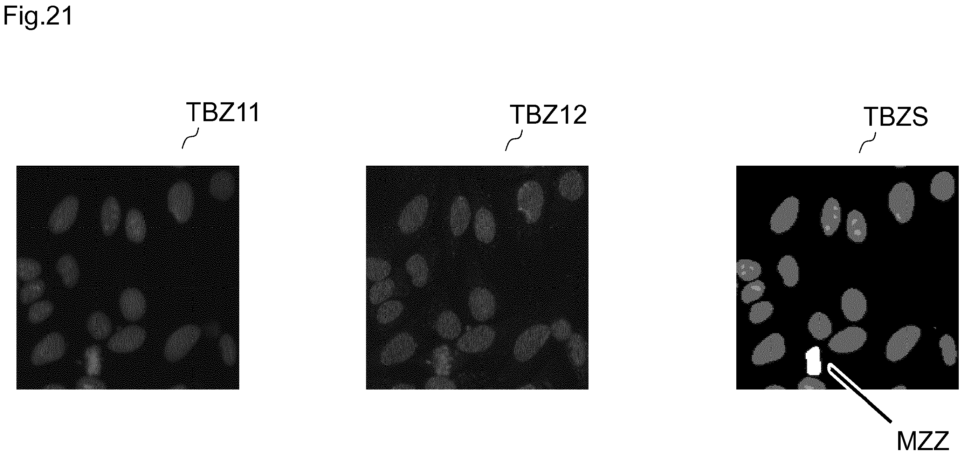

Die Figur 21 zeigt hierzu das beispielhafte Teilbild TBZ11 als das erste Teilbild des ersten Fluoreszenzfarbstoffes und das zweite Teilbild TBZ12, welches zu dem ersten Teilbild TBZ11 korrespondiert, aus dem zweiten Fluoreszenzfarbkanal. Ferner zeigt die Figur 21 das dazu segmentierte Teilbild TBZS.

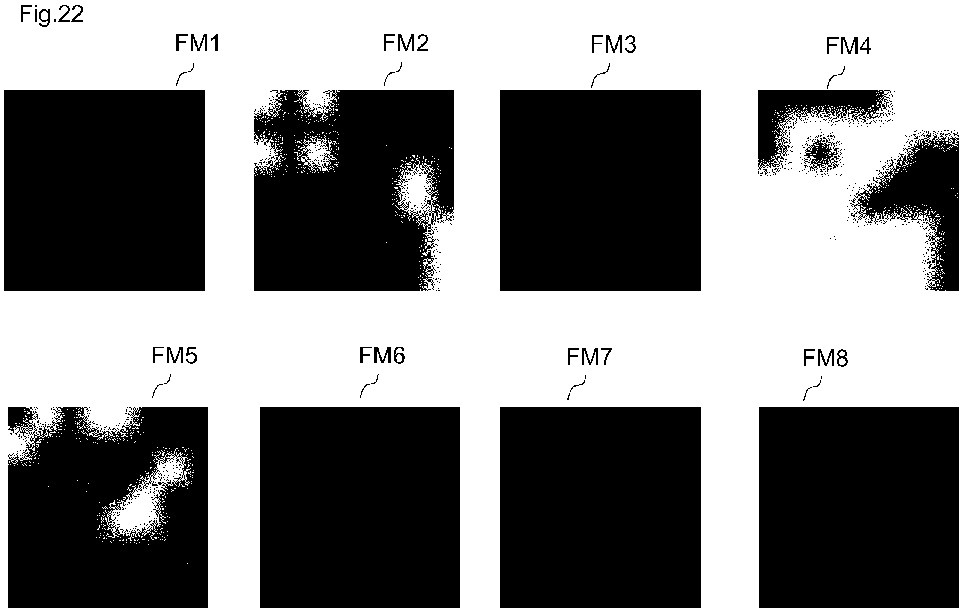

Die Figur 22 zeigt hierzu entsprechende Feature-Maps FM1,..., FM8 für die verschiedenen Fluoreszenzmustertypen beziehungsweise Fluoreszenzmusterklassen n=1...8 bezogen auf die Teilbilder TBZ11, TBZ12 aus Figur 21.

Klar ersichtlich ist hier die Aktivierung in der Feature-Map FM4 der entsprechenden Klasse n=4 für das dicht fein gesprenkelte Muster als Fluoreszenzmustertyp (AC-2), welches hier klar dominiert.

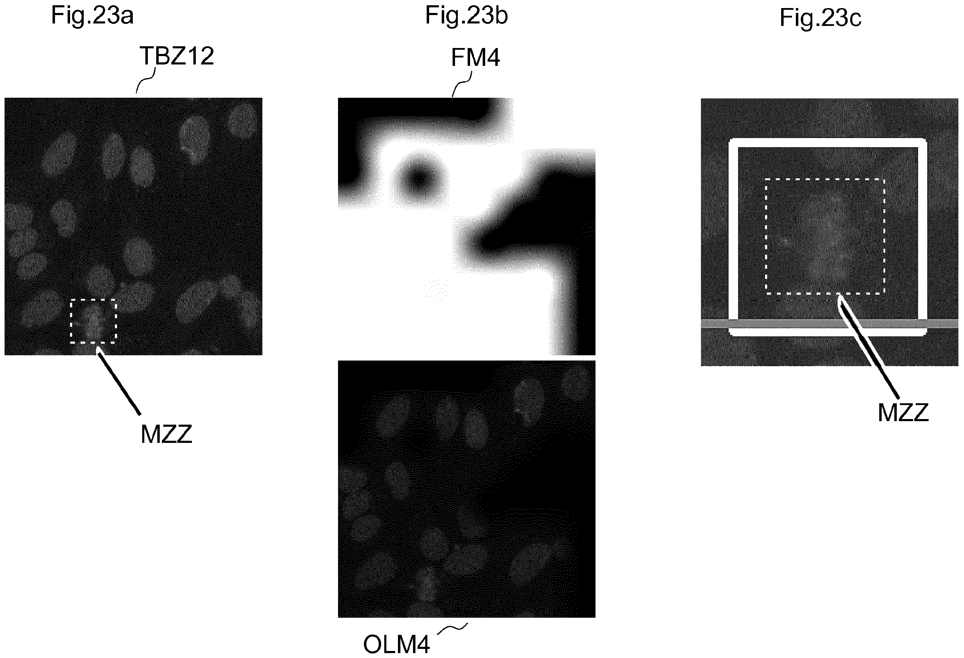

Hierzu zeigt die Figur 23a noch einmal das zweite Teilbild TBZ12, welches die in Fig.23c vergrößert dargestellte Mitosezelle MZZ aufweist, In der Figur 23b ist die Feature-Map FM4 des entsprechenden Fluoreszenzmustertypen n=4 zu sehen sowie eine Überlagerung dieser Feature-Map FM4 über das zweite Teilbild TBZ12 als das überlagerte Bild OLM4. Es ist klar ersichtlich, dass die Feature-Map FM4 auf jene Bildbereiche des zweiten Teilbildes TBZ12 abstellt, welche die entsprechend des Fluoreszenzmustertypen gefärbten Interphase-Zellen und auch die entsprechend des Fluoreszenzmustertypen gefärbte Mitosezelle aufweist.

Ferner zeigt die Figur 23c noch einmal in vergrößerter Darstellung die Mitosezelle MZZ, welche in dem Teilbild TBZ12 vorhanden ist. Die eindeutige Färbung der Metaphasenplatte der Mitosezelle MZZ ermöglicht hier die Detektion des dicht-fein gesprenkelten Fluoreszenzmustertyps n=4.

Das erfindungsgemäße Verfahren wurde hier beschrieben, indem in einem Schritt SC2 aus der Figur 1 das erste und das zweite Bild erfasst werden. Es wird daher eben ein Verfahren zur Detektion jeweiliger potentieller Präsenzen jeweiliger unterschiedlicher zellulärer Fluoreszenzmustertypen auf einem biologischen Zellsubstrat aufweisend humane Epitheliomzellen mittels digitaler Bildverarbeitung vorgeschlagen, welches mit dem Schritt des Erfassens des ersten Bildes und des zweiten Bildes beginnt.

Alternativ zu dem Schritt SC2 des Erfassens des ersten und des zweiten Bildes kann auch ein Verfahren zur digitalen Bildverarbeitung entsprechend und in analoger Weise durchgeführt werden, bei welchem in einem entsprechenden Schritt ein solches erstes Bild und ein solches zweites Bild bereitgestellt bzw. entgegengenommen werden und bei welchem dann die weiteren Schritte SC3, SC4, SC5 durchgeführt werden.

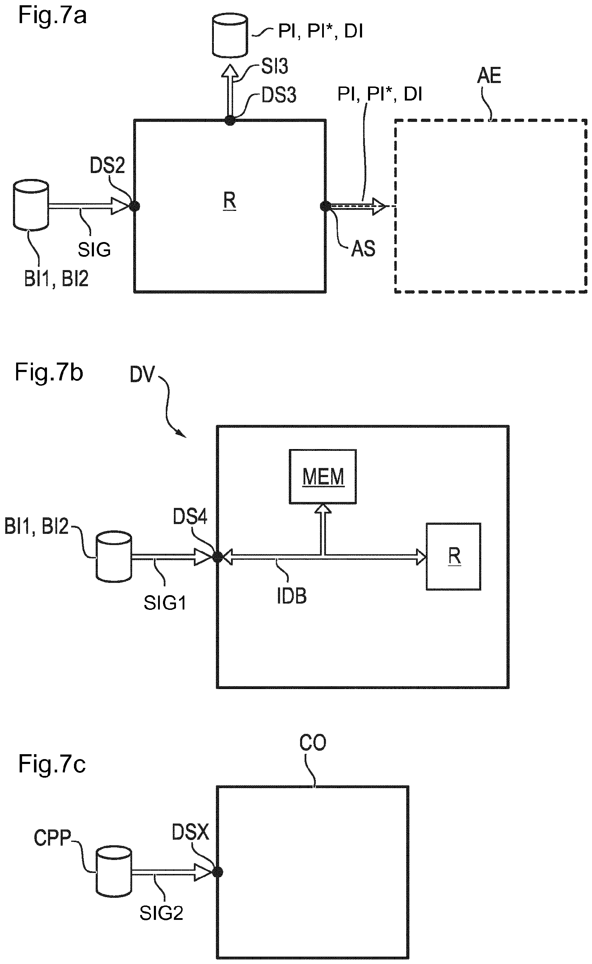

Hierzu zeigt die Figur 7A eine vorgeschlagene Recheneinheit R, welche ein entsprechendes erstes Bild beziehungsweise eine erste Bildinformation BI1 als auch ein entsprechendes zweites Bild beziehungsweise eine entsprechende zweite Bildinformation BI2 in Form eines vorzugsweise Datensignals SIG über eine Schnittstelle DS2 entgegennimmt. Die Recheneinheit R kann dann vorzugsweise über eine Ausgabeschnittstelle AS, insbesondere an eine Anzeigeeinheit AE, die ermittelten Konfidenzmaße PI, die verifizierten Konfidenzmaße PI* und/oder die Detektionsinformation DI ausgeben.

Diese Informationen können vorzugsweise auch über eine Datenschnittstelle DS3 in Form einer Datennetzwerkschnittstelle in Form eines Signals SI3 ausgegeben werden.

Die Figur 7B zeigt hierzu eine vorgeschlagene Datennetzwerkvorrichtung, welche über eine Schnittstelle S4 und ein entsprechendes Datensignal SIG1 die Bilder beziehungsweise die Bildinformationen B11, BI2 entgegennimmt. Die Datennetzwerkvorrichtung weist ferner vorzugsweise einen internen Datenbus IDB auf, welcher eine zuvor beschriebene Recheneinheit R mit vorzugsweise einer Speichereinheit MEM verbindet.

Die Figur 7C illustriert ein vorgeschlagenes Computerprogrammprodukt CPP, welches Befehle umfasst, die bei der Ausführung des Programms durch einen Computer diesen veranlassen, das vorgeschlagene Verfahren zur digitalen Bildverarbeitung durchzuführen. Dieses Computerprogrammprodukt kann beispielsweise in vorgeschlagener Weise über ein Datensignal SIG2 bereitgestellt werden beziehungsweise übertragen werden. Diese Übertragung kann zu einem Computer CO beziehungsweise an eine Datenstelle DSX des Computers CO erfolgen.

Die Figur 6 illustriert ein Ausführungsbeispiel der erfindungsgemäßen Vorrichtung V1. Die Vorrichtung V1 weist eine Haltevorrichtung HL für das Substrat SU auf. Anregungslicht AL einer Anregungslichtquelle LQ wird über ein optisches Filter F1 vorgefiltert und dann mittels eines dichroitischen Spiegels SP1 durch eine Optik O hin zum Substrat geleitet. Entstehende Fluoreszenzstrahlung bzw. entstehendes Fluoreszenzlicht FL tritt dann vom Substrat SU her zurück durch das Objektiv O durch den dichroitischen Spiegel SP1 und durch ein optisches Filter F2 hindurch. Das optische Filter F2 filtert eine Wellenlänge der Anregungsstrahlung bzw. des Anregungslichtes AL heraus. Das Fluoreszenzlicht FL wird dann wenigstens einer Bilderfassungseinheit in Form einer Kamera K1 sowie vorzugsweise einer weiteren Kamera K2 zugeführt. Über einen dichroitischen Spiegel SP2 wird das Fluoreszenzlicht FL aufgeteilt. Über einen optischen Filter FR wird Fluoreszenzlicht FR1 eines ersten Farbkanals, vorzugsweise eines roten Farbkanals, herausgefiltert und der Bilderfassungseinheit K1 zugeführt. Die Bildererfassungseinheit K1 erfasst ein erstes Fluoreszenzbild des Substrates SU in dem ersten Farbkanal. Über einen optischen Filter FG wird Fluoreszenzstrahlung FL2 des zweiten Farbkanals, vorzugsweise des grünen Farbkanals, herausgefiltert und der Bilderfassungseinheit K2 zugeführt, welche ein zweites Fluoreszenzbild des Substrates SU in dem zweiten Farbkanal erfasst.

Eine Recheneinheit R ist ausgebildet, das erste Fluoreszenzbild in Form digitaler Bilddaten BI1 entgegenzunehmen. Ferner ist die Recheneinheit R ausgebildet, das zweite Fluoreszenzbild in Form digitaler Bilddaten BI2 entgegenzunehmen. Die Recheneinheit R ist ferner ausgebildet, auf Basis des ersten Bildes jeweilige Bildsegmente zu detektieren, welche jeweils wenigstens eine mitotische Zelle repräsentieren, ferner Teilbilder des ersten Bildes und dazu korrespondierenden Teilbildern des zweiten Bildes auf Basis der detektierten Bildsegmente zu selektieren und ferner auf Basis der selektierten Teilbilder des ersten Bildes und der selektierten Teilbilder des zweiten Bildes jeweilige tatsächliche Präsenzen jeweiliger der zellulären Fluoreszenzmustertypen mittels eines Convolutional Neural Network zu detektieren.

Über eine Datenschnittstelle DS1 kann die Vorrichtung V1 eine Detektionsergebnis in Form von einer Deketionsinformation DI und/oder eine Präsenzinformation bzw. eine Prädiktionsinformation PI, PI* bereitstellen.

Obwohl manche Aspekte im Zusammenhang mit einer Vorrichtung beschrieben wurden, versteht es sich, dass diese Aspekte auch eine Beschreibung der entsprechenden Verfahren darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung die Recheneinheit R oder die Datennetzwerkvorrichtung DV in Hardware und/oder in Software umsetzen. Eine Umsetzung einer hier genannten Recheneinheit R kann hier als wenigstens eine Recheneinheit erfolgen oder aber durch mehrere Recheneinheiten im Verbund. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann als Recheneinheit durch einen Prozessor, einen Computerprozessor (CPU = Central Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird.

Allgemein können Ausführungsbeispiele oder Teile der Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren oder ein Teil eines Verfahrens durchzuführen, wenn das Programm auf einem Prozessor oder einer programmierbaren Hardwarekomponente abläuft.

Für eine Implementation eines oder mehrerer Ausführungsbeispiele der hier vorgeschlagenen Convolutional Neural Networks kann ein Fachmann auf eine sogenannte Open Source Deep-Learning Bibliothek namens "Keras" zurückgreifen. Detaillierte Informationen findet der Fachmann unter https://keras.io .

**Beispiel 1**

Das vorgegebene System bzw. das erfindungsgemäße Verfahren wurde auf 161 unterschiedlichen Patientenproben getestet, dabei wurden die Proben in einer linearen Verdünnungsreihe startend von 1:40 ausverdünnt. Das heißt, die Patientenproben wurden beginnend von 1:40 in einer linear steigenden Folge ausverdünnt und inkubiert. In diesem Falle wurden Proben entweder in den Schritten 1:40, 1:80, 1:640, 1:1280 oder 1:40, 1:80, 1:160, 1:320, 1:640, 1:1280, 1:2560, 1:5120, 1:10240. Das System ist jedoch in keiner Weise auf vorgegebene Verdünnungsreihen beschränkt.

Ein Muster wurde durch einen Experten bezogen auf eine bestimmte Probe als präsent erklärt, wenn es in irgendeiner Verdünnung bzw. irgendeinem Fluoreszenzbild bezogen auf die bestimmte Probe als präsent erkannt wurde. Das Muster musste nicht in allen Verdünnungen bzw. allen Fluoreszenzbildern bezogen auf die bestimmte Probe durch den Experten erkannt werden, um als präsent erklärt zu werden.

Ein Muster wurde durch einen Experten bezogen auf eine bestimmte Probe als negativ (kein Muster aufweisend) erklärt, wenn es in allen Verdünnungen bzw. in allen Fluoreszenzbildern bezogen auf die bestimmte Probe als negativ (kein Muster aufweisend) erkannt wurde.

Ein Muster wurde durch das erfindungsgemäße Verfahren bezogen auf eine bestimmte Probe als generell präsent detektiert, wenn es in irgendeiner Verdünnung bzw. irgendeinem Fluoreszenzbild bezogen auf die bestimmte Probe als präsent detektiert wurde. Das Muster musste nicht durch das erfindungsgemäße Verfahren in allen Verdünnungen bzw. allen Fluoreszenzbildern bezogen auf die bestimmte Probe detektiert werden, um generell als präsent detektiert zu werden.

Ein Muster wurde durch das erfindungsgemäße Verfahren bezogen auf eine bestimmte Probe als negativ (kein Muster aufweisend) detektiert, wenn es in allen Verdünnungen bzw. allen Fluoreszenzbildern bezogen auf die bestimmte Probe als negativ (kein Muster aufweisend) detektiert wurde.

Bei einer Präsenz von beispielsweise zwei unterschiedlichen tatsächlich präsenten Mustern wurden die beiden Muster durch einen Experten auch dann als beide präsent erklärt, wenn er die beiden Muster in unterschiedlichen Verdünnungen bzw. unterschiedlichen Fluoreszenzbildern einer gleichen Probe erkannte.

Bei einer Präsenz von beispielsweise zwei unterschiedlichen tatsächlich präsenten Mustern wurden die beiden Muster durch das erfindungsgemäße Verfahren auch dann als beide präsent detektiert, wenn das Verfahren die beiden Muster in unterschiedlichen Verdünnungen bzw. unterschiedlichen Fluoreszenzbildern einer gleichen Probe detektierte.

Wurde also beispielsweise das Muster Homogene in der Verdünnung 1:40 detektiert und ein anderes Muster in einer anderen Ausverdünnung, beispielsweise 1:80, werden für diese Probe beide Muster ausgegeben.

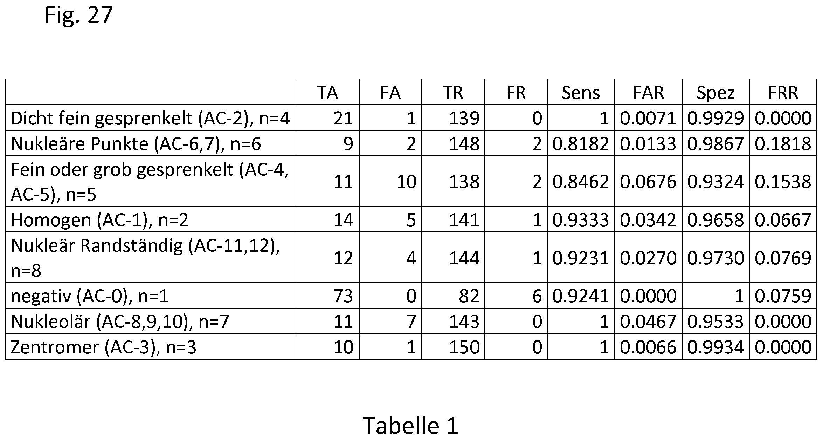

Die in Figur 27 in der Tabelle 1 stehenden Erkennerraten beziehen sich auf eine gesamte Patientenprobe aller Ausverdünnungen. Die Tabelle 1 zeigt für unterschiedliche Muster als auch den Fall einer negativen Probe jeweils die folgenden Kennwerte:

- TA: "True Acceptance", dies ist die Anzahl der Proben, bei welchen in irgendeiner Verdünnung ein Experte für dieses Muster auf präsent entschieden hat und bei welchen das System in wenigstens einer Verdünnung für dieses Muster auf präsent entschieden hat.

- FA: "False Acceptance", dies ist die Anzahl der Proben, bei welchen in allen Verdünnung ein Experte für dieses Muster auf nicht präsent entschieden hat und bei welchen das System in wenigstens einer Verdünnung für dieses Muster auf präsent entschieden hat.

- TR: "True Rejection", dies ist die Anzahl der Proben, bei welchen in allen Verdünnungen ein Experte für dieses Muster auf nicht präsent entschieden hat und bei welchen das System in allen Verdünnungen für dieses Muster auf nicht präsent entschieden hat.

- FR: "False Rejection", dies ist die Anzahl der Proben, bei welchen in irgendeiner Verdünnung ein Experte für dieses Muster auf präsent entschieden hat und bei welchen das System in wenigstens allen Verdünnungen für dieses Muster auf nicht präsent entschieden hat.

- Sens: Sensitivität des Systems als Absolutzahl.

- FAR: "False Acceptance Rate" als Absolutzahl.

- Spez: Spezifität als Absolutzahl.

- FRR: "False Rejection Rate" als Absolutzahl.

Die Ergebnisse ergeben sich durch die Anwendung des kompletten Systems, sprich Segmentierungsnetzwerk, Klassifikationsnetzwerk, Anwendung von Schwellenwerten der Wahrscheinlichkeiten der einzelnen Muster sowie Anwendung der Schwellenwerte der Helligkeiten der einzelnen Muster.

Das Convolutional Neural Network für die Erkennung einer Präsenz der Muster nutzte 5949 insgesamt Teilbild-Tupel zum Training, davon wurden 25% der jeweiligen Mustertypen für eine separate Validierung genutzt. Für das Convolutional Neural Network für die Segmentierung standen insgesamt 6463 Bilder, davon 4847 in der eigentlichen Trainingsmenge und 1616 in einer Validierungsmenge, zur Verfügung.

## Patentansprüche

1. Verfahren zur Detektion jeweiliger potentieller Präsenzen jeweiliger unterschiedlicher zellulärer Fluoreszenzmustertypen auf einem biologischen Zellsubstrat (SU) aufweisend humane Epitheliomzellen,

   wobei die zellulären Fluoreszenzmustertypen mehrere unterschiedliche antinukleäre Antikörper-Fluoreszenzmustertypen umfassen,
   das Verfahren aufweisend

   - Inkubieren des Zellsubstrates (SU) mit einer flüssigen Patientenprobe, welche potentiell primäre Antikörper aufweist, ferner mit einem ersten Fluoreszenzfarbstoff sowie ferner mit sekundären Antikörpern, welche mit einem zweiten Fluoreszenzfarbstoff markiert sind,
   - Erfassen eines ersten Bildes (B1), welches eine Färbung des Zellsubstrates durch den ersten Fluoreszenzfarbstoff repräsentiert, sowie Erfassen eines zweiten Bildes (B2), welches eine Färbung des Zellsubstrates durch den zweiten Fluoreszenzfarbstoff repräsentiert,
   - Detektieren jeweiliger Bildsegmente (VMZ), welche jeweils wenigstens eine mitotische Zelle (MZX, MZY) von einer hinreichenden Qualität repräsentieren, auf Basis des ersten Bildes (B1), wobei eine Mitosezelle dann eine hinreichende Qualität aufweist, wenn sie sich in der Metaphase befindet,
   - Selektieren von Teilbildern (TB11) des ersten Bildes (B1) und dazu korrespondierenden Teilbildern (TB12) des zweiten Bildes (B2) auf Basis der detektierten Bildsegmente (VMZ), welche jeweils wenigstens eine mitotische Zelle (MZX, MZY) einer hinreichenden Qualität repräsentieren, und
   - Detektieren jeweiliger tatsächlicher Präsenzen jeweiliger der zellulären Fluoreszenzmustertypen mittels eines Convolutional Neural Network (CNN2) auf Basis der selektierten Teilbilder (TB11) des ersten Bildes (B1) und der selektierten Teilbilder (TB12) des zweiten Bildes (B2)

   **gekennzeichnet durch**

   - Bestimmen jeweiliger Konfidenzmaße (PI, PI*) für die jeweiligen tatsächlichen Präsenzen der jeweiligen Fluoreszenzmustertypen mittels des Convolutional Neural Network (CNN2) auf Basis der selektierten Teilbilder (TB11) des ersten Bildes (B1) und der selektierten Teilbilder (TB12) des zweiten Bildes (B2)
   - wobei für ein jeweiliges Teilbild-Tupel ein jeweiliges Teilbild-Konfidenzmaß (PI1) für jeweilige tatsächliche Teilbild-Präsenzen jeweiliger zellulärer Fluoreszenzmustertypen mittels des Convolutional Neural Network (CNN2) bestimmt wird, wobei ein Teilbild-Tupel ein Teilbild (TB11) des ersten Bildes (B1) und ein korrespondierendes Teilbild (TB12) des zweiten Bildes (B2) aufweist und ein Teilbild-Konfidenzmaß einem Vektor von Konfidenzmaßen entspricht, wobei einem jeweiligen Konfidenzmaß ein jeweiliger Fluoreszenzmustertyp entspricht,
   - wobei das Convolutional Neural Network das Teilbild des ersten Bildes (B1) und das korrespondierende Teilbild des zweiten Bildes (B2) gleichzeitig auswertet,
- und Bestimmen jeweiliger Konfidenzmaße für die jeweiligen tatsächlichen Präsenzen der jeweiligen Fluoreszenzmustertypen auf Basis der Teilbild-Konfidenzmaße.

2. Verfahren nach Anspruch 1,

   wobei das Convolutional Neural Network (CNN2) ein Ausgabe-Layer (LP, AVS) aufweist, welches für einen jeweiligen zellulären Fluoreszenzmustertypen eine jeweilige Feature-Map (FM1,..., FMN) generiert, und wobei das Convolutional Neural Network (CNN2) ein jeweiliges Konfidenzmaß (P1,..., PN) auf Basis einer jeweiligen Feature-Map (FM1,..., FMN) bestimmt.

3. Verfahren nach Anspruch 1,
   ferner aufweisend

   - Segmentieren des ersten Bildes (B1) in Bildsegmente unterschiedlicher Segmentklassen,
   - Bestimmen wenigstens eines Helligkeitswertes (HI) für wenigstens einen Fluoreszenzmustertypen basierend auf einem oder mehreren Bildsegmenten wenigstens einer bestimmten Segmentklasse und
   - Verifizieren des Konfidenzmaßes (PI, PI*) für die tatsächliche Präsenz des wenigstens einen Fluoreszenzmustertypen auf Basis des Helligkeitswertes (HI) des wenigstens einen Fluoreszenzmustertypen.

4. Verfahren nach Anspruch 3,
   wobei das Verifizieren des Konfidenzmaßes (PI, PI*) auf Basis des Helligkeitswertes (HI) und in Abhängigkeit eines durch einen Nutzer vorgebbaren Schwellenwertes (H1,...,HN) erfolgt.

5. Verfahren nach Anspruch 1,
   ferner aufweisend

   - Aufteilen des zweiten Bildes (B2) in eine Menge von Teilbildern nach einem vorgegebenen Aufteilungsschema,
   - Selektieren von Teilbildern (TB12) des zweiten Bildes (B2) auf Basis der detektierten Bildsegmente und Selektieren von dazu korrespondierenden Teilbildern (TB11) des ersten Bildes (B1)
   - und Detektieren jeweiliger tatsächlicher Präsenzen jeweiliger der zellulären Fluoreszenzmustertypen mittels des Convolutional Neural Network (CNN2) auf Basis der selektierten Teilbilder (TB12) des zweiten Bildes (B2) und auf Basis der selektierten Teilbilder (TB11) des ersten Bildes (B1).

6. Recheneinheit (R) zur Detektion jeweiliger potentieller Präsenzen jeweiliger unterschiedlicher zellulärer Fluoreszenzmustertypen, wobei die zellulären Fluoreszenzmustertypen mehrere unterschiedliche antinukleäre Antikörper-Fluoreszenzmustertypen umfassen, welche ausgebildet ist im Zuge einer digitalen Bildverarbeitung

   - ein erstes Bild (B1) entgegenzunehmen, welches eine Färbung eines biologischen Zellsubstrates durch einen ersten Fluoreszenzfarbstoff repräsentiert, wobei das biologische Zellsubstrat humane Epitheliomzellen aufweist,
   - ein zweites Bild (B2) entgegenzunehmen, welches eine Färbung des biologischen Zellsubstrates durch einen zweiten Fluoreszenzfarbstoff repräsentiert,
   - auf Basis des ersten Bildes (B1) jeweilige Bildsegmente (VMZ) zu detektieren, welche jeweils wenigstens eine mitotische Zelle von einer hinreichenden Qualität repräsentieren, wobei eine Mitosezelle dann eine hinreichende Qualität aufweist, wenn sie sich in der Metaphase befindet,
   - auf Basis der detektierten Bildsegmente (VMZ) Teilbilder (TB11) des ersten Bildes (B1) und dazu korrespondierende Teilbilder (TB12) des zweiten Bildes (B2) zu selektieren, welche jeweils wenigstens eine mitotische Zelle (MZX, MZY) einer hinreichenden Qualität repräsentieren, und
   - auf Basis der selektierten Teilbilder (TB11) des ersten Bildes (B1) und der selektierten Teilbilder (TB12) des zweiten Bildes (B2) jeweilige tatsächliche Präsenzen jeweiliger der zellulären Fluoreszenzmustertypen mittels eines Convolutional Neural Network (CNN2) zu detektieren
   **dadurch gekennzeichnet, dass** die Recheneinheit ferner ausgebildet ist,

   - jeweilige Konfidenzmaße (PI, PI*) für die jeweiligen tatsächlichen Präsenzen der jeweiligen Fluoreszenzmustertypen mittels des Convolutional Neural Network (CNN2) auf Basis der selektierten Teilbilder (TB11) des ersten Bildes (B1) und der selektierten Teilbilder (TB12) des zweiten Bildes (B2) zu bestimmen,
   - für ein jeweiliges Teilbild-Tupel ein jeweiliges Teilbild-Konfidenzmaß (PI1) für jeweilige tatsächliche Teilbild-Präsenzen jeweiliger zellulärer Fluoreszenzmustertypen mittels des Convolutional Neural Network (CNN2) zu bestimmen, wobei ein Teilbild-Tupel ein Teilbild (TB11) des ersten Bildes (B1) und ein korrespondierendes Teilbild (TB12) des zweiten Bildes (B2) aufweist und ein Teilbild-Konfidenzmaß einem Vektor von Konfidenzmaßen entspricht, wobei einem jeweiligen Konfidenzmaß ein jeweiliger Fluoreszenzmustertyp entspricht,

- wobei das Convolutional Neural Network das Teilbild des ersten Bildes (B1) und das korrespondierende Teilbild des zweiten Bildes (B2) gleichzeitig auswertet,
- und jeweilige Konfidenzmaße für die jeweiligen tatsächlichen Präsenzen der jeweiligen Fluoreszenzmustertypen auf Basis der Teilbild-Konfidenzmaße zu bestimmen.

7. Datennetzwerkvorrichtung (DV), aufweisend

wenigstens eine Datenschnittstelle (DS4) zum Entgegennehmen eines ersten Bildes (B1), welches eine Färbung eines biologischen Zellsubstrates durch einen ersten Fluoreszenzfarbstoff repräsentiert, wobei das biologische Zellsubstrat humane Epitheliomzellen aufweist, sowie zum Entgegennehmen eines zweiten Bildes (B2), welches eine Färbung des biologischen Zellsubstrates durch einen zweiten Fluoreszenzfarbstoff repräsentiert, **gekennzeichnet durch** eine Recheneinheit (R) nach Anspruch 6.

8. Computerprogrammprodukt (CPP), umfassend Befehle, die bei der Ausführung des Programms durch einen Computer (CO) diesen veranlassen, ein Verfahren zur digitalen Bildverarbeitung zur Detektion jeweiliger potentieller Präsenzen jeweiliger unterschiedlicher zellulärer Fluoreszenzmustertypen, wobei die zellulären Fluoreszenzmustertypen mehrere unterschiedliche antinukleäre Antikörper-Fluoreszenzmustertypen umfassen, durchzuführen aufweisend

- Entgegennehmen eines ersten Bildes (B1), welches eine Färbung eines biologischen Zellsubstrates durch einen ersten Fluoreszenzfarbstoff repräsentiert, wobei das biologische Zellsubstrat humane Epitheliomzellen aufweist,
- Entgegennehmen eines zweiten Bildes (B2), welches eine Färbung des biologischen Zellsubstrates durch einen zweiten Fluoreszenzfarbstoff repräsentiert,
- Detektieren jeweiliger Bildsegmente (VMZ), welche jeweils wenigstens eine mitotische Zelle (MZX, MZY) von einer hinreichenden Qualität repräsentieren, auf Basis des ersten Bildes (B1), wobei eine Mitosezelle dann eine hinreichende Qualität aufweist, wenn sie sich in der Metaphase befindet,
- Selektieren von Teilbildern (TB11) des ersten Bildes (B1) und dazu korrespondierenden Teilbildern (TB12) des zweiten Bildes (B2) auf Basis der detektierten Bildsegmente (VMZ), welche jeweils wenigstens eine mitotische Zelle (MZX, MZY) einer hinreichenden Qualität repräsentieren, und
- Detektieren jeweiliger tatsächlicher Präsenzen jeweiliger der zellulären Fluoreszenzmustertypen mittels eines Convolutional Neural Network (CNN2) auf Basis der selektierten Teilbilder (TB11) des ersten Bildes (B1) und der selektierten Teilbilder (TB12) des zweiten Bildes (B2).

**gekennzeichnet durch**

- Bestimmen jeweiliger Konfidenzmaße (PI, PI*) für die jeweiligen tatsächlichen Präsenzen der jeweiligen Fluoreszenzmustertypen mittels des Convolutional Neural Network (CNN2) auf Basis der selektierten Teilbilder (TB11) des ersten Bildes (B1) und der selektierten Teilbilder (TB12) des zweiten Bildes (B2),
- für ein jeweiliges Teilbild-Tupel Bestimmen eines jeweiligen Teilbild-Konfidenzma-ßes (PI1) für jeweilige tatsächliche Teilbild-Präsenzen jeweiliger zellulärer Fluoreszenzmustertypen mittels des Convolutional Neural Network (CNN2), wobei ein Teilbild-Tupel ein Teilbild (TB11) des ersten Bildes (B1) und ein korrespondierendes Teilbild (TB12) des zweiten Bildes (B2) aufweist und ein Teilbild-Konfidenzmaß einem Vektor von Konfidenzmaßen entspricht, wobei einem jeweiligen Konfidenzmaß ein jeweiliger Fluoreszenzmustertyp entspricht,
- wobei das Convolutional Neural Network das Teilbild des ersten Bildes (B1) und das korrespondierende Teilbild des zweiten Bildes (B2) gleichzeitig auswertet,
- und Bestimmen jeweiliger Konfidenzmaße für die jeweiligen tatsächlichen Präsenzen der jeweiligen Fluoreszenzmustertypen auf Basis der Teilbild-Konfidenzmaße.

9. Datenträgersignal (SIG2), welches das Computerprogrammprodukt (CPP) nach Anspruch 8 überträgt.

**Claims**

1. Method for detecting respective potential presences of respective different cellular fluorescence pattern types on a biological cell substrate (SU) comprising human epithelioma cells,

   wherein the cellular fluorescence pattern types comprise a plurality of different antinuclear antibody fluorescence pattern types,
   the method comprising

   - incubating the cell substrate (SU) with a liquid patient sample which potentially comprises primary antibodies, further with a first fluorescent dye and further with secondary antibodies which are labelled with a second fluorescent dye,
   - acquiring a first image (B1) which represents staining of the cell substrate by the first fluorescent dye and acquiring a second image (B2) which represents staining of the cell substrate by the second fluorescent dye,
   - detecting, on the basis of the first image (B1), respective image segments (VMZ) which in each case represent at least one mitotic cell (MZX, MZY) of a sufficient quality, wherein a mitotic cell is of sufficient quality if it is in metaphase.
   - selecting, on the basis of the detected image segments (VMZ), subimages (TB11) of the first image (B1) and subimages (TB12) corresponding thereto of the second image (B2), which in each case represents at least one mitotic cell (MZX, MZY) of a sufficient quality and
   - detecting, on the basis of the selected subimages (TB11) of the first image (B1) and of the selected subimages (TB12) of the second image (B2), respective actual presences of respective cellular fluorescence pattern types by means of a convolutional neural network (CNN2)
   **characterized by**
   - determining, on the basis of the selected subimages (TB11) of the first image (B1) and of the selected subimages (TB12) of the second image (B2), respective confidence measures (PI, PI*) for the respective actual presences of the respective fluorescence pattern types by means of the convolutional neural network (CNN2).
   - wherein for a respective subimage tuple a respective subimage confidence measure (PI1) is determined for respective actual subimage presences of respective cellular fluorescence pattern types by means of the convolutional neural network (CNN2), wherein a subimage tuple comprises a subimage (TB11) of the first image (B1) and a corresponding subimage (TB12) of the second image (B2) and a subimage confidence measure corresponds to a vector of confidence measures, wherein a respective confidence measure corresponds to a respective fluorescence pattern type,
   - wherein the convolutional neural network simultaneously evaluates the subimage of the first image (B1) and the corresponding subimage of the second image (B2),
   - and determining respective confidence measures for the respective actual presences of the respective fluorescence pattern types on the basis of the subimage confidence measures.

2. Method according to Claim 1,

   wherein the convolutional neural network (CNN2) comprises an output layer (LP, AVS) which generates a respective feature map (FM1,...,FMN) for a respective cellular fluorescence pattern type,
   and wherein the convolutional neural network (CNN2) determines a respective confidence measure (P1,...,PN) on the basis of a respective feature map (FM1,...,FMN).

3. Method according to Claim 1,
   further comprising

   - segmenting the first image (B1) into image segments of different segment classes,
   - determining at least one lightness value (HI) for at least one fluorescence pattern type on the basis of one or more image segments of at least one specific segment class and
   - verifying the confidence measure (PI, PI*) for the actual presence of the at least one fluorescence pattern type on the basis of the lightness value (HI) of the at least one fluorescence pattern type.

4. Method according to Claim 3,
   wherein the verification of the confidence measure (PI, PI*) is carried out on the basis of the lightness value (HI) and as a function of a user-predeterminable threshold value (H1,...,HN).

**5.** Method according to Claim 1,
further comprising

- dividing the second image (B2) into a set of subimages in accordance with a predetermined division scheme,
- selecting subimages (TB12) of the second image (B2) on the basis of the detected image segments and selecting subimages corresponding thereto (TB11) of the first image (B1)
- and detecting, on the basis of the selected subimages (TB12) of the second image (B2) and on the basis of the selected subimages (TB11) of the first image (B1), respective actual presences of respective cellular fluorescence pattern types by means of the convolutional neural network (CNN2).

**6.** Computing unit (R) for detecting respective potential presences of respective different cellular fluorescence pattern types, wherein the cellular fluorescence pattern types comprise a plurality of different antinuclear antibody fluorescence pattern types,

which, in the course of digital image processing, is configured

- to receive a first image (B1) which represents staining of a biological cell substrate by a first fluorescent dye, wherein the biological cell substrate comprises human epithelioma cells,
- to receive a second image (B2) which represents staining of the biological cell substrate by a second fluorescent dye,
- to detect, on the basis of the first image (B1), respective image segments (VMZ) which in each case represent at least one mitotic cell of a sufficient quality, wherein a mitotic cell is of sufficient quality if it is in metaphase,
- to select, on the basis of the detected image segments (VMZ), subimages (TB11) of the first image (B1) and subimages (TB12) corresponding thereto of the second image (B2), which in each case represent at least one mitotic cell (MZX, MZY) of a sufficient quality and
- to detect, on the basis of the selected subimages (TB11) of the first image (B1) and of the selected subimages (TB12) of the second image (B2), respective actual presences of respective cellular fluorescence pattern types by means of a convolutional neural network (CNN2)

**characterized in that** the computing unit is further configured

- to determine, on the basis of the selected subimages (TB11) of the first image (B1) and of the selected subimages (TB12) of the second image (B2), respective confidence measures (PI, PI*) for the respective actual presences of the respective fluorescence pattern types by means of the convolutional neural network (CNN2).
- for a respective subimage tuple a respective subimage confidence measure (PI1) is determined for respective actual subimage presences of respective cellular fluorescence pattern types by means of the convolutional neural network (CNN2), wherein a subimage tuple comprises a subimage (TB11) of the first image (B1) and a corresponding subimage (TB12) of the second image (B2) and a subimage confidence measure corresponds to a vector of confidence measures, wherein a respective confidence measure corresponds to a respective fluorescence pattern type,
- wherein the convolutional neural network simultaneously evaluates the subimage of the first image (B1) and the corresponding subimage of the second image (B2),
- and determining respective confidence measures for the respective actual presences of the respective fluorescence pattern types on the basis of the subimage confidence measures.

**7.** Data network apparatus (DV) comprising

at least one data interface (DS4) for receiving a first image (B1) which represents staining of a biological cell substrate by a first fluorescent dye, wherein the biological cell substrate comprises human epithelioma cells, and for receiving a second image (B2) which represents staining of the biological cell substrate by a second fluorescent dye,
**characterized by** a computing unit (R) according to claim 6.

**8.** Computer program product (CPP)

comprising commands which, on execution of the program by a computer (CO), cause the latter to carry out the method for digital image processing for detecting respective potential presences of respective different cellular fluorescence pattern types, wherein the cellular fluorescence pattern types comprise a plurality of different antinuclear antibody fluorescence pattern types,
comprising

- receiving a first image (B1) which represents staining of a biological cell substrate by a first fluorescent dye, wherein the biological cell substrate comprises human epithelioma cells,
- receiving a second image (B2) which represents staining of the biological cell substrate by a second fluorescent dye,
- detection, on the basis of the first image (B1), respective image segments (VMZ) which in each case represent at least one mitotic cell (MZX, MZY) of a sufficient quality, wherein a mitotic cell is of sufficient quality if it is in metaphase,
- selecting, on the basis of the detected image segments (VMZ), subimages (TB11) of the first image (B1) and subimages (TB12) corresponding thereto of the second image (B2), which in each case represent at least one mitotic cell (MZX, MZY) of a sufficient quality and
- detecting, on the basis of the selected subimages (TB11) of the first image (B1) and of the selected subimages (TB12) of the second image (B2), of respective actual presences of respective cellular fluorescence pattern types by means of a convolutional neural network (CNN2)

**characterized by**

- determining, on the basis of the selected subimages (TB11) of the first image (B1) and of the selected subimages (TB12) of the second image (B2), of respective confidence measures (PI, PI*) for the respective actual presences of the respective fluorescence pattern types by means of the convolutional neural network (CNN2).
- for a respective subimage tuple a respective subimage confidence measure (PI1) is determined for respective actual subimage presences of respective cellular fluorescence pattern types by means of the convolutional neural network (CNN2), wherein a subimage tuple comprises a subimage (TB11) of the first image (B1) and a corresponding subimage (TB12) of the second image (B2) and a subimage confidence measure corresponds to a vector of confidence measures, wherein a respective confidence measure corresponds to a respective fluorescence pattern type,
- wherein the convolutional neural network simultaneously evaluates the subimage of the first image (B1) and the corresponding subimage of the second image (B2),
- and determining respective confidence measures for the respective actual presences of the respective fluorescence pattern types on the basis of the subimage confidence measures.

9. Data carrier signal (SIG2) which transmits the computer program product (CPP) according to claim 8.

**Revendications**

1. Procédé de détection de présences potentielles respectives de différents types de motifs de fluorescence cellulaire respectifs sur un substrat cellulaire biologique (SU) comportant des cellules d'épithéliome humain,

les types de motifs de fluorescence cellulaire comprenant plusieurs types différents de motifs de fluorescence d'anticorps antinucléaires,
le procédé comportant les étapes suivantes :

- faire incuber le substrat cellulaire (SU) avec un échantillon de patient liquide qui contient potentiellement des anticorps primaires, en outre avec un premier colorant fluorescent et en outre avec des anticorps secondaires qui sont marqués avec un deuxième colorant fluorescent,
- capturer une première image (B1), qui représente une coloration du substrat cellulaire par le premier colorant fluorescent, et capturer une deuxième image (B2), qui représente une coloration du substrat cellulaire par le deuxième colorant fluorescent,
- détecter des segments d'image respectifs (VMZ), qui représentent chacun au moins une cellule mitotique (MZX, MZY) de qualité suffisante, sur la base de la première image (B1), une cellule mitotique présentant une qualité suffisante lorsqu'elle est en métaphase,
- sélectionner des images partielles (TB11) de la première image (B1) et des images partielles correspondantes (TB12) de la deuxième image (B2) sur la base des segments d'image détectés (VMZ), qui représentent chacun au moins une cellule mitotique (MZX, MZY) de qualité suffisante, et
- détecter des présences réelles respectives des types de motifs de fluorescence cellulaire respectifs à l'aide d'un réseau neuronal convolutif (CNN2) sur la base des images partielles sélectionnées (TB11) de la première image (B1) et des images partielles sélectionnées (TB12) de la deuxième image (B2),

**caractérisé par**

- déterminer des mesures de confiance respectives (PI, PI*) pour les présences réelles respectives des types de motifs de fluorescence respectifs à l'aide du réseau neuronal convolutif (CNN2) sur la base des images partielles sélectionnées (TB11) de la première image (B1) et des images partielles sélectionnées (TB12) de la deuxième image (B2),
- une mesure de confiance d'image partielle respective (PI1) pour les présences d'images partielles réelles respectives de types de motifs de fluorescence cellulaire respectifs étant déterminée pour un uplet d'images partielles respectif au moyen du réseau neuronal convolutif (CNN2), un uplet d'images partielles comportant une image partielle (TB11) de la première image (B1) et une image partielle correspondante (TB12) de la deuxième image (B2) et une mesure de confiance d'image partielle correspondant à un vecteur de mesures de confiance, un type de motif de fluorescence respectif correspondant à une mesure de confiance respective,
- le réseau neuronal convolutif évaluant simultanément l'image partielle de la première image (B1) et l'image partielle correspondante de la deuxième image (B2),
- et déterminer des mesures de confiance respectives pour les présences réelles respectives des types de motifs de fluorescence respectifs sur la base des mesures de confiance d'image partielle.

2.  Procédé selon la revendication 1,

    le réseau neuronal convolutif (CNN2) comportant une couche de sortie (LP, AVS) qui génère une carte de caractéristiques respective (FM1,..., FMN) pour un type de motif de fluorescence cellulaire respectif,
    et le réseau neuronal convolutif (CNN2) déterminant une mesure de confiance respective (P1,..., PN) sur la base d'une carte de caractéristiques respective (FM1,..., FMN).

3.  Procédé selon la revendication 1,
    ledit procédé comprenant en outre les étapes suivantes

    - segmenter la première image (B1) en segments d'image de différentes classes de segments,
    - déterminer au moins une valeur de luminosité (HI) pour au moins un type de motif de fluorescence sur la base d'un ou plusieurs segments d'image d'au moins une classe de segments déterminée et
    - vérifier la mesure de confiance (PI, PI*) pour la présence réelle dudit au moins un type de motif de fluorescence sur la base de la valeur de luminosité (HI) dudit au moins un type de motif de fluorescence.

4.  Procédé selon la revendication 3,
    la vérification de la mesure de confiance (PI, PI*) étant effectuée sur la base de la valeur de luminosité (HI) et en fonction d'une valeur seuil (H1,..., HN) pouvant être spécifiée par un utilisateur.

5.  Procédé selon la revendication 1,
    ledit procédé comportant en outre les étapes suivantes

    - diviser la deuxième image (B2) en un ensemble d'images partielles selon un schéma de division spécifié,
    - sélectionner des images partielles (TB12) de la deuxième image (B2) sur la base des segments d'image détectés et sélectionner des images partielles correspondantes (TB11) de la première image (B1)
    - et détecter des présences réelles respectives de types de motifs de fluorescence cellulaire respectifs au moyen du réseau neuronal convolutif (CNN2) sur la base des images partielles sélectionnées (TB12) de la deuxième image (B2) et sur la base des images partielles sélectionnées (TB11) de la première image (B1).

6.  Unité de calcul (R) destinée à détecter les présences potentielles respectives de différents types de motifs de fluorescence cellulaire respectifs, les types de motifs de fluorescence cellulaire comprenant plusieurs types différents de motifs de fluorescence d'anticorps antinucléaires, laquelle unité de calcul est conçue pour, au cours d'un traitement d'image numérique,

- recevoir une première image (B1), qui représente une coloration d'un substrat cellulaire biologique par un premier colorant fluorescent, le substrat cellulaire biologique comportant des cellules d'épithéliome humain,
- recevoir une deuxième image (B2), qui représente une coloration du substrat cellulaire biologique par un deuxième colorant fluorescent,
- détecter, sur la base de la première image (B1), des segments d'image respectifs (VMZ), qui représentent chacun au moins une cellule mitotique de qualité suffisante, une cellule mitotique présentant une qualité suffisante lorsqu'elle est en métaphase,
- sélectionner, sur la base des segments d'image détectés (VMZ), des images partielles (TB11) de la première image (B1) et des images partielles correspondantes (TB12) de la deuxième image (B2), chacune d'elles représentant au moins une cellule mitotique (MZX, MZY) de qualité suffisante, et
- détecter, sur la base des images partielles sélectionnées (TB11) de la première image (B1) et des images partielles sélectionnées (TB12) de la deuxième image (B2), des présences réelles respectives des types de motifs de fluorescence cellulaire respectifs à l'aide d'un réseau neuronal convolutif (CNN2),
**caractérisée en ce que** l'unité de calcul est en outre conçue pour
- déterminer des mesures de confiance respectives (PI, PI*) pour les présences réelles respectives des types de motifs de fluorescence respectifs à l'aide du réseau neuronal convolutif (CNN2) sur la base des images partielles sélectionnées (TB11) de la première image (B1) et des images partielles sélectionnées (TB12) de la deuxième image (B2),
- déterminer, pour un uplet d'images partielles respectif, une mesure de confiance d'image partielle respective (PI1) pour des présences d'images partielles réelles respectives de types de motifs de fluorescence cellulaire respectifs à l'aide du réseau neuronal convolutif (CNN2), un uplet d'images partielles comportant une image partielle (TB11) de la première image (B1) et une image partielle correspondante (TB12) de la deuxième image (B2) et une mesure de confiance d'image partielle correspondant à un vecteur de mesures de confiance, un type de motif de fluorescence respectif correspondant à une mesure de confiance respective,
- le réseau neuronal convolutif évaluant simultanément l'image partielle de la première image (B1) et l'image partielle correspondante de la deuxième image (B2),
- et déterminer des mesures de confiance respectives pour les présences réelles respectives des types de motifs de fluorescence respectifs sur la base des mesures de confiance d'images partielles.

7. Dispositif de réseau de données (DV), comportant

au moins une interface de données (DS4) destinée à recevoir une première image (B1), qui représente une coloration d'un substrat cellulaire biologique par un premier colorant fluorescent, le substrat cellulaire biologique comportant des cellules d'épithéliome humain, et recevoir une deuxième image (B2), qui représente une coloration du substrat cellulaire biologique par un deuxième colorant fluorescent,
**caractérisé par** une unité de calcul (R) selon la revendication 6.

8. Produit-programme informatique (CPP), comprenant des instructions qui, lorsque le programme est exécuté par un ordinateur (CO), amènent celui-ci à mettre en oeuvre un procédé de traitement d'images numériques destiné à détecter des présences potentielles respectives de différents types de motifs de fluorescence cellulaire respectifs, les types de motifs de fluorescence cellulaire comprenant plusieurs types différents de motifs de fluorescence d'anticorps antinucléaires :

ledit procédé comportant les étapes suivantes

- recevoir une première image (B1), qui représente une coloration d'un substrat cellulaire biologique par un premier colorant fluorescent, le substrat cellulaire biologique comportant des cellules d'épithéliome humain,
- recevoir une deuxième image (B2), qui représente une coloration du substrat cellulaire biologique par un deuxième colorant fluorescent,
- détecter des segments d'image respectifs (VMZ), qui représentent chacun au moins une cellule mitotique (MZX, MZY) de qualité suffisante, sur la base de la première image (B1), une cellule mitotique présentant une qualité suffisante lorsqu'elle est en métaphase,
- sélectionner des images partielles (TB11) de la première image (B1) et des images partielles correspondantes (TB12) de la deuxième image (B2) sur la base des segments d'image détectés (VMZ), qui représentent chacun au moins une cellule mitotique (MZX, MZY) de qualité suffisante, et
- détecter des présences réelles respectives des types de motifs de fluorescence cellulaire respectifs à l'aide d'un réseau neuronal convolutif (CNN2) sur la base des images partielles sélectionnées (TB11) de la première image (B1) et des images partielles sélectionnées (TB12) de la deuxième image (B2),

**caractérisé par** les étapes suivantes

- déterminer des mesures de confiance respectives (PI, PI*) pour les présences réelles respectives des types de motifs de fluorescence respectifs à l'aide du réseau neuronal convolutif (CNN2) sur la base des images partielles sélectionnées (TB11) de la première image (B1) et des images partielles sélectionnées (TB12) de la deuxième image (B2),
- déterminer, pour un uplet d'images partielles respectif, une mesure de confiance d'image partielle respective (PI1) pour des présences d'images partielles réelles respectives de types de motifs de fluorescence cellulaire respectifs à l'aide du réseau neuronal convolutif (CNN2), un uplet d'images partielles comportant une image partielle (TB11) de la première image (B1) et une image partielle correspondante (TB12) de la deuxième image (B2), et une mesure de confiance d'image partielle correspondant à un vecteur de mesures de confiance, un type de motif de fluorescence respectif correspondant à une mesure de confiance respective,
- le réseau neuronal convolutif évaluant simultanément l'image partielle de la première image (B1) et l'image partielle correspondante de la deuxième image (B2),
- et déterminer des mesures de confiance respectives pour les présences réelles respectives des types de motifs de fluorescence respectifs sur la base des mesures de confiance d'image partielle.

9. Signal porteur de données (SIG2), lequel transmet le produit-programme informatique (CPP) selon la revendication 8.

## FIG. 1

SC1

SC2

BI1, BI2

SC3

51

SC4

TBI1, TBI2

SC5

DI

V

**FIG. 2**

# FIG. 3

# FIG. 4

EP 3 767 587 B1

**FIG. 5**

Fig.6

Fig.7a

Fig.7b

Fig.7c

Fig.8

Fig.9

Fig.10

Fig.11

BM1

BM2

BM3

BM4

BM5

BM6

BM7

BM8

Fig.12a

SB

Fig.12b

LG

s=1

s=2

s=3

s=4

s=5

s=6

Fig.13

Fig.14

EP 3 767 587 B1

TB11

TB12, TBA

TBS

VMZ

VMZ

Fig.15

FM1  FM2  FM3  FM4

FM5  FM6  FM7  FM8

EP 3 767 587 B1

Fig.16a
TB12

Fig.16b
FM5

Fig.16c
FM2

OLM5

OLM2

Fig.17a

BX

IZX

MZX

Fig.17b

IZX

MZX

Fig.18

Fig.19b

Fig.19a

Fig.20

Fig.21

TBZ11　　　　TBZ12　　　　TBZS

MZZ

Fig.22

FM1  FM2  FM3  FM4

FM5  FM6  FM7  FM8

EP 3 767 587 B1

Fig.23a

TBZ12

MZZ

Fig.23b

FM4

OLM4

Fig.23c

MZZ

Fig.24

HEp-2 cell patterns

| n=1 | | | |
|---|---|---|---|
| **Negativ** AC-0 | **Nukleär** AC-1 bis AC-14, AC-29 | **Zytoplasmatisch** AC-15 bis AC-23 | **Mitotisch** AC-24 bis AC-28 |

n=2 · n=3 · n=6 · n=7 · n=8

| Homogen AC-1 | Gesprenkelt AC-2,4,5, 29 | Zentromer AC-3 | Nukleäre Punkte AC-6, 7 | Nukleolär AC-8,9,10 | Nukleär Randständig AC-11,12 | Pleomorph AC-13,14 | Fibrillär AC-15,16,17 | Gesprenkelt AC-18,19,20 | AMA AC-21 | Golgi AC-22 | Stäbchen und Ringe AC-23 |

Wird nur in ausgewählten Folien angezeigt

n=4

| Dicht fein gesprenkelt AC-2 |
| Fein gesprenkelt AC-4 |
| Grob gesprenkelt AC-5 |
| Topo I AC-29 |

n=5

| Mehrere Punkte AC-6 |
| Wenige Punkte AC-7 |

| Homogen nukleolär AC-8 |
| Schollig nukleolär AC-9 |
| Punktiert nukleolär AC-10 |

| Glatt randständig AC-11 |
| Punktiert randständig AC-12 |

| PCNA AC-13 |
| CENP-F AC-14 |

| linear fibrillär AC-15 |
| filamentös fibrillär AC-16 |
| kurzfasrig AC-17 |

| diskrete Punkte AC-18 |
| dicht fein gesprenkelt AC-19 |
| fein gesprenkelt AC-20 |

| NuMA AC-26 |

| Zentrosomen AC-24 |
| Spindelfasern AC-25 |
| Interzelluläre Brücke AC-27 |
| Chromosomenhülle AC-28 |

Routine-Level
Experten-Level
Chromosomen in der Metaphase angefärbt

EP 3 767 587 B1

**FIG. 25 (1/2)**

**FIG. 25 (2/2)**

## FIG. 26 (1/2)

BI1

PS21 — conv2d_1: Conv2D

| input: | (None, 800, 800, 1) |
|---|---|
| output: | (None, 800, 800, 32) |

PS22 — conv2d_2: Conv2D

| input: | (None, 800, 800, 32) |
|---|---|
| output: | (None, 800, 800, 32) |

PS23

max_pooling2d_1: MaxPooling2D

| input: | (None, 800, 800, 32) |
|---|---|
| output: | (None, 400, 400, 32) |

PS24 — conv2d_3: Conv2D

| input: | (None, 400, 400, 32) |
|---|---|
| output: | (None, 400, 400, 64) |

PS25 — conv2d_4: Conv2D

| input: | (None, 400, 400, 64) |
|---|---|
| output: | (None, 400, 400, 64) |

PS26

max_pooling2d_2: MaxPooling2D

| input: | (None, 400, 400, 64) |
|---|---|
| output: | (None, 200, 200, 64) |

PS27

conv2d_5: Conv2D

| input: | (None, 200, 200, 64) |
|---|---|
| output: | (None, 200, 200, 128) |

PS28

conv2d_6: Conv2D

| input: | (None, 200, 200, 128) |
|---|---|
| output: | (None, 200, 200, 128) |

PS29

conv2d_transpose_1: Conv2DTranspose

| input: | (None, 400, 400, 128) |
|---|---|
| output: | (None, 400, 400, 64) |

## FIG. 26 (2/2)

| 1 |
|---|
| 2 |

PS30

| concatenate_1: Concatenate | input: | (None, 400, 400, 64), (None, 400, 400, 64) |
|---|---|---|
| | output: | (None, 400, 400, 128) |

PS31

| conv2d_7: Conv2D | input: | (None, 400, 400, 128) |
|---|---|---|
| | output: | (None, 400, 400, 64) |

PS32

| conv2d_8: Conv2D | input: | (None, 400, 400, 64) |
|---|---|---|
| | output: | (None, 400, 400, 64) |

PS33

| conv2d_transpose_2: Conv2DTranspose | input: | (None, 400, 400, 64) |
|---|---|---|
| | output: | (None, 800, 800, 32) |

PS34

| concatenate_2: Concatenate | input: | [(None, 800, 800, 32), (None, 800, 800, 32)] |
|---|---|---|
| | output: | (None, 800, 800, 64) |

PS35

| conv2d_9: Conv2D | input: | (None, 800, 800, 64) |
|---|---|---|
| | output: | (None, 800, 800, 32) |

PS36

| conv2d_10: Conv2D | input: | (None, 800, 800, 32) |
|---|---|---|
| | output: | (None, 800, 800, 32) |

PS37

| conv2d_11: Conv2D | input: | (None, 800, 800, 32) |
|---|---|---|
| | output: | (None, 800, 800, 6) |

MS

M1

CNNS

57

Fig. 27

| | TA | FA | TR | FR | Sens | FAR | Spez | FRR |
|---|---|---|---|---|---|---|---|---|
| Dicht fein gesprenkelt (AC-2), n=4 | 21 | 1 | 139 | 0 | 1 | 0.0071 | 0.9929 | 0.0000 |
| Nukleäre Punkte (AC-6,7), n=6 | 9 | 2 | 148 | 2 | 0.8182 | 0.0133 | 0.9867 | 0.1818 |
| Fein oder grob gesprenkelt (AC-4, AC-5), n=5 | 11 | 10 | 138 | 2 | 0.8462 | 0.0676 | 0.9324 | 0.1538 |
| Homogen (AC-1), n=2 | 14 | 5 | 141 | 1 | 0.9333 | 0.0342 | 0.9658 | 0.0667 |
| Nukleär Randständig (AC-11,12), n=8 | 12 | 4 | 144 | 1 | 0.9231 | 0.0270 | 0.9730 | 0.0769 |
| negativ (AC-0), n=1 | 73 | 0 | 82 | 6 | 0.9241 | 0.0000 | 1 | 0.0759 |
| Nukleolär (AC-8,9,10), n=7 | 11 | 7 | 143 | 0 | 1 | 0.0467 | 0.9533 | 0.0000 |
| Zentromer (AC-3), n=3 | 10 | 1 | 150 | 0 | 1 | 0.0066 | 0.9934 | 0.0000 |

Tabelle 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006027516 A1

**In der Beschreibung aufgeführte Nicht-Patentliteratur**

- **C.-H. LIN et al.** *SAT0646 Development and validation of a deep learning algorithm for classifying anti-nuclear antibody patterns in indirect immunofluorescence images,* 01. Juni 2018
- **INFANTINO M ; MEACCI F ; GROSSI V ; MANFREDI M ; BENUCCI M ; MERONE M ; SODA P.** The burden of the variability introduced by the HEp-2 assay kit and the CAD system in ANA indirect immunofluorescence test. *Immunol Res.,* Februar 2017, vol. 65 (1), 345-354
- **HIEMANN, RICO ; ROGGENBUCK, DIRK ; SACK, ULRICH ; ANDERER, URSULA ; CONRAD, KARSTEN.** Die HEp-2-Zelle als Target für multiparametrische Autoantikörperanalytik - Automatisierung und Standardisierung/The HEp-2 cell as target for multiparametric autoantibody analyses: automation and standardisation. *LaboratoriumsMedizin,* 2011, vol. 35 (6), 351-361
- **EGERER, K. ; ROGGENBUCK, D. ; HIEMANN, R. et al.** Automated evaluation of autoantibodies on human epithelial-2 cells as an approach to standardize cell-based immunofluorescence tests. *Arthritis Res Ther,* 2010, vol. 12, R40